# EUROPEAN PATENT APPLICATION

(11) **EP 4 682 164 A1**
(43) Date of publication of application: **21.01.2026**
(21) Application number: 24775147.2
(22) Date of filing: 15.03.2024
(51) Int. Cl.: C07K 14/715, C07K 14/705, A61P 37/02, A61K 38/00

(54) **NOVEL FUSION PROTEIN FOR TREATING AUTOIMMUNE DISEASES**

(30) Priority: 17.03.2023 US 202363452915 P; 17.03.2023 US 202363452932 P; 02.06.2023 US 202363470665 P
(71) Applicant: Curogen Technology Co., Ltd., Suwon-si, Gyeonggi-do 16419 (KR)
(72) Inventor: KIM, Hyun-Soo, Seoul 07998 (KR); MIN, Hee Kyung, Incheon 21526 (KR); SONG, Boseul, Suwon-si Gyeonggi-do 16491 (KR); OH, Jiyeon, Anyang-si Gyeonggi-do 14101 (KR); YANG, Jehoon, Seongnam-si Gyeonggi-do 13499 (KR); CHO, Minsok, Hwaseong-si Gyeonggi-do 18342 (KR); HWANG, In Seong, Suwon-si Gyeonggi-do 16353 (KR); LIM, Nuri, Seoul 07788 (KR); KIM, Eun Ku, Seoul 07519 (KR)
(74) Representative: Mewburn Ellis LLP
(86) International application number: PCT/KR2024/003322
(87) International publication number: WO 2024/196099

(57) **Abstract**

The present invention provides a novel fusion protein for the treatment of autoimmune diseases that significantly reduces the expression of pro-inflammatory cytokines without side effects and demonstrates excellent therapeutic efficacy against various autoimmune diseases.

## Description

### Field of the Invention

The present invention relates to a novel fusion protein for treating autoimmune diseases by regulating pathogenic T helper 17 (Th17) cells in autoimmune diseases. More particularly, it pertains to a novel fusion protein designed to inhibit Interleukin-17A (IL-17A), Interleukin-17F (IL-17F), and tumor necrosis factor alpha (TNFα) signaling pathways and to prevent or treat immunity-related and inflammatory diseases mediated by IL-17A, IL-17F, or TNFα.

### Background Art

IL-17A and IL-17F promote inflammation by inducing the production of numerous inflammatory cytokines, chemokines, and adhesion molecules, as well as recruiting neutrophils and macrophages to the site of inflammation. Several therapeutic antibodies targeting IL-17A (e.g., Secukinumab or Ixekizumab) or IL-17F (e.g., Brodalumab) have demonstrated high clinical efficacy in patients with psoriasis, psoriatic arthritis, and ankylosing spondylitis, and have been approved as the treatments for these conditions. Biological products, such as Bimekizumab and Sonelokimab, which target both IL-17A and IL-17F, have also demonstrated high clinical efficacy and have been approved. The Interlukin-17 family can form six different homodimeric cytokines (IL-17A, IL-17B, IL-17C, IL-17D, IL-17E, and IL-17F) and the heterodimeric IL-17A/F. IL-17 receptor family consists of five members: IL-17RA, IL-17RB, IL-17RC, IL-17RD, and IL-17RE, all of which are type I transmembrane glycoproteins. These receptors interact with IL-17 ligands, mediating host defense while also contributing to inflammatory and autoimmune responses. IL-17RA is a shared receptor that can mediate different IL-17 ligands and distinct signaling pathways. This receptor forms a heterodimer with IL-17RC and mediates signaling induced by the IL-17A or IL-17F homodimer, as well as the IL-17A/F heterodimer. IL-17RC and IL-17RA share 22% sequence identity overall and 55% sequence identity within the extracellular domain (ECD). Compared to IL-17RA, IL-17RC has a longer ECD, but a shorter cytoplasmic domain, which exists in multiple splice forms. IL-17RA has approximately 100-fold weaker affinity for IL-17F compare to IL-17A. IL-17RA and IL-17RC have different expression profiles and tissue distributions. Notably, a distinct cytokine environment involving transforming growth factor beta (TGFβ), IL-6, TNFα, and IL-23 influences the expression patterns of IL-17AA and IL-17FF homodimers, as well as the IL-17AF heterodimer, thereby affecting both naive and differentiated Th17 cells.

TNFα is an inflammatory cytokine produced by activated macrophages and immune cells such as CD4⁺ T cells and natural killer (NK) cells. It is involved in the expansion of both Th1 and Th17 cells and plays a role in regulating the inflammatory response. TNFα is a crucial cytokine for normal immune responses, but imbalances, such as its overexpression in the body, can lead to various autoimmune diseases. Several TNFα inhibitors, such as Infliximab and Adalimumab, as well as the fusion protein Etanercept is used to alleviate and treat autoimmune diseases. However, 30% of patients with autoimmune diseases, including rheumatoid arthritis, do not respond to TNFα inhibitors. Furthermore, in patients who do respond, the response rate decreases with prolonged treatment, often leading to relapses that require switching to other medications. Side effects of TNFα inhibitors include infections such as tuberculosis, lymphoma, and renal failure. However, the long-term release of IL-17A and TNFα into the circulatory system in patients with RA synovitis may cause systemic effects, particularly on the cardiovascular system.

The etiology of autoimmune diseases has not yet been fully identified and is associated with in a variety of factors. For these reasons, it is difficult to achieve effective treatment or a reduction in relapses using single-targeted therapy in patients with autoimmune diseases. Dual-targeted therapy for both TNFα and IL-17A has shown an effective reduction in spondylitis and peripheral arthritis. In the spine, numerical reductions in osteophyma, along with significant decreases in the severity of inflammation and edema, were observed. Additionally, reductions in receptor activator of nuclear factor-κB (RANK) and Osteoprotegerin (OPG) biomarkers were identified.

Rheumatoid arthritis (RA), osteoarthritis (OA), and ankylosing spondylitis are chronic autoimmune diseases characterized by persistent proliferation of the synovial membrane in the joints and destruction of the articular bone and cartilage, caused by dysregulated immune and inflammatory responses influenced by genetic and environmental factors. In particular, the prevalence of RA is rapidly increasing with aging. Degenerative arthritis is classified into approximately 120 subtypes, including rheumatoid arthritis caused by chronic inflammation of the synovial membrane and osteoarthritis. Rheumatoid arthritis, characterized by inflammation, involves the infiltration of immune cells, including CD4⁺ T cells, into approximately 40-50% of synovial cells, along with structural alterations such as synovial hypertrophy caused by inflammation mediated by various cytokines and chemokines. Initial studies focused on Th1 cells and Interferon gamma (IFN-γ) as the main cause of RA but revealed that IFN-γ blockade does not improve the condition. More recently, Th17 cells have been identified as the primary cause of rheumatoid arthritis.

Structural damage in rheumatoid arthritis is associated with bone and cartilage destruction caused by the binding of pathogenic IL-17, produced by Th17 cells, to IL-17 receptors expressed on articular fibroblasts, endothelial cells, and epithelial cells. In human rheumatoid arthritis, IL-17 has been found to induce the production of inflammatory cytokines, chemokines, and matrix metalloproteinases (MMPs), and to amplify inflammatory responses through synergy with other inflammatory driver cytokines, such as TNFα.

A clinical study on anti-TNFα therapy showed significant increases in the levels of circulating Th17 cells and IL-17 in patients who do not respond to this treatment (2.94% vs. 4.23%; 92.1 pg/ml vs. 148.6 pg/ml; respectively, both p<0.05). This result revealed that a significant therapeutic effect of anti-TNFα therapy is strongly associated with a decrease in circulating Th17 cell and IL-17 levels. A proof-of-concept (PoC) study (Study No. NCT02430909) using a combination therapy of Bimekizumab and Certolizumab Pegol (CZP) to target both IL-17 and TNFα was conducted based on the results from post-marketing clinical studies on anti- TNFα therapy. According to the results of this study, Bimekizumab plus CZP achieved more rapid remission compared to CZP plus placebo. However, the incidence rate of treatment-emergent adverse events (TEAEs), such as infections and infestations, was higher in the Bimekizumab plus CZP group (50.0%, 26/52 patients) than in the CZP plus placebo group (22.2%, 6/22 patients). Adverse events (AEs) categorized under the SOC Skin and Subcutaneous Tissue Disorders and the SOC Gastrointestinal Disorders occurred at a higher rate in the Bimekizumab plus CZP group compared to the CZP plus placebo group.

In autoimmune diseases, IL-17 induces the dimerization of IL-17RA and IL-17RC on the cell membrane and contributes to inflammatory responses through the signaling pathways of nuclear factor kappa B (NF-κB) and mitogen-activated protein kinase (MAPK), including extracellular regulated protein kinase (ERK), p38, and c-Jun N-terminal kinase (JNK), via the adaptor protein Act1 and TNF receptor-associated factor 6 (TRAF6). In addition, IL-17 signaling through the Act1-TRAF2-TRAF5 complex stabilizes IL-17 mRNA and promotes the stable expression TNFα and IL-1β mRNAs, thereby leading to stronger inflammatory responses (Shadi Swidani et al., Front. Immunol. 10: 1293, 2019). The Th17 signal pathway, which is involved in various inflammatory responses through pathogenic IL-17 signaling, differs from Th1-dominant inflammatory responses mediated by TNFα. Accordingly, therapies that simultaneously target IL-17 and other cytokines may increase the likelihood of treating autoimmune diseases. Inflammatory responses driven by the synergistic action of IL-17 and TNFα amplify the expression of C-reactive proteins (CRPs), IL-6, IL-8, C-C motif chemokine ligand 20 (CCL20), and monocyte chemoattractant protein 1 (MCP1). When IL-17-TNFα-IL-36 simultaneously stimulated both IL-17 and TNFα in patients with psoriasis, IL-36γ levels in keratinocytes exhibited an IκBζ-dependent increase, leading to serial inflammatory responses characteristic of psoriasis (Manjeet K Paintlia, et al., J. Neurochem. 116(4): 508-521, 2011).

### DETAILED DESCRIPTION OF THE INVENTION

### Technical Problem

The present invention aims to solve the above-mentioned and other problems by providing novel fusion proteins for the treatment of autoimmune diseases. These fusion proteins exhibit excellent therapeutic effectiveness by significantly reducing the expression of inflammatory cytokines without causing side effects. It should be noted, however, that the problems addressed herein are merely exemplary and do not limit the scope of the present invention.

### Technical Solution

According to one aspect of the present invention, there is provided an IL-17RA/RC variant hybrid protein having a sequence homology of at least 90% to the amino acid sequence of SEQ ID NO: 69 and containing amino acid substitutions selected from the group consisting of:
i) Q267R,
ii) T24D, N88D, D122G, and Q267R,
iii) T24D, F59V, N88D, D122G, and Q267R,
iv) L9P, T24D, F59V, N88D, D122G, and Q267R, and
v) L9P, T24D, F59V, N88D, D122G, A156P, and Q267R.

According to another aspect of the present invention, there is provided a polynucleotide encoding the aforementioned IL-17RA/RC variant hybrid proteins.

According to another aspect of the present invention, there is provided an expression vector comprising the aforementioned polynucleotide.

According to another aspect of the present invention, there is provided a transformed cell comprising the aforementioned expression vector.

According to another aspect of the present invention, there is provided a homodimer or heterodimer comprising the aforementioned IL-17RA/RC variant hybrid.

According to another aspect of the present invention, there is provided an IL-17RA/RC-Fc variant hybrid protein in which the antibody Fc domain is linked to either the N-terminus or the C-terminus of the IL-17RA/RC variant hybrid protein.

According to another aspect of the present invention, there is provided a polynucleotide encoding the aforementioned IL-17RA/RC-Fc variant hybrid protein.

According to another aspect of the present invention, there is provided an expression vector comprising the aforementioned polynucleotide.

According to another aspect of the present invention, there is provided a transformed cell transduced withg the aforementioned expression vector.

According to another aspect of the present invention, there is provided a homodimer or heterodimer comprising the aforementioned IL-17RA/RC-Fc variant hybrid protein.

According to another aspect of the present invention, there is provided a TNFR2 ECD variant hybrid protein having a sequence homology of at least 90% to the amino acid sequence of SEQ ID NO: 77 and comprisin amino acid substitutions selected from the group consisting of:
i) S33A,
ii) S33A and S36P,
iii) S33A, S36P, and R119S,
iv) S33A, S36P, R119S, and N164S,
v) S33A, S36P, R119S, N164S, and E210G,
vi) S33A, S36P, R119S, N164S, E210G, and F219I,
vii) S33A, S36P, G75S, R119S, N164S, E210G, F219I,
viii) G75S, and
ix) S33A, S36P, G75S, R119S, and N164S.

According to another aspect of the present invention, there is provided a polynucleotide encoding the aforementioned TNFR2 ECD variant protein.

According to another aspect of the present invention, there is provided an expression vector comprising the aforementioned polynucleotide.

According to another aspect of the present invention, there is provided a transformed cell transduced with the aforementioned expression vector.

According to another aspect of the present invention, there is provided a homodimer or heterodimer comprising the aforementioned TNFR2 ECD variant protein.

According to another aspect of the present invention, there is provided a TNFR2 ECD-Fc variant protein in which the antibody Fc domain is linked to either the N-terminus or the C-terminus of the TNFR2 ECD variant protein.

According to another aspect of the present invention, there is provided a polynucleotides encoding the aforementioned TNFR2 ECD-Fc variant protein.

According to another aspect of the present invention, there is provided an expression vector containing the aforementioned polynucleotide.

According to another aspect of the present invention, there is provided a transformed cell transduced with the aforementioned expression vector.

According to another aspect of the present invention, there is provided a homodimer or heterodimer comprising the aforementioned TNFR2 ECD-Fc variant protein.

According to another aspect of the present invention, there is provided a triple fusion protein comprising:
an IL-17RA/RC variant hybrid protein linked to the N-terminus of an antibody Fc domain, and an extra cellular domain (ECD) of tumor necrosis factor 2 (TNFR2) linked to the C-terminus the antibody Fc domain; or
the ECD of TRFR2 is linked to the N-terminus of the antibody Fc domain and the IL-17RA/RC variant hybrid protein is linked to the C-terminus of the antibody Fc domain.

According to another aspect of the present invention, there is provided a polynucleotide encoding the aforementioned triple fusion protein.

According to another aspect of the present invention, there is provided an expression vector comprising the aforementioned polynucleotide.

According to another aspect of the present invention, there is provided a transformed cell transduced with the aforementioned expression vector.

According to another aspect of the present invention, there is provided a homodimer or heterodimer comprising the aforementioned triple fusion protein.

According to another aspect of the present invention, there is provided a pharmaceutical composition for the treatment of autoimmune diseases comprising the aforementioned IL-17RA/RC variant hybrid protein and TNFR2 ECD variant protein as active ingredients.

According to another aspect of the present invention, there is provided a pharmaceutical composition for the treatment of autoimmune diseases comprising the aforementioned IL-17RA/RC-Fc variant hybrid protein and TNFR2 ECD-Fc variant protein as active ingredients.

According to another aspect of the present invention, there is provided a pharmaceutical composition for the treatment of autoimmune diseases comprising the aforementioned triple fusion protein or the aforementioned homodimer or heterodimer as active ingredients.

### Effect of the Invention

Novel fusion proteins for the treatment of autoimmune diseases, provided by the present invention, can effectively mitigate excessive inflammatory responses mediated by the synergistic stimulation of IL-17A, IL-17F, and TNFα, as well as the irreversible inflammatory disease milieu. In addition, multi-target therapies alone are effective in reducing safety issues related to infection risks caused by the combined use of single-target therapies, as well as the medical costs incurred by patients with autoimmune diseases. It should be noted, however, that these effects do not limit the scope of the present invention.

### Brief Description of the Drawings

FIG. 1 is a graph showing the productivity of wild-type and mutation-introduced IL-17RA/RC-Fc and IL-17RA/RC-Fc-TNFR2 constructs, as shown in Table 1, analyzed by Protein A affinity chromatography.
FIG. 2a shows the S33, S36, G75, R119, N164, E210, and F219 residues that deviate from the family consensus of TNFR2 ECD sequences derived from mammals, as presented in Table 3
FIG. 2b is a graph showing the productivity of eleven types of IL-17RA/RC-Fc-TNFR2 constructs with introduced TNFR2 mutations, as presented in Table 3, of the present invention.
FIG. 2c is a graph showing the affinity (EC₅₀) for TNFα of eleven types of IL-17RA/RC-Fc-TNFR2 constructs with introduced TNFR2 mutations, as presented in Table 3, of the present invention. No. 12 represents the affinity (EC₅₀) for TNFα of Adalimumab, used as the positive control.
FIG. 3a is an SDS-PAGE gel image of IL-17RA/RC-Fc proteins purified using a Protein A column.
FIG. 3b is a graph showing the protein fractions of two types of IL-17RA/RC-Fc-TNFR2 variants (T1-T4-14 and T1-T4-52) purified using a Protein A column.
FIG. 3c is an SDS-PAGE gel image of the proteins obtained after fractionating two types of IL-17RA/RC-Fc-TNFR2 variants (T1-T4-14 and T1-T4-52), purified using a Protein A column, over time.
FIG. 3d is a graph showing the productivity of twenty-six types of IL-17RA/RC-Fc-TNFR2 variants obtained after cloning from pBispec vectors into pcDNA 3.4 vectors, as presented in Table 10. Proteins purified using the method described in Example 3 were used to analyze the yield rate.
FIG. 4 is a graph showing the results of the thermal stability analysis of the IL-17RA-Fc variant protein with an introduced mutation, as presented in Table 2.
FIG. 5a is a graph showing the results of size-exclusion chromatography (SEC) analysis conducted after incubating the IL-17RA/RC-Fc-TNFR2 and IL-17A proteins, produced by coexpression in ExpiCHO cells (as presented in Table 10), and TNFα, produced in *E. coli* cells, at 4°C for 24 hours.
FIG. 5b shows non-reducing and reducing gel images of the fractionated proteins used in the SEC analysis described above.
FIG. 6a is a graph showing the neutralizing ability of the fusion protein IL-17RA/RC-Fc-TNFR2 against human IL-17A (hIL-17A), as measured by secreted alkaline phosphatase (SEAP) analysis.
FIG. 6b is a graph showing the neutralizing ability of the fusion protein IL-17RA/RC-Fc-TNFR2 against hIL-17F, as measured by SEAP analysis.
FIG. 6c is a graph showing the neutralizing ability of the fusion protein IL-17RA/RC-Fc-TNFR2 against TNFα, as measured by SEAP analysis.
FIG. 7a is a graph showing the affinity of the fusion protein IL-17RA/RC-Fc-TNFR2 for hIL-17A.
FIG. 7b is a graph showing the affinity of the fusion protein IL-17RA/RC-Fc-TNFR2 for hIL-17F.
FIG. 7c is a graph showing the affinity of the fusion protein IL-17RA/RC-Fc-TNFR2 for hTNFα.
FIG. 8 is a graph showing the anti-inflammatory activity of the fusion protein IL-17RA/RC-Fc-TNFR2, evaluated using HaCaT cells in a psoriasis-like milieu. The anti-inflammatory effect was assessed by measuring the expression levels of cytokines and chemokines.
FIG. 9 is a graph showing the expression level of IL-6 in human fibroblast-like synoviocytesrheumatoid arthritis (hFLS-RA) cells treated with the fusion protein IL-17RA/RC-Fc-TNFR2.
FIG. 10a shows the structure of the tmTNFα-expressing vector (pLVX tmTNFα-IRES-ZsGreen1). Transmembrane TNFα (tmTNFα)-expressing cells were generated to analyze the binding ability of the fusion protein IL-17RA/RC-Fc-TNFR2 to tmTNFα.
FIG. 10b is a concept map illustrating the binding of the fusion protein IL-17RA/RC-Fc-TNFR2 in tmTNFα-expressing cell lines.
FIG. 10c is a fluorescence micrograph showing the binding capacity of the fusion protein IL-17RA/RC-Fc-TNFR2 to tmTNFα.
FIG. 11a is a diagram illustrating the procedures for analyzing major histocompatibility complex (MHC) Class I and Class II epitopes to assess the immunogenicity of the fusion protein IL-17RA/RC-Fc-TNFR2.
FIG. 11b is a graph showing the predicted binding sites and immunogenicity levels of MHC Class II epitopes (Red: high, Orange: medium, Yellow: low) to assess the immunogenicity of the fusion protein IL-17RA/RC-Fc-TNFR2.
FIG. 12a is a graph showing the *in vitro* immunogenicity of the fusion protein IL-17RA/RC-Fc-TNFR2.
FIG. 12b is a graph showing the *in vitro* immunogenicity of the fusion protein IL-17RA/RC-Fc-TNFR2.
FIG. 12c is a graph displaying the stimulation index (SI) analyzed to evaluate the immunogenicity of the fusion protein IL-17RA/RC-Fc-TNFR2.
FIG. 13 is a graph displaying the expression levels of IL-17A and IL-17F cytokines by the fusion protein IL-17RA/RC-Fc-TNFR2 in peripheral blood mononuclear cells (PBMCs) derived from patients with psoriasis.
FIG. 14a is a graph displaying the expression of hIL-17A and hIL-17F, along with the absence of mouse IL-17A (mIL-17A) and mIL-17F expression, in hIL-17A/hIL-17F double knock-in mice. The upper and lower graphs represent heterozygous and homozygous mice, respectively.
FIG. 14b is a graph displaying the response of the fusion protein IL-17RA/RC-Fc-TNFR2 to hIL-17A and mTNFα.
FIG. 14c illustrates the process of an *in vivo* experiment using C57BL/6 mice treated with IL-17RA/RC-Fc-mTNFR2.
FIG. 14d is a graph displaying the neutralizing ability against hIL-17A and mTNFα in C57BL/6 mice and hIL-17A/hIL-17F double knock-in mice.
FIG. 15a is a 2D view of the structure of the fusion protein IL-17RA/RC-Fc-TNFR2. It is a collection of 2D classification images, identified from various angles.
FIG. 15b is a 3D view of the structure of the fusion protein IL-17RA/RC-Fc-TNFR2, observed using an electron microscope. The structure was reconstructed in 3D based on 2D classification images.
FIG. 15c is an electron density map displaying the structure of the fusion protein IL-17RA/RC-Fc-TNFR2. The structure was superimposed with the IL-17RC-IL-17F complex (PDB ID: 6HG9), IgG1, and TNFα-TNFR2 complex (PDB ID: 3ALQ) in the electron density map, which was reconstructed in 3D based on 2D classification images.
FIG. 16a shows the mass spectrometry results for the hydrolase trypsin of the fusion protein IL-17RA/RC-Fc-TNFR2 (T1-T4-14).
FIG. 16b shows the mass spectrometry results for the hydrolase chymotrypsin of the fusion protein IL-17RA/RC-Fc-TNFR2 (T1-T4-14).
FIG. 17a displays the production schedule for the glycan sialylation of the IL-17RA/RC-Fc-TNFR2 hybrid fusion protein.
FIGs. 17b, 17c, and 17d show an SDS-PAGE image of proteins following separation and purification from the culture medium of ExpiCHO-S cells transfected with IL-17RA/RC-Fc-TNFR2 and GT6 using a Protein A column, the results of SEC analysis, and a western blot image of proteins with an increased size due to sialylation, respectively.
FIGs. 17e, 17f, and 17g display the binding affinity of the IL-17RA/RC-Fc-TNFR2 hybrid fusion protein (T1-T4-14+GT6) undergoing glycan sialylation for IL-17A, IL-17F, and TNFα, as determined by enzyme-linked immunosorbent assay (ELISA).
FIGs. 18a and 18b present a series of graphs showing the neutralizing ability of the IL-17RA/RC-Fc-TNFR2 hybrid fusion protein against TNFR2 and Adalimumab, as determined by analyzing the expression levels of IL-6 (upper) and MMP3 (lower) in SW982 cells.
FIG. 19 illustrates the stereoscopic structure of the fusion protein IL-17RA/RC-Fc-TNFR2.

### Best Mode

### Definitions of Terms:

Among the six IL-17 family members, the terms "IL-17A and IL-17F", as used herein, share a high sequence homology of at least 50%. These members form either two homodimers (IL-17AA and IL-17FF) or one heterodimer (IL-17AF), which induces pathological IL-17 signaling through the IL-17RA/RC signaling assembly. The IL-17RA/RC heterodimer causes inflammatory responses through the NF-κB, MAPK, ERK, p38, and JNK signaling pathways via the adaptor protein Act1 and TRAF6. IL-17 signaling through the Act1-TRAF2-TRAF5 complex stabilizes the mRNAs of inflammatory cytokines and chemokines (e.g., IL-17A, TNFα, and IL-1β), thereby amplifying the inflammatory response (Shadi Swidani et al. Front. Immunol. 10: 1293, 2019).

Pathogenic IL-17 signaling in Th17 cells follows a different pathway compared to the inflammatory response induced by TNFα from Th1 cells. When IL-17 and TNFα act synergistically, inflammation may be irreversibly amplified. Irreversibly amplified inflammation may cause difficulty in mitigating autoimmune diseases with single-target therapies. Accordingly, therapies targeting both IL-17 and TNFα may enhance the potential for treating autoimmune diseases.

The term "TNFα", as used herein, refers to a membrane protein consisting of 233 amino acids that mediates biological activity by releasing soluble TNFα (sTNFα), which comprises 157 amino acids, through the action of TNFα converting enzyme (TACE). TNFα is involved in signaling mediated by two distinct TNFα receptors (TNFR1 and TNFR2) and in inflammatory responses mediated by tmTNFα, the precursor of sTNFα. TNFR1 is expressed in most tissue cells, while TNFR2 is primarily expressed in immune cells. TNFR2 is activated by tmTNFα rather than sTNFα. The activation of TNFR1 causes inflammation-related responses mediated by apoptosis. TNFR2 signaling in activated T cells promotes cell proliferation. Currently, five TNFα inhibitors (Etanercept, Infliximab, Golimumab, Adalimumab, and Certolizumab pegol) have been approved and marketed as biological products for the treatment of TNFα-induced diseases. Each antibody differs in therapeutic effectiveness, suggesting that actions, other than equal levels of TNFα neutralization, may determine a difference in treatment outcomes. For instance, Etanercept failed to demonstrate efficacy in clinical studies on patients with Crohn disease, Wegener's granulomatosis, or sarcoidosis and showed lower therapeutic effectiveness in psoriasis compared to antibody-based TNFα inhibitors. Therapeutic antibodies against TNFα can stably bind to both sTNFα and tmTNFα, unlike the TNF receptor-associated protein (TRAP), Etanercept. During treatment with therapeutic antibodies against TNFα, the activation of tmTNFα is closely associated with the production of TGF-β and IL-10 induced by tmTNFα. Furthermore, anti-TNFα antibody therapy has been reported to activate regulatory T cells, thereby offering additional benefits in some autoimmune diseases compared to Etanercept (S Bombardieri et al., Rheumatology, 46(7): 1191-1199, 2007).

### Detailed Description of the Invention

According to one aspect of the present invention, there is provided an IL-17RA/RC variant hybrid proteinhaving a sequence homology of at least 90% to the amino acid sequence of SEQ ID NO: 69 and comprising amino acid substitutions selected from the group consisting of:
i) Q267R,
ii) T24D, N88D, D122G, and Q267R,
iii) T24D, F59V, N88D, D122G, and Q267R,
iv) L9P, T24D, F59V, N88D, D122G, and Q267R, and
v) L9P, T24D, F59V, N88D, D122G, A156P, and Q267R.

In the aforementioned hybrid protein, a glycan may be attached and may include at least one selected from the group consisting of N-glycan, O-glycan, and sialylated glycan.

The aforementioned hybrid protein may be selected from the group consisting of the amino acid sequences of SEQ ID NOs: 66 to 69.

According to another aspect of the present invention, there is provided a polynucleotide encoding the aforementioned IL-17RA/RC variant hybrid protein.

According to another aspect of the present invention, there is provided an expression vector containing the aforementioned polynucleotide.

According to another aspect of the present invention, there is provided a transformed cell transduced with the aforementioned expression vector.

According to another aspect of the present invention, there is provided a homodimer or heterodimer containing the aforementioned IL-17RA/RC variant hybrid protein.

According to another aspect of the present invention, there is provided an IL-17RA/RC-Fc variant hybrid protein in which the antibody Fc domain is linked to either the N-terminus or C-terminus of the IL-17RA/RC variant hybrid protein.

In the aforementioned hybrid protein, the antibody Fc domain may be a hybrid Fc domain composed of at least two homozygous Fc domains and may be selected from the group consisting of the amino acid sequences of SEQ ID NOs: 33 to 65.

According to another aspect of the present invention, there is provided a polynucleotide encoding the aforementioned IL-17RA/RC-Fc variant hybrid protein.

According to another aspect of the present invention, there is provided an expression vector containing the aforementioned polynucleotide.

According to another aspect of the present invention, there is provided a transformed cell containing the aforementioned expression vector.

According to another aspect of the present invention, there is provided a homodimer or heterodimer containing the aforementioned IL-17RA/RC variant hybrid protein.

According to another aspect of the present invention, there is provided a TNFR2 ECD variant protein having a sequence homology of at least 90% to the amino acid sequence of SEQ ID NO: 77 and comprising amino acid substitutions selected from the group consisting of:
i) S33A,
ii) S33A and S36P,
iii) S33A, S36P, and R119S,
iv) S33A, S36P, R119S, and N164S,
v) S33A, S36P, R119S, N164S, and E210G,
vi) S33A, S36P, R119S, N164S, E210G, and F219I,
vii) S33A, S36P, G75S, R119S, N164S, E210G, F219I,
viii) G75S, and
ix) S33A, S36P, G75S, R119S, and N164S.

The aforementioned variant protein may form a complex in which at least one of i) to ix) is linked to another by a linker peptide. A glycan may be attached to the variant protein. The glycan may include at least one selected from the group consisting of N-glycan, O-glycan, and sialylated glycan and may be further selected from the group consisting of the amino acid sequences of SEQ ID NOs: 70 to 81.

According to another aspect of the present invention, there is provided a polynucleotide encoding the aforementioned TNFR2 ECD variant protein.

According to another aspect of the present invention, there is provided an expression vector comprising the aforementioned polynucleotide.

According to another aspect of the present invention, there is provided a transformed cell comprising the aforementioned expression vector.

According to another aspect of the present invention, there is provided a homodimer or heterodimer comprising the aforementioned TNFR2 ECD variant protein.

According to another aspect of the present invention, there is provided a TNFR2 ECD-Fc variant protein in which the antibody Fc domain is linked to either the N-terminus or the C-terminus of the TNFR2 ECD variant protein.

In the aforementioned variant protein, the antibody Fc domain may be a hybrid Fc domain composed of at least two homozygous Fc domains and may be selected from the group consisting of the amino acid sequences of SEQ ID NOs: 33 to 65.

According to another aspect of the present invention, there is provided a polynucleotide encoding the aforementioned TNFR2 ECD-Fc variant protein.

According to another aspect of the present invention, there is provided an expression vector containing the aforementioned polynucleotide.

According to another aspect of the present invention, there is provided a transformed cell containing the aforementioned expression vector.

According to another aspect of the present invention, there is provides a homodimer or a heterodimer comprising the aforementioned TNFR2 ECD-Fc variant proteins.

According to another aspect of the present invention, there is provided a triple fusion protein comprising:
an IL-17RA/RC variant hybrid protein linked to the N-terminus of an antibody Fc domain, and an extra cellular domain (ECD) of tumor necrosis factor 2 (TNFR2) linked to the C-terminus the antibody Fc domain; or
an the ECD of TRFR2 is linked to the N-terminus of the antibody Fc domain and the IL-17RA/RC variant hybrid protein is linked to the C-terminus of the antibody Fc domain.

In the aforementioned triple fusion protein, the IL-17RA/RC variant hybrid protein may be characterized by having a sequence homology of at least 90% to the amino acid sequence of SEQ ID NO: 69 and by including amino acid substitutions selected from the group consisting of:
i) Q267R,
ii) T24D, N88D, D122G, and Q267R,
iii) T24D, F59V, N88D, D122G, and Q267R,
iv) L9P, T24D, F59V, N88D, D122G, and Q267R, and
v) L9P, T24D, F59V, N88D, D122G, A156P, and Q267R.

In the aforementioned triple fusion protein, the TNFR2 ECD may be characterized by having a sequence homology of at least 90% to the amino acid sequence of SEQ ID NO: 77 and by including amino acid substitutions selected from the group consisting of:
i) S33A,
ii) S33A and S36P,
iii) S33A, S36P, and R119S,
iv) S33A, S36P, R119S, and N164S,
v) S33A, S36P, R119S, N164S, and E210G,
vi) S33A, S36P, R119S, N164S, E210G, and F219I,
vii) S33A, S36P, G75S, R119S, N164S, E210G, F219I,
viii) G75S, and
ix) S33A, S36P, G75S, R119S, and N164S.

The aforementioned triple fusion protein may form a complex in which at least one of i) to ix) is linked to another by a linker peptide. A glycan may be attached to the fusion protein and may include at least one selected from the group consisting of N-glycan, O-glycan, and sialylated glycan.

In the aforementioned triple fusion protein, the TNFR2 may be selected from the group consisting of the amino acid sequences of SEQ ID NOs: 83 to 94 and may simultaneously target IL-17A, IL-17F, and TNFα. It may be selected from the group consisting of the amino acid sequences of SEO ID NOs: 1, 2, 4, 6, 8, 10, 12, 14, 16, 18, 20, 22, 24, 26, 28, 30, and 32.

According to another aspect of the present invention, there is provided a polynucleotide encoding the aforementioned triple fusion protein.

According to another aspect of the present invention, there is provided an expression vector comprising the aforementioned polynucleotide.

According to another aspect of the present invention, there is provided a transformed cell containing the aforementioned expression vector.

According to another aspect of the present invention, there is provided a homodimer or a heterodimer containing the aforementioned triple fusion protein.

According to another aspect of the present invention, there is provided a pharmaceutical composition for the treatment of autoimmune diseases comprising the aforementioned IL-17RA/RC-Fc variant hybrid protein and TNFR2 ECD-Fc variant protein as active ingredients.

According to another aspect of the present invention, there is provided a pharmaceutical composition for the treatment of autoimmune diseases comprising the aforementioned triple fusion protein and homodimer or heterodimer as active ingredients.

In the aforementioned pharmaceutical compositions, the autoimmune diseases may be selected from the group consisting of rheumatoid arthritis (RA), psoriasis, inflammatory bowel disease (IBD), systemic lupus erythematosus (SLE), ankylosing spondylitis, pneumonia, asthma, atopic dermatitis, periodontitis, pyorrhea, conjunctivitis, keratitis, dry eye syndrome, fibromyositis, lupus, systemic sclerosis, aphthous ulcers, Sjogren's syndrome, Guillain-Barre syndrome (GBS), alopecia areata, polymyositis, dermatomyositis, polyarteritis nodosa (PAN), relapsing polychondritis (RP), thrombocytopenia, and multiple sclerosis (MS). The IBD may be selected from the group consisting of ulcerative colitis, Crohn's disease, and Behget's disease.

To improve the stability and neutralizing ability of the IL-17RA/RC hybrid and TNFR2 provided by the present invention, information on the similarity of amino acid sequences and domains between proteins from different species was used for the introduction and reconstruction of mutations. Back-to-consensus and ancestral mutations, based on the bioinformatics, are used to study the co-evolution of specific protein residues that enable adaptation to the environment over long periods. A small gene library based on evolutional methodologies is generated to enhance the structural stability and function of proteins for analyzing protein-protein interactions (PPI). Through the simulation of this gene library, residue substitution patterns and structural stabilization patterns, resulting from structural changes with other residues before and after substitutions, are identified *in vitro,* thereby elucidating the mechanism that maintains high affinity and specificity. This procedure was used to produce and add the IL-17RA/RC hybrid and the TNFR2 variant.

A multi-specific fusion protein is a protein capable of binding to at least two antigens, either simultaneously or sequentially. Specifically, for a fusion protein specific to three targets, the first antigen-binding domain can bind to the first antigen expressed on the cell surface as well as its secreted form. Similarly, the second and third antigen-binding domains can bind their respective targets in the same manner. This fusion protein may bind the first antigen and the second antigen more effectively when it binds a homodimer or a heterodimer consisting of the first antigen or the second antigen. In addition, the multi-specific fusion proteins can effectively target two cells or facilitate their interaction, thereby inducing or blocking specific signaling pathways. The multi-specific fusion protein provided by the present invention may regulate the activation of various immune cells, including CD4⁺ T cells, CD8⁺ T cells, monocytes, neutrophils, and macrophages, and may effectively improve the immune milieu in disease conditions.

In one embodiment, the multi-specific fusion protein described by the present application can bind to one or more targets, antigens, or epitopes with a dissociation constant (K_{D}) of approximately 1 µM or lower, 100 nM or lower, 40 nM or lower, 20 nM or lower, 10 nM or lower, 1 nM or lower, 0.1 nM or lower, 50 pM or lower, 10 pM or lower, or 1 pM or lower. The binding affinity of the first antigen-binding domain for the first antigen may be higher than that of the second or third antigen-binding domain for the second or third antigen.

The present invention relates to a multi-specific fusion protein, which may be in a chemically modified form. In some embodiments, the aforementioned fusion protein may be chemically modified through glycosylation, acetylation, pegylation, amidation, derivatization with known protecting groups or blocking groups, proteolytic cleavage, or binding to cellular ligands or other proteins. These chemical modifications can be performed using established techniques.

The term "expression vector", as used herein, refers to a vector designed to enable the expression of genetic material within cells. This is achieved by inserting segments after polymerase chain reaction (PCR) amplification of an antibody, the receptor expression domain of a protein, or a fragment thereof, into a vector truncated with a restriction enzyme.

For the expression vector provided by the present invention, any expression vector system may be used, including pcDNA, pBispecific, pTT, pTT3, pEFBOS, pBV, pJV, pcDNA3.4 TOPO, pEF6 TOPO, or pBJ. Specifically, the expression vector may be a pBispecific vector or a pcDNA3.4 vector.

One embodiment of the fusion protein provided by the present invention may have the following structural formulas:

N'-A-L1-B-L2-X-L3-C-C' (I)

or

N'-C-L1-X-L1-B-L3-A-C' (II)

In the above structural formulas (I) and (II), N' refers to the N-terminus of the fusion protein, and C' refers to its C-terminus. A, B, and C represent domains specific to the first antigen, the second antigen, and the third antigen, respectively. L1 to L3 represent peptide hinges or linkers. X is the Fc domain and the C-terminal region of an immunoglobulin (Ig), which includes a natural Fc domain, a recombinant Fc domain, and a variant Fc domain and. It refers to a constant domain, known as "Fc", which is linked to a protein with effector functions. The Fc domain of a therapeutic protein may contribute to an extended half-life or integrate functions such as Fc receptor binding, Protein A binding, complement fixation, and placental transfer. In particular, the extended half-life provided by the Fc domain is attributed to its binding affinity for the neonatal Fc receptor (FcRn). Besides increasing half-life, the Fc domain can inactivate Fc domain-dependent effects of an antibody, such as antibody-dependent cellular cytotoxicity (ADCC) or complement-dependent cytotoxicity (CDC), which may occur as side effects during the use of antibody-based protein therapies. It should be noted, however, that the effectiveness of the present invention is not limited to the explanations provided above.

The variant Fc domain refers to a recombinant antibody Fc domain protein created by combining derivatives from various types of antibody molecules (e.g., IgG, IgD, IgE, IgM), in which some or all parts of the Fc components of an immunoglobulin, including the hinge, CH2, and CH3 domains, differ. An example of a modified IgG4 immunoglobulin may be one of the hybrid Fc (hyFc) domain proteins (WO2008147143A2, WO2020102728A1, and WO2020102728A1).

Increasing the Fc affinity for FcRn under endosomal conditions (acidic pH) through Fc engineering to extend the half-life of a variant Fc domain has been used as an effective approach to prolong the pharmacokinetics of monoclonal antibodies (Atsuhiko Maeda et al., Mabs. 9(5): 844-853, 2017). The following mutation sets can be used individually or in combination: LS mutation (M428L/N434S, Xencor), YTE mutation (M252Y/S254T/T256E, Medimmune), HN mutation (H433K/N434F), QL mutation (T250Q/M428L. Paul R. Hinton et al., J. Biol. Chem. 279(8): 6213-6216, 2004), ABDEG mutation (M252Y/S254T/T256E/H433K/N434F), EDHS mutation (V264E/L309D/Q311H/N434S), EDHY mutation (V264E/L309D/Q311H/N434Y), DHS mutation (L309D/Q311H/N434S), DHY mutation (L309D/Q311H/N434Y), IgG2-DHS mutation (V309D/Q311H/N434S), IgG3-DHS mutation (L309D/Q311H/N434S), or IgG4-DHS mutation (L309D/Q311H/N434S).

In the above structural formulas (I) and (II), L1 to L3 represent peptide hinges or linkers. The lengths of linker peptides in the fusion protein may range from 2 to 60 amino acids (a.a.), 4 to 55 a.a., 5 to 50 a.a., 5 to 46 a.a., 5 to 45 a.a., or 5 to 30 a.a. The linker peptide connecting an active pharmaceutical ingredient (API) to the N-terminus of an immunoglobulin Fc domain variant protein among the above linker peptides may include some or all parts of the hinge region of the IgG1 antibody heavy chain. When part of the hinge region is included, the linker peptide may have an artificial linker peptide added to either the N-terminus or C-terminus of the hinge region. Furthermore, the hinge region may be in a hybrid form, combining the hinge regions of two or more antibody heavy chains. The aforementioned linker peptides may be one or more combinations selected from the group consisting of:
· GGGGSGGGGSGGGGSEKEKEEQEERTHTCPPCP (SEQ ID NO: 83),
· RNTGRGGEEKKGSKEKEEQEERETKTPECP (SEQ ID NO: 84),
· RNTGRGGEEKKSGKEKEEQEERETKTPECP (SEQ ID NO: 85),
· RNTGRGGEEKKGGKEKEEQEERETKTPECP (SEQ ID NO: 86),
· RNTGRGGEEKKSSKEKEEQEERETKTPECP (SEQ ID NO: 87),
·RNTGRGGEEKKKEKEKEEQEERETKTPECP (SEQ ID NO: 88),
· GGGGSGGGGSGGGGSLE (SEQ ID NO: 89),
· TGIEGRMD (SEQ ID NO: 90),
· TGKLSGSASAPKLEEGEFSEARV (SEQ ID NO: 91),
· KLSGSASAPKLEEGEFSEARVLE (SEQ ID NO: 92),
· TGGSGEGEGSEGSG (SEQ ID NO: 93),
· GSGEGEGSEGSGLE (SEQ ID NO: 94),
· AEAAAKEAAAAKA (SEQ ID NO: 95),
· GGGGSGGGGGSGGGGS (SEQ ID NO: 96),
· A(EAAAK)₄ALEA(EAAAK)₄A (SEQ ID NO: 97),
· EPKSSDKTHTCPPCP (SEQ ID NO: 98), and
· EPKSCDKTHTCPPCP (SEQ ID NO: 99).

Changes in the glycan composition and structure of the Fc-fusion protein may affect its structural domain. In addition, alterations in specific glycosylation of a therapeutic Fc-fusion protein may impact the pharmacokinetics (PK) and pharmacodynamics (PD) of the molecule. Glycans that significantly affect PK and PD include mannose, sialic acid, fucose, and galactose.

In some embodiments of the present invention, specific mutations may be considered to modify the glycosylation of polypeptides. The applicable mutation may be selected to introduce or remove one or more glycosylation sites, such as O-linked or N-linked glycosylation sites. An increase or decrease in the number of carbohydrate residues on polypeptides is caused by the chemical and/or enzymatic binding of glycosides to polypeptides. In some embodiments, the sequence of polypeptides may be appropriately adjusted depending on the type of expression system used, such as a mammalian or yeast system. In both insect and plant cells, a different glycosylation pattern, likely influenced by the amino acid sequence of peptides, may be introduced. Polypeptides provided by the present invention for human use can be expressed in mammalian cell lines that offer adequate glycosylation, such as HEK293 or CHO cell lines.

N-linked glycosylation is a process that attaches glycan structures to the amide nitrogen of asparagine residues in a protein. A glycan is a chain of carbohydrates that can be branched and flexible. The exact structure of the glycan attached to the asparagine residues of a protein depends on the expression system used for glycoprotein production. The N-glycan in the IL-17RA/IL-17RC hybrid protein (SEQ ID NO: 69) may be attached to at least one amino acid residue selected from N17, N35, N173, N186, N213, N223, N309, N332, and N366. The N-glycan in the TNFR2 ECD (SEQ ID NO: 77) may be attached to at least one amino acid residue selected from N149 and N171.

In some embodiments of the present invention, the terminal residue of a glycan in the therapeutic protein can increase its *in vivo* half-life. Accordingly, methods to decrease the content of N-glycolylneuraminic acid (Neu5Gc) and increase the content of N-acetylneuraminic acid (Neu5Ac) in a glycoprotein expressed in CHO cells can be introduced. Sialylated glycoproteins at levels higher than intrinsic levels can be produced through cellular manipulation of working cell lines, thereby increasing the half-life of the multi-target fusion protein described in the present invention.

Rheumatoid arthritis, osteoarthritis, and ankylosing spondylitis are chronic autoimmune diseases characterized by persistent proliferation of the synovial membrane in the joints and destruction of the articular bone and cartilage, caused by dysregulated immune and inflammatory responses influenced by genetic and environmental factors. In particular, the prevalence of rheumatoid arthritis is rapidly increasing with aging. Degenerative arthritis is classified into approximately 120 subtypes, including, rheumatoid arthritis caused by chronic inflammation of the synovial membrane and osteoarthritis. Rheumatoid arthritis, characterized by inflammation, involves the infiltration of immune cells, including CD4⁺ T cells, into approximately 40-50% of synovial cells, along with structural alterations such as synovial hypertrophy caused by inflammation mediated by various cytokines and chemokines. Initial studies focused on Th1 cells and IFN-γ as the main cause of RA but revealed that IFN-γ blockade does not improve the condition. More recently, Th17 cells have been identified as the primary cause of rheumatoid arthritis.

In patients with rheumatoid arthritis, pro-inflammatory responses resulting from the binding of IL-17 receptors on articular fibroblasts, endothelial cells, and epithelial cells in structurally damaged tissues have been identified. IL-17 contributes to the destruction of bone and cartilage and induces the production of inflammatory cytokines, chemokines, and MMPs. The role of IL-17, the primary inflammatory driver cytokine, in its synergistic effect with TNFα has been studied in RA patients. Synergistic signaling of IL-17 and TNFα amplifies the mRNA expression of pro-inflammatory cytokines and chemokines. The inflammatory response caused by the synergistic stimulation of IL-17 and TNFα amplifies the expression of CRP, IL-6, IL-8, CCL20, and MCP1. In patients with psoriasis, simultaneous stimulation of both IL-17 and TNFα by IL-17-TNFα-IL-36 led to an IκBζ-dependent increase in IL-36γ levels in keratinocytes, along with a cascade of inflammatory responses characteristic of psoriasis (Manjeet K Paintlia, et al., J. Neurochem. 116(4): 508-521, 2011).

The tri-specific fusion protein IL-17RA/RC-L-Fc-L-TNFR2 (hereinafter referred to as 'IL-17RA/RC-Fc-TNFR2', where L refers to a linker), provided by the present invention, is a protein therapy designed to effectively improve an inflammatory milieu. This effectiveness is achieved by regulating inflammatory signaling mediated by IL-17A and IL-17F, which are elevated in certain non-T cells, including pathogenic Th17 cells, NKT cells, γδ T cells, monocytes, neutrophils, macrophages, and eosinophils. This fusion protein exhibits target affinity equivalent to or greater than that of a single-target antibody against IL-17A (e.g., Secukinumab) or a multi-target antibody that simultaneously targets both IL-17A and IL-17F (e.g., Bimekizumab).

The fusion protein IL-17RA/RC-Fc-TNFR2 provided by the present invention exhibits target affinity that is equivalent to or greater than that of a single-target antibody against TNFα (e.g., Adalimumab) or a TNFα-inhibitor fusion protein (e.g., Etanercept). The fusion protein is a protein therapy that effectively blocks inflammatory signaling of TNFR1/2 by binding to the ligands sTNFα and tmTNFα, activates regulatory T (Treg) cells, and induces anti-inflammatory responses through reverse signaling.

The fusion protein IL-17RA/RC-Fc-TNFR2 provided by the present invention is a protein therapy that treats autoimmune diseases by simultaneously or sequentially blocking inflammatory responses mediated by the cytokines IL-17A, IL-17F, or TNFα and effectively improving the pro-inflammatory immune milieu. Furthermore, the fusion protein reduces disease relapse by stably maintaining the improved immune milieu.

The fusion protein IL-17RA/RC-Fc-TNFR2 provided by the present invention additionally offers methods for treating inflammation or inflammatory disorders through intravenous or subcutaneous administration to subjects. However, IL-17RA/RC-Fc-TNFR2 is administered to subjects with a route of administration, dose, and dosing frequency determined based on the patient's health condition and the presence or absence of side effects. The optimal dosing regimen, dose, and dosing frequency can be determined within an appropriate range. In addition, the fusion protein may be co-administered with other medications or physiological active substances known for their therapeutic effectiveness against the intended disease, or it may be formulated into a dosage form combined with other medications.

The fusion protein IL-17RA/RC-Fc-TNFR2 disclosed in the present invention provides methods for treating inflammation, inflammatory autoimmune diseases, and chronic inflammation-derived cancers. These methods include the administration or reconstitution of separated peptides, or a pharmaceutical composition containing the separated peptides, at a therapeutically effective dose in subjects requiring treatment.

The fusion protein IL-17RA/RC-Fc-TNFR2 provided by the present invention serves as a treatment that combines a therapeutically effective dose of separated peptides, or a pharmaceutical composition containing the separated peptides, with at least one anti-inflammatory agent. The anti-inflammatory agent contains corticosteroids and provides a method for treating inflammation or inflammatory disorders. Specifically, it may be an immunosuppressant agent used either as monotherapy or in combination therapy with any of the following options: cortisol, aldosterone, hydrocortisone, hydrocortisone acetate, cortisol acetate, tixocortol pivalate, prednisolone, methylprednisolone, prednisone, triamcinolone acetonide, salicylic acid, resorcinol, sulfacetamide, urea, imidazole, betamethasone, betamethasone sodium phosphate, dexamethasone, dexamethasone sodium phosphate, nonsteroidal anti-inflammatory drugs (NSAIDs), COX-2 inhibitors, capsaicin, ibuprofen, chlorprednisone, cyclosporine, cytokine synthesis inhibitors, tetracycline, minocycline, or doxycycline.

The present invention provides an antagonist tageting the pro-inflammatory cytokines IL-17A, IL-17F, and TNFα. IL-17A, IL-17F, and TNFα are cytokines that mediate pro-inflammatory responses associated with autoimmune diseases and chronic inflammatory diseases. Specifically, the synergistic action of these cytokines excessively induces inflammatory activity, resulting in an irreversible inflammatory disease milieu. The present invention is an antagonist capable of regulating (e.g., blocking, suppressing, reducing, antagonizing, neutralizing, or inhibiting) the production of pro-inflammatory cytokines and chemokines mediated by IL-17A, IL-17F, and TNFα. This antagonist is superior to therapies that target one or two of these three cytokines and offer the advantage of fundamentally blocking the worsening of inflammation caused by the synergistic action of IL-17A, IL-17F, and TNFα.

The present invention is a multi-target fusion protein comprising an IL-17A/IL-17RC ECD hybrid protein that may include a mutant as the moiety binding to both IL-17A and IL-17F, and a TNFR2 ECD that may include a mutant as the moiety that binds to TNFα. The IL-17A/IL-17RC ECD hybrid protein shares at least 95% sequence identity with the amino acid sequences of SEQ ID NOs: 66-69. In other words, these sequences may share at least 95% identity with IL-17RA/RC that contains mutants such as L9P, T24D, F59V, N88D, D122G, A156P, or Q267R. In addition, the TNFR2 ECD shares at least 95% sequence identity with the amino acid sequences of SEQ ID NOs: 83-93. These sequences may share at least 95% identity with TNFR2 ECD, which contains mutants such as S33A, S36P, G75S, R119S, E210G, or F219I.

Tne embodiment of the multi-target fusion protein provided by the present invention may have the following structural formulas:

N'-A-L1-B-L2-X-L3-C-C' (I)

or

N'-C-L1-X-L1-B-L3-A-C' (II)

In the above structural formulas (I) and (II), N' refers to the N-terminus of the fusion protein, and C' refers to its C-terminus. A, B, and C represent domains specific to the first antigen, the second antigen, and the third antigen, respectively. L1 to L3 represent peptide hinges or linkers consisting of 0-31, 2-10, or 3-10 amino acids, respectively. X is the Fc domain, which includes a natural Fc domain, a recombinant Fc domain, and a variant Fc domain. The Fc domain of the therapeutic protein may contribute to an extended half-life. The binding affinity to FcRN, enhanced through Fc engineering, can increase the half-life. The Fc variant, which includes a mutation to enhance affinity for FcRn or to minimize effector functions such as ADCC or CDC, may correspond to the Fc domain.

In the above structural formulas (I) and (II), A represents the first antigen-binding domain, which may be a human IL-17RA ECD polypeptide or IL-17RA ECD polypeptide variant. B represents the second antigen-binding domain, which may be a human IL-17RC ECD polypeptide or IL-17RC ECD polypeptide variant. The second antigen-binding domain may be specific to either the first antigen, IL-17A, and the second antigen, IL-17F, individually, or to both antigens simultaneously." (Rolf E. Kuestner et al., J. Immunol. 46(7): 1191-1199, 2007). IL-17RA exhibits high affinity for IL-17A but relatively low affinity for IL-17F. In some embodiments of the present invention, A is an IL-17RA ECD variant, and B is an IL-17RC ECD variant, both of which are capable of binding in a hybrid form characterized by a linker length of "0". Furthermore, the A domain of the IL-17A/IL-17RC ECD hybrid protein corresponds to exons 1-6 of IL-17RA (wild-type, SEQ ID NO: 114) and may form a polypeptide corresponding to exons 1-6 of the wild-type hIL-17RA, or a variant thereof. The B domain of the IL-17A/IL-17RC ECD hybrid protein corresponds to exons 8-16 of IL-17RC (wild-type, SEQ ID NO: 115) and may form a polypeptide corresponding to exons 8-16 of the wild-type hIL-17RC, or a variant thereof. In the structural formulas (I) and (II) above, IL-17RA/RC in each of A-L7-B and B-L3-A can be fused to the C-terminus or N-terminus of the Fc domain, either directly or via a linker, and shares at least 95% sequence identity with the amino acid sequences of SEQ ID NOs: 66-69. In the IL-17A/IL-17RC ECD hybrid protein provided by the present invention, the first antigen-binding domain, corresponding to the A domain, is IL-17RA, and the second antigen-binding domain, corresponding to the B domain, is IL-17RC. While the fusion protein consists of both domains combined in a hybrid form, A-L1-B or B-L3-A can bind to both the first antigen, IL-17A, and the second antigen, IL-17F. These antigen-binding domains are fusion domains with a novel form and function, capable of binding to IL-17A/F heterodimers. It should be noted, however, that the IL-17RA/RC provided by the present invention is not limited to the description provided above.

In the structural formulas (I) and (II) above, C, representing the third antigen-binding domain, includes the human TNFR2 ECD and can be fused to the C-terminus or N-terminus of the Fc domain, either directly or via a linker. The TNFR2 ECD provided by the present invention is characterized either by blocking inflammatory responses through neutralization of sTNFα or signal blockage of TNFR1/2 with sTNFα or tmTNFα or by minimizing side effects through beneficial anti-inflammatory effects via reverse signaling of tmTNFα. The TNFR2 ECD provided by the present invention is characterized by its ability to inhibit inflammatory responses either through the neutralization of sTNFα or the blockage of TNFR1/2 signaling by sTNFα or tmTNFα, or to minimize side effects by promoting beneficial anti-inflammatory effects via the reverse signaling of tmTNFα. The TNFR2 ECD corresponding to C in the structural formulas (I) and (II) above shares at least 95% sequence identity with the amino acid sequence of SEQ ID NO: 77.

The multi-target fusion protein provided in some embodiments of the present invention includes a dual-target fusion protein designed to prevent, diagnose, or treat diseases associated with the activity of IL-17A, IL-17F, and TNFα. The present invention, which relates to a fusion protein as described herein, simultaneously or sequentially binds to IL-17A, IL-17F, and TNFα, thereby inhibiting their activity. This inhibition suppresses the expression of cytokines and chemokines in immune or non-immune cells, including IL-8, IL-6, IL-1β, G-CSF, hBD2, DEFB4, IL-36γ, IL-17C, CCL20, CCL23, CXCL10, LCN2, MMP3, E-selectin, and RANTES, through the NF-κB or MAPK signaling pathways. Additionally, it induces the secretion of anti-inflammatory cytokines. Accordingly, the fusion protein offers therapeutic and preventive methods that enable rapid and sustained improvement of a pro-inflammatory disease milieu through this mechanism.

The fusion protein of the present invention is administered to patients with a variety of inflammatory, immunogenic, and proliferative diseases, including the following: psoriasis, rheumatoid arthritis, osteoarthritis, ankylosing spondylitis, axial spondyloarthritis, juvenile idiopathic arthritis, rheumatoid arthritis-associated osteoporosis, inflammatory fibrosis (e.g., scleroderma, pulmonary fibrosis, cirrhosis), gingivitis, periodontitis or other periodontal diseases, inflammatory bowel diseases (e.g., Crohn's disease, ulcerative colitis), asthma (including allergic asthma), allergies, chronic obstructive pulmonary disease (COPD), multiple sclerosis, and cancers.

The aforementioned TNFα and IL-17 are the primary cytokines responsible for causing inflammatory and autoimmune diseases. Five biological products, including the TNFα inhibitor Etanercept, which inhibits the biological activity of TNFα by binding to it, as well as Infliximab, Golimumab, Adalimumab, and Certolizumab pegol, have been approved and marketed. The therapeutic effectiveness of these antibodies varies depending on disease progression or the presence or absence of underlying conditions. All TNFα inhibitors have equivalent levels of neutralizing ability against sTNFα, but patient responses vary. Some patients do not respond to TNFα inhibitors, and cases of sustained therapeutic effectiveness are rare. Specifically, 30% of patients with autoimmune diseases, such as rheumatoid arthritis, do not benefit from the therapeutic effects of TNFα inhibitors. Even among patients who initially respond to TNFα inhibitors, a considerable number experience a decreased response rate over prolonged treatment or require a drug switch due to relapse (M H Buch et al. Rheumatology, 46, Issue 7, 1153-1156, 2007). Infections such as tuberculosis, as well as lymphoma and heart failure, have been reported as side effects of TNFα inhibitors. Notably, increased cardiovascular morbidity and mortality have been closely associated with the prolonged release of IL-17A and TNFα into the circulation in patients with RA-associated synovitis (Anass Bouchnita et al. C. R. Biol. 340(11-12): 456-473, 2017).

A clinical study on a TNFα inhibitor demonstrated significantly increased levels of circulating Th17 cells and IL-17 in patients with an inadequate response to the treatment. Based on the results of this study, a PoC study (Study No. NCT02430909) was conducted on a therapy targeting both IL-17 and TNFα simultaneously. When co-administering two different biological products, including the anti-IL-17A/F therapy Bimekizumab and the anti-TNFα therapy Certolzumab pegol, to patients who do not respond to a TNFα inhibitor, rapid remission as well as decreases in the swelling joint count (SJC) and tender joint count (TJC) were identified in the combination therapy-treated group, compared to the monotherapy-treated group. In patients with an inadequate response to a TNFα inhibitor, co-administration of the anti-IL-17A/IL-17F antibody Bimekizumab and the anti-TNFα antibody Certolzumab pegol resulted in rapid remission, along with significant reductions in swollen joint count (SJC) and tender joint count (TJC), compared to monotherapy. However, the combination therapy-treated group had higher infection risks and treatment-emergent adverse events (TEAEs) compared to the monotherapy- and placebo-treated groups, with a greater incidence of TEAEs classified under the SOC Skin and Subcutaneous Tissue Disorders (Sophie Glatt et al., Ann. Rheum. Dis. 78(8): 1033-1040, 2019).

Dual-target antibodies that simultaneously target both TNFα and IL-17, such as ABT-122, JNJ-8104, and COVA322, have been developed and evaluated in clinical studies for rheumatoid arthritis. In Phase 2 clinical studies, rapid remission was observed; however, it was challenging to determine a relative advantage of the anti-TNFα antibody, Adalimumab. A high level of anti-drug antibodies (ADAs) was observed. Further developments have not been pursued at this time (Study Nos. NCT01853033, NCT02141997, NCT02243787, NCT02349451, and NCT02758392).

Based on the description above, the co-administration of an anti-TNFα antibody with an anti-IL-17 antibody, or the development of dual-target antibodies that simultaneously target both TNFα and IL-17, have been achieved to date. For the co-administration of two different antibodies, the dose and dosing schedule may be flexible, potentially resulting in poor patient compliance and increased pain. The co-administration of biological products targeting cytokines involved in both innate immunity and pro-inflammatory autoimmune diseases may increase the risk of infection, making it challenging to achieving a low disease activity index. Consequently, this co-administration failed to provide a therapeutic benefit. A recently developed combination drug can also offer a certain degree of dose flexibility. However, finding formulation conditions that ensure the chemical and physical stability of both antibodies is challenging due to the differing molecular properties of the two drugs. In addition, the co-administration or use of combination drugs incurs additional expenses due to the use of two different drugs, thereby increasing medical costs for patients. Most dual-target antibodies designed to simultaneously target both TNFα and IL-17 have been reported to exhibit a high potential for immunogenicity (Mark A. Kroenke et al., Front. Immunol. 12:782788, 2021). Due to the significant impact of high immunogenicity on the efficacy, pharmacokinetics, and safety of dual-target antibodies, their development has been discontinued.

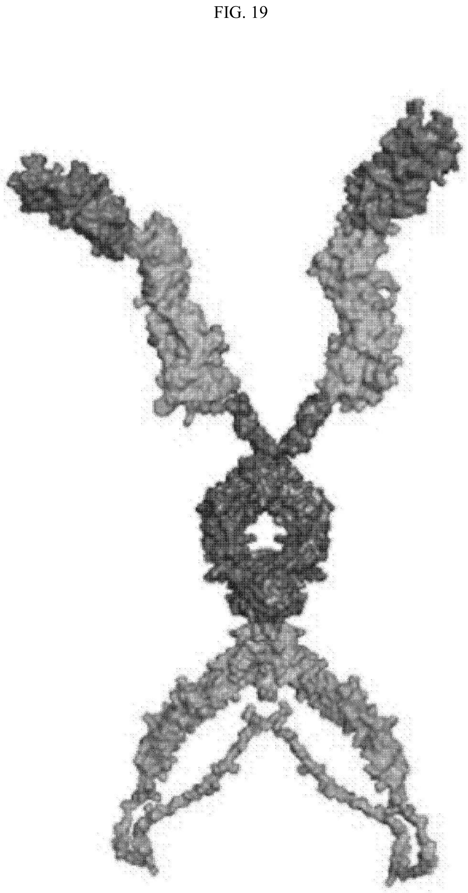

The multi-target fusion protein, produced according to one embodiment of the present invention, binds to IL-17A, IL-17F, and TNFα either simultaneously or sequentially, enabling the neutralization of all three cytokines. This fusion protein can effectively mitigate excessive inflammatory responses mediated by the synergistic stimulation of IL-17A, IL-17F, and TNFα, and improve an irreversible inflammatory disease environment. Furthermore, the low immunogenicity of the fusion protein allows its therapeutic effects to persist, thereby reducing the disease activity index and increasing the likelihood of achieving remission. A multi-target therapy alone is expected likely to reduce safety issues related to infection risks from the co-administration of single-target therapies and to lower the medical cost burden for patients with autoimmune diseases (see FIG. 19).

The pharmaceutical compositions provided by the present invention may vary depending on several factors, including the type of lesion, application site, number of treatments, treatment duration, dosage form, patient's condition, and type of adjuvant. The dosage is not specifically limited but may range from 0.01 µg/kg/day to 10 mg/kg/day. The aforementioned daily dose may be administered once daily or two to three times daily at appropriate intervals. Intermittent dosing over several days is also possible.

The compounds in the pharmaceutical composition of the present invention can be administered orally or via a parenteral route. Parenteral administration, such as intravenous, subcutaneous, intracerebroventricular, intracerebrospinal, intramuscular, or intraperitoneal infusion, is preferable.

The pharmaceutical compositions of the present invention may include more carriers, excipients, and diluents than those typically used in the manufacture of pharmaceutical compositions. In addition, solid or liquid additives for formulation may be used in the manufacture of pharmaceutical compositions. Additives for formulation can be either organic or inorganic. Lactose, sucrose, white sugar, glucose, cornstarch, starch, talc, sorbitol, crystalline cellulose, dextrin, kaolin, potassium carbonate, and silicon dioxide are used as excipients. Examples of binders include polyvinyl alcohol, polyvinyl ether, ethyl cellulose, methyl cellulose, gum Arabic, tragacanth, gelatin, shellac, hydroxypropyl cellulose, hydroxypropyl methylcellulose, calcium citrate, dextrin, and pectin. Magnesium stearate, talc, polyethylene glycol, silica, and hydrogenated vegetable oil are used as lubricants. Any colorants approved as additives for pharmaceutical products can be used. Tablets and granules of the pharmaceutical compositions can be adequately coated, as needed, with a sugar coating or a gelatin coating. Preservatives or antioxidants can be used as needed. If the pharmaceutical composition is a medication, one or more of the following additives may also be included: fillers, anti-caking agents, lubricants, humectants, fragrances, emulsifiers, or preservatives. The dosage form of the pharmaceutical composition in the present invention may be preferable depending on the method of use. In particular, it is preferable to formulate the pharmaceutical compositions using methods well known in the pharmaceutical industry to provide rapid, sustained, or delayed release of the active ingredient after administration to mammals. The pharmaceutical compositions may be formulated in any of the following dosage forms: plasters, granules, lotions, liniments, lemonades, powders, syrups, liquids and solutions, aerosols, extracts, elixirs, fluid extracts, emulsions, suspensions, decoctions, infusions, tablets, suppositories, injections, spirits, cataplasms, capsules, troches, tinctures, pastes, pills, or soft or hard gelatin capsules.

Ingredients commonly used in the pharmaceutical compositions of the present invention may also be included. For example, adjuvants such as stabilizers, solubilizing agents, and fragrances, and carriers may be added.

The compositions, in one embodiment of the present invention, may include pharmaceutically acceptable carriers, as well as pharmaceutically acceptable adjuvants, excipients, or diluents other than the carriers mentioned above.

The term "Pharmaceutically acceptable", as used herein, refers to a physiologically acceptable composition that does not cause gastrointestinal disorders, allergic reactions (such as vertigo), or similar adverse effects when administered to humans. Examples of the aforementioned carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils. Fillers, anti-caking agents, lubricants, humectants, fragrances, emulsifiers, and preservatives may also be added.

The compositions described in one embodiment of the present invention may be formulated using methods well known in the pharmaceutical industry to provide rapid, sustained, or delayed release of the active ingredient in various dosage forms, including powders, granules, tablets, emulsions, syrups, aerosols, soft or hard gelatin capsules, sterile water for injection, and sterile powders.

The compositions described in one embodiment of the present invention may be administered via various routes, including oral administration and parenteral routes such as suppositories or subcutaneous, intravenous, intraperitoneal, intramuscular, intralesional, intranasal, and intrathecal infusions. In addition, implantable devices may be used for extended, sustained, or repeated release. The dosing frequency may range from once to several times daily, as desired, and the treatment duration is not specifically limited.

The compositions described in one embodiment of the present invention may be typically administered via systemic or local routes, including intramuscular or intravenous injections. However, compositions containing polynucleotides or expression vectors comprising them are preferably infused using an electroporator.

The compositions described in one embodiment of the present invention may be administered via any typical route that can reach the target tissue. Such routes of administration may include, but are not limited to the following parenteral routes: intraperitoneal, intravenous, intramuscular, subcutaneous, and intrasynovial infusions.

The compositions described in one embodiment of the present invention may be formulated in suitable forms with commonly used pharmaceutically acceptable carriers. These carriers include parenteral carriers such as water, suitable oils, saline, aqueous glucose, and glycol. Stabilizers and preservatives may also be included in the formulations. Sodium hydrogen sulfite is used as a stabilizer and an antioxidant for sodium sulfite or ascorbic acid. Preservatives such as benzalkonium chloride, methylparaben, propylparaben, and chlorobutanol are appropriate. The following additives may be appropriately used, as needed, depending on the route of administration or dosage form: suspending agents, solubilizing agents, stabilizers, isotonic agents, preservatives, antiadherents, surfactants, diluents, excipients, pH modifiers, pain relief agents, buffering agents, and antioxidants. Pharmaceutically acceptable carriers and formulations suitable for the present invention, including the aforementioned additives, are detailed in the literature (Remington's Pharmaceutical Sciences, latest edition).

The dosage of the compositions described in one embodiment of the present invention may vary depending on various factors, including the patient's height, body surface area, age, specific compound administered, sex, dosing schedule, and route of administration, health condition, and concomitant medications. The compositions of the present invention may be administered at doses ranging from 100 µg/kg (body weight) to 10 mg/kg, with more preferable doses ranging from 1 µg/kg to 1 mg/kg and the most preferable does ranging from 5 to 500 µg/kg. The dosage may be adjusted based on the factors described above. The pharmaceutical compositions of the present invention are administered at therapeutically effective doses.

The term "therapeutically effective dose", as used herein, refers to an amount sufficient to treat a disease while maintaining a reasonable benefit-risk ratio applicable to the medical treatment. The effective dose level may be determined based on various factors, including species, severity of the condition, age, sex, drug activity, sensitivity to the drug, dosing schedule, route of administration, excretion rate, treatment duration, concomitant medications, and other factors well known in the medical field. The pharmaceutical compositions of the present invention may be administered at doses ranging from 0.1 mg/kg to 1 g/kg, with more preferable doses ranging from 1 mg/kg to 500 mg/kg. The dosage mentioned above may be appropriately adjusted based on the patient's age, sex, and health condition.

The compositions of the present invention may be administered orally or via a parenteral route, including any systemic or local route of administration. The systemic routes of administration include intravenous, intraperitoneal, and intramuscular injection, while the local routes of administration include intracranial, intracerebrospinal, and subcutaneous injection.

### Mode for Invention

A more detailed explanation of the present invention is provided through the embodiments described below. It should be noted that the present invention is not limited to the embodiments disclosed below but may be embodied in various different forms. The embodiments described below are provided to complete the disclosure of the present invention and to fully inform a person of ordinary skill in the pertinent art of the scope of the invention.

### Example 1: Preparation of IL-17A and IL-17F Antagonists

The IL-17RA/RC-Fc protein was prepared to obtain an antagonist capable of effectively neutralizing IL-17AA, IL-17AF, or IL-17FF. Particularly, antagonists that bind to IL-17A and IL-17F, either simultaneously or sequentially, may more effectively treat autoimmune and inflammatory diseases (Sascha Gerdes & Joerg Albrecht., Br. J. Dermatol. Volume 186, Issue 4, 2022). IL-17RC is capable of binding to the ligands IL-17F and IL-17A with high affinity (Rolf E. Kuestner et al., J. Immunol. 179(8): 5462-5473, 2007). An IL-17RA/RC hybrid protein, included in the IL-17RA/RC-Fc protein, utilized a protein segment from IL-17RA and IL-17RC ECDs, associated with binding affinity for IL-17A or IL-17F, as a functional fragment. The IL-17RA/RC-Fc protein as a hybrid protein was prepared by combining functional fragments from IL-17RA and IL-17RC. In addition, back-to-consensus mutations were introduced into the functional fragments of IL-17RA and IL-17RC to enhance binding affinity for IL-17A and IL-17F, improve the stability of hybrid proteins, and ensure low immunogenicity. For IL-17RA/RC variants, the following mutations may be introduced: proline substituted for leucine at position 9, aspartate substituted for threonine at position 24, tyrosine substituted for histidine at position 52, valine substituted for phenylalanine at position 59, lysine substituted for glutamic acid at position 78, aspartic acid substituted for asparagine at position 88, isoleucine substituted for phenylalanine at position 89, lysine substituted for arginine at position 108, glycine substituted for aspartic acid at position 122, proline substituted for alanine at position 156, or arginine substituted for glutamic acid at position 267.

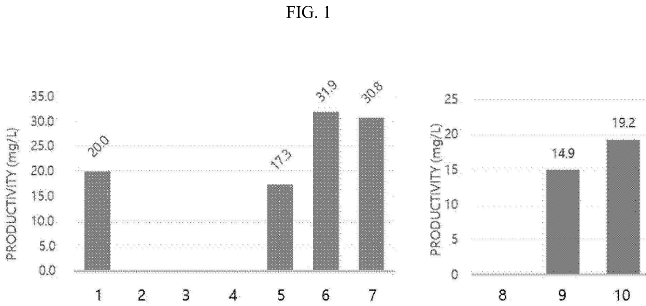

IL-17RA/RC hybrid protein variants and variants of the tri-specific fusion protein IL-17RA/RC-Fc-TNFR2 were prepared to identify mutations that enhance affinity, stability, and productivity, with wild-type IL-17RA/RC hybrid proteins serving as the control group. Embodiment 2 of the present invention relates to an experiment aimed at selecting mutations that either have no impact on the affinity, stability, and productivity of the IL-17RA/RC hybrid protein or improve these properties after adding a moiety targeting TNFα to the IL-17RA/RC hybrid. As presented in Table 1, the IL-17RA and IL-17RC constructs significantly enhanced productivity when various mutations were introduced, compared to the control group, wtIL-17RA/RC or wtIL-17RA/RC-Fc variants (see FIG. 1). Additionally, the stability, ligand affinity, and neutralizing ability improved significantly (see Table 1). The complete sequence information of the fusion protein is provided in Table 1. Information on the mutations in the IL-17RA/RC hybrid protein introduced into the IL-17RA/RC-Fc or IL-17RA/RC-Fc-TNFR2 constructs is summarized in Tables 2 and 3, respectively.

**Table 1: Complete sequence information of the fusion protein in one embodiment of the present invention**

| **No.** | **Name** | **Structure** | **SEQ ID NO** |
|---|---|---|---|
| 1 | T1-T4-01 | T1Rf-Fc-T4wt | 1 |
| 2 | T1-T4-05 | T1a-Fc-T4wt | 2 |
| 3 | T1-T4-06 | T1b-Fc-T4wt | 4 |
| 4 | T1-T4-07 | T1c-Fc-T4wt | 6 |
| 5 | T1-T4-08 | T1c-Fc-T4a | 8 |
| 6 | T 1-T4-09 | T1c-Fc-T4b | 10 |
| 7 | T1-T4-10 | T1c-Fc-T4c | 12 |
| 8 | T1-T4-11 | T1c-Fc-T4d | 14 |
| 9 | T1-T4-12 | T1c-Fc-T4e | 16 |
| 10 | T1-T4-13 | T1c-Fc-T4f | 18 |
| 11 | T1-T4-14 | T1c-Fc-T4g | 20 |
| 12 | T1-T4-30 | T1c-Fc-T4h | 22 |
| 13 | T1-T4-31 | T1c-Fca-T4g | 24 |
| 14 | T1-T4-32 | T1c-Fcb-T4g | 26 |
| 15 | T1-T4-33 | T1c-Fc-T4i | 28 |
| 16 | T1-T4-TANDEM | T1c-Fc-T4TR | 30 |
| 17 | T1-T4-35 | T1c-Fc-mT4m | 32 |

**Table 2: Information on IL-17RA/RC mutations**

| **No.** | **Construct** | **IL-17RA/RC mutation** |
|---|---|---|
| 1 | IL-71RA/RC-Fc (wt) | |
| 2 | IL-71RA/RC-Fc | Q267R |
| 3 | IL-71RA/RC-Fc | T24D/N88D/Q267R |
| 4 | IL-71RA/RC-Fc | T24D/N88D/D 122G/Q267R |
| 5 | IL-71RA/RC-Fc | T24D/F59V/N88D/D 122G/Q267R |
| 6 | IL-71RA/RC-Fc | L9P/T24D/F59V/N88D/D 122G/Q267R |
| 7 | IL-71RA/RC-Fc | L9P/T24D/F59V/N88D/D122G/A156P/Q267R |
| 8 | IL-71RA/RC-Fc | Q267R |
| 9 | IL-71RA/RC-Fc | L9P/T24D/F59V/N88D/D 122G/Q267R |
| 10 | IL-71RA/RC-Fc | L9P/T24D/F59V/N88D/D122G/A156P/Q267R |

PCR was performed in order to induce protein expression in IL-17RA/RC-Fc after preparing a pair of primers for each construct. Recombinant vectors were generated using pBispec vectors (Genexine, Inc., Republic of Korea). Expression vectors were prepared by linking biologically active proteins or the API IL-17RA/RC to the N-terminus of the Fc to produce the IL-17RA/RC-Fc fusion protein of the present invention through a linker peptide. Expression of the IL-17RA/RC-Fc fusion protein was performed using ExpiCHO^{™} (Gibco, Cat# A29127) cells, in accordance with the protocol of ExpiCHO^{™} expression system kit. In order to prepare the fusion protein, the ExpiCHO^{™} cells were incubated in ExpiCHO^{™} expression media (Gibco, Cat# A29100-01) for one day at 37°C with 8% CO₂ at 120 rpm. The cultivated cells ware diluted with a density of 0.6-1.0 × 10⁷ cells/mL on the day of DNA transfection and a survival rate of 95% or higher, to 1.0 × 10⁷ cells/mL using fresh media. And then, cold OptiPRO^{™} SFM^{®} (Gibco, Cat: 12309019) medium was dispensed into each well, and the appropriately prepared DNA and ExpiFectamine^{™} CHO reagent were each added, mixed, and incubated at room temperature for 5 minutes. This mixture was then added to the host cells for transfection, after which the transfected cells were cultured. Eighteen hours after transfection, the enhancer and feed included in the ExpiFectamine^{™} CHO Transfection Kit were added to the transfected cells. The cells were then cultured under conditions of 8% CO₂, 37°C, and 120 rpm for 7 to 10 days to complete the production of the recombinant protein. After cultivation, the cell culture was centrifuged at 4°C and 4000 rpm for 60 minutes. The supernatant was then separated and stored frozen at -80°C.

### Example 2: Preparation of IL-17A, IL-17F, and TNFα Antagonists

The pro-inflammatory cytokines IL-17 and TNFα are therapeutic targets for various chronic inflammatory diseases. The synergistic stimulation of IL-17 and TNFα activates MAPK and NFκB, creating a pro-inflammatory environment that leads to serial tissue damage. In particular, chronic drug therapy for severe autoimmune diseases may activate compensatory mechanisms that inhibit certain activities and cause unwanted toxicity. Simultaneously targeting the inflammatory response pathways of both IL-17 and TNFα is necessary to address these issues. A TNFα-binding moiety with enhanced affinity for TNFα compared to wtTNFR2 was used to prepare antagonists capable of simultaneously inhibiting or neutralizing IL-17A, IL-17F, and TNFα. Mutations selected from the back-to-consensus library were transfected into wtTNFR2 (see Table 3). The tri-specific fusion protein IL-17RA/RC-Fc-TNFR2 was generated by linking the IL-17RA/RC hybrid protein, described in Embodiment 1, and the TNFR2 variant to the N-terminus and C-terminus of the Fc domain, respectively, via linkers.

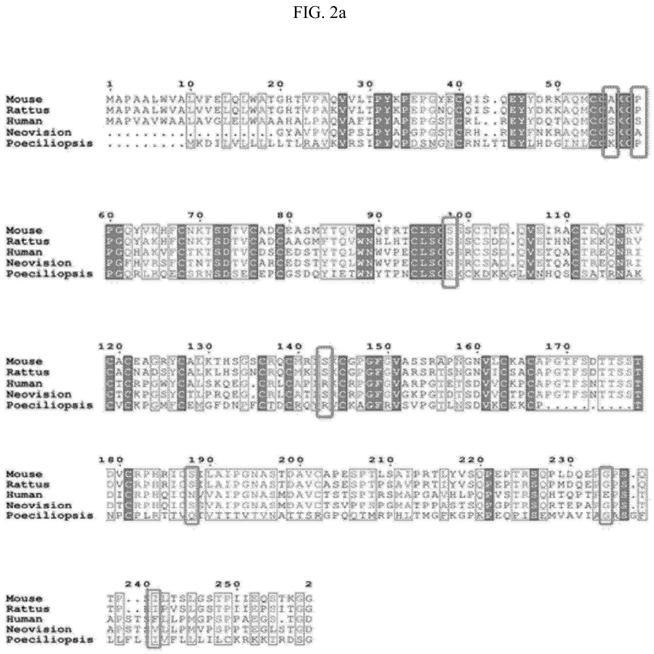

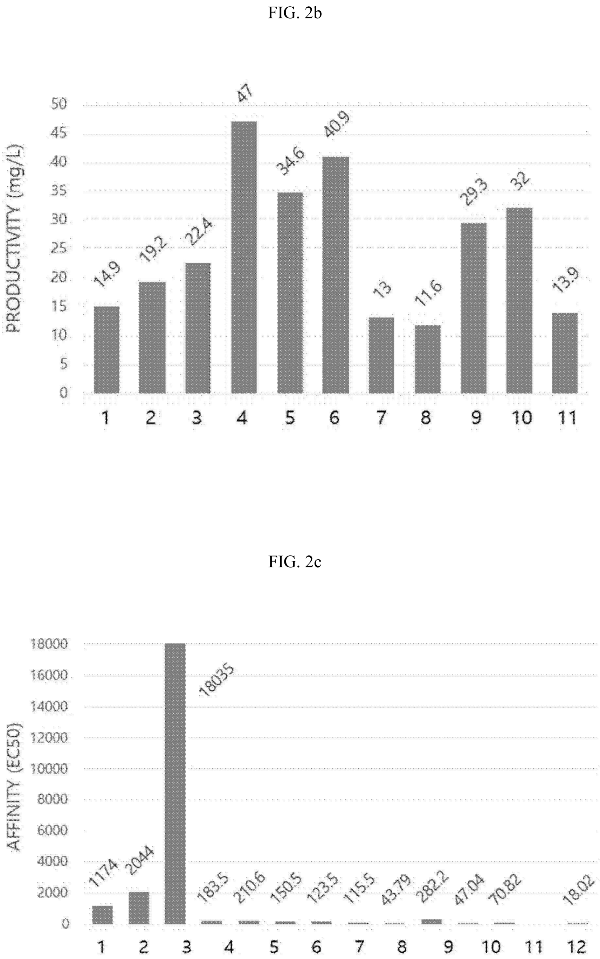

To improve the stability, productivity, and neutralizing ability of the TNFR2 ECD variant, as an embodiment of the present invention, the positions of mutations were selected by searching and aligning interspecies sequences of 258 amino acids in the TNFR2 ECD, including signal peptides, using the NCBI database. Accordingly, the mutations S33A, S36P, G75S, R119S, N164S, E210G, F219I, and various combinations thereof were selected as superior variants to the wild type based on assessments of thermal stability, neutralizing ability, and productivity (see FIG. 2a). The TNFR2 variant T4i (SEQ ID NO: 92), listed as No. 10 in Table 3, is a variant in which the C-terminus of wtTNFR2 was partially deleted. The TNFR2 variant T4TR (SEQ ID NO: 93), listed as No. 11 in the same table, is a variant in which TNFR2 was additionally linked via a linker and exhibits decreased productivity compared to other variants (see FIG. 2b).

The improvement in the neutralizing ability of the TNFR2 variant for TNFα was assessed by comparing the affinity (EC₅₀ value) obtained from the ELISA analysis (see FIG. 2c). The neutralizing ability of the TNFR2 ECD variant with introduced mutations was mostly improved compared to the wild-type TNFR2 ECD. The TNFR2 variant T4g (SEQ ID NO: 77), listed as No. 8 in Table 3, exhibited a 40-fold increase in neutralizing ability compared to the wild-type TNFR2 ECD. The neutralizing ability was found to be equivalent to that of the positive control, Adalimumab (see No. 2 in FIG. 2c). The mutations introduced into the TNFR2 ECD variant, which resulted in increases in both neutralizing ability and productivity, were S33A, S36P, G75S, R119S, N164S, E210G, and F219I. The mutations introduced into the TNFR2 variant T4g (SEQ ID NO: 77) were S33A, S36P, G75S, R119S, N164S, E210G, and F219I. Table 3 provides information on the TNFR2 mutations introduced into the IL-17RA/RC-Fc-TNFR2 construct. For the TNFR2 variant, the following mutations may be introduced: alanine substituted for serine at position 33, proline substituted for serine at position 36, serine substituted for glycine at position 75, serine substituted for arginine at position 119, serine substituted for asparagine at position 164, glycine substituted for glutamic acid at position 210, or isoleucine substituted for phenylalanine at position 219.

**Table 3: Information on TNFR2 mutations**

| **No.** | **Construct** | **TNFR2 mutation** |
|---|---|---|
| 1 | T4 wt | Wild type |
| 2 | T4a | S33A |
| 3 | T4b | S33A/S36P |
| 4 | T4c | S33A/S36P/R119S |
| 5 | T4d | S33A/S36P/R119S/N164S |
| 6 | T4e | S33A/S36P/R119S/N164S/E210G |
| 7 | T4f | S33A/S36P/R119S/N164S/E210G/F219I |
| 8 | T4g | S33A/S36P/G75S/R119S/N164S/E210G/F219I |
| 9 | T4h | G75S |
| 10 | T4i | S33A/S36P/G75S/R119S/N164S |
| 11 | T4TR | S33A/S36P/G75S/R119S/N164S/E210G/F219I-linker3-S33A/S39P/G75S/R119S/N164S |

The tri-specific fusion protein IL-17RA/RC-Fc-TNFR2, provided in the present invention was prepared by linking the IL-17RA/RC variants and TNFR2 variants, as prepared according to Embodiment 1, using various Fc domains and linkers. The tri-specific fusion protein of the present invention may have the following structural formulas (I) and (II):

N'-A-L1-B-L2-X-L3-C-C' (I)

or

N'-C-L1-X-L1-B-L3-A-C' (II)

In the above structural formulas (I) and (II), N' refers to the N-terminus of the fusion protein, and C' refers to its C-terminus. A and B are specific domains of the IL-17RA ECD and IL-17RC ECD, respectively, with each domain corresponding to a fragment of a protein that binds to the ligands IL-17A or IL-17F as a functional unit. However, A-L1-B or B-L3-A can bind to both the first antigen, IL-17A, and the second antigen, IL-17F, as well as to IL-17A/F heterodimers. L1 in A-L1-B is a linker that may consist of 0-31 amino acids, with '0' being applicable in the case of IL-17RA/RC. IL-17RA/RC may be located at the N-terminus of the wild-type Fc domain or its variant using the linker L2. In the structural formulas (I) and (II) described above, C is specific to the third antigen TNFα and may be located at the C-terminus of the wild-type Fc domain or its variant using the linker L3.

The linkers used in the fusion protein IL-17RA/RC-Fc-TNFR2 described in the present invention are listed in Table 4. The Fc domain introduced into the aforementioned fusion protein refers to the Fc domain proteins of recombinant antibodies, prepared by combining all or part of the hinge, CH2, and CH3 regions that constitute the Fc domain of an immunoglobulin (Ig) with derivatives from different types of antibody molecules, such as IgG, IgD, IgE, or IgM. It may be one of the hyFc domain proteins (e.g., WO2008147143A2, WO2020102728A1, WO2021046404A1), which are examples of modified IgG4 immunoglobulins. Table 5 provides examples of the Fc domains described in the present invention.

To ensure a prolonged half-life, mutations that increase Fc affinity for FcRn under endosomal conditions were introduced into the Fc variants listed in Table 5. Fc engineering was used to extend the retention time of monoclonal antibody drugs in the body (Atsuhiko Maeda et al., Mabs. 9(5):844-853, 2017). Each of the following mutation sets, in which Fc engineering was applied, or a combination thereof, may be utilized: LS mutation (M428L/N434S, Xencor), YTE mutation (M252Y/S254T/T256E, Medimmune), QL mutation (T250Q/M428L) (Paul R. Hinton et al., J. Biol. Chem. 279(8): 6213-6216, 2004), or KF mutation (H433K/N434F). Table 4 summarizes the linker sequences of the tri-specific fusion protein IL-17RA/RC-Fc-TNFR2.

**Table 4: Information on the linker sequences of IL-17RA/RC-Fc-TNFR2**

| **Linker** | **Amino Acid Sequence** | **SEQ ID NO** |
|---|---|---|
| L1 | TGIEGRMD | 90 |
| L2 | GGGGSGGGGSGGGGSLE | 89 |
| L 3 | TGKLSGSASAPKLEEGEFSEARV | 91 |
| L4 | KLSGSASAPKLEEGEFSEARVLE | 92 |
| L5 | TGGSGEGEGSEGSG | 93 |
| L6 | GSGEGEGSEGSGLE | 94 |

**Table 5: Information of Fc sequences**

| **Name** | **Fc Sequence** | **SEQ ID NO** |
|---|---|---|
| hyFc (1^{st} generation) | RNTGRGGEEKKKEKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 33 |
| hyFc1+YTE | RNTGRGGEEKKKEKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 34 |
| hyFc1+KF | RNTGRGGEEKKKEKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 35 |
| hyFc1+YTE+KF | RNTGRGGEEKKKEKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 36 |
| hyFc1+LS | RNTGRGGEEKKKEKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVLHE ALHSHYTQKSLSLSLGK | 37 |
| hyFc1+YTE+LS | RNTGRGGEEKKKEKEKEEQEERETKTPECPSHTQPLGV FLFPPKPKDTLYITREPEVTCVVVDVSQEDPEVQFNWY DGVEVHNAKTKPREEQFNSTYRVVSVLTVLHQDWLNG KEYKCKVSNKGLPSSIEKTISKAKGQPREPQVYTLPPSQ EEMTKNQVSLTCLVKGFYPSDIAVEWESNGQPENNYKT TPPVLDSDGSFFLYSRLTVDKSRWQEGNVFSCSVLHEA LHSHYTQKSLSLSLGK | 38 |
| hyFc (2^{nd} generation) GG | RNTGRGGEEKKGGKEKEEQEERETKTPECPSHTQPLG VFLFPPKPKDTLMISRTPEVTCVVVDVSQEDPEVQFNW YVDGVEVHNAKTKPREEQFNSTYRVVSVLTVLHQDWL NGKEYKCKVSNKGLPSSIEKTISKAKGQPREPQVYTLPP SQEEMTKNQVSLTCLVKGFYPSDIAVEWESNGQPENNY KTTPPVLDSDGSFFLYSRLTVDKSRWQEGNVFSCSVNH EALHNHYTQKSLSLSLGK | 39 |
| hyFc (2^{nd} generation) GS | RNTGRGGEEKKGSKEKEEQEERETKTPECPSHTQPLGV FLFPPKPKDTLMISRTPEVTCVVVDVSQEDPEVQFNWY VDGVEVHAKTKPREEQFNSTYRVVSVLTVLHQDWLNG KEYKCKVSNKGLPSSIEKTISKAKGQPREPQVYTLPPSQ EEMTKNQVSLTCLVKGFYPSDIAVEWESNGQPENNYKT TPPVLDSDGSFFLYSRLTVDKSRWQEGNVFSCSVNHEA LHNHYTQKSLSLSLGK | 40 |
| hyFc (2^{nd} generation) SG | RNTGRGGEEKKSGKEKEEQEERETKTPECPSHTQPLGV FLFPPKPKDTLMISRTPEVTCVVVDVSQEDPEVQFNWY VDGVEVHNAKTKPREEQFNSTYFVVSVLTVLHQDWLN GKETKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 41 |
| hyFc (2^{nd} generation) SS | RNTGRGGEEKKSSKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 42 |
| Mouse Fc | VPRDCGCKPCICTVPEVSSVFIFPPKPKDVLTITLTPKVT CVVVDISKDDPEVQFSWFVDDVEVHTAQTQPREEPFNS TFRSVSELPIMHQDWLNGKEFKCRVNSAAFPAPIEKTIS KTKGRPKAPQVYTIPPPKEQMAKDKVSLTCMITDFFPE DITVEWQWNGQPAENYKNTQPIMNTNGSYFVYSKLNV QKSNWEAGNTFTCSVLHEGLHNHHTEKSLSHSPGK | 43 |
| IgG1 | EPKSCDKTHTCPPCPAPELLGGPSVFLFPPKPKDTLMISR TPEVTCVVVDVSHEDPEVKFNWYVDGVEVHNAKTKP REEQYNSTYRVVSVLTVLHQDWLNGKEYKCKVSNKA LPAPIEKTISKAKGQPREPQVYTLPPSRDELTKNQVSLTC LVKGFYPSDIAVEWESNGQPENNYKTTPPVLDSDGSFFL YSKLTVDKSRWQQGNVFSCSVMHEALHNHYTQKSLSL SPGK | 44 |
| IgG4 | THTCPPCPSHTQPLGVFLFPPKPKDTLMISRTPEVTCVV VDVSQEDPEVQFNWYVDGVEVHNAKTKPREEQFNST YRVVSVLTVLHQDWLNGKEYKCKVSNKGLPSSIEKTIS KAKGQPREPQVYTLPPSQEEMTKNQVSLTCLVKGFYPS DIAVEWESNGQPENNYKTTPPVLDSDGSFFLYSRLTVD KSRWQEGNVFSCSVMHEALHNHYTQKSLSLSLGK | 45 |
| hyFc2 (GG)+YTE | RNTGRGGEEKKGGKEKEEQEERETKTPECPSHTQPLG VFLFPPKPKDTLYITREPEVTCVVVDVSQEDPEVQFNW YVDGVEVHNAKTKPREEQFNSTYRVVSVLTVLHQDWL NGKETKCKVSNKGLPSSIEKTISKAKGQPREPQVYTLPP SQEEMTKNQVSLTCLVKGFYPSDIAVEWESNGQPENNY KTTPPVLDSDGSFFLYSRLTVDKSRWQEGNVFSCSVMH EALHNHYTQKSLSLSLGK | 46 |
| hyFc2 (GG)+KF | RNTGRGGEEKKGGKEKEEQEERETKTPECPSHTQPLG VFLFPPKPKDTLMISRTPEVTCVVVDVSQEDPEVQFNW YVDGVEVHNAKTKPREEQFNSTYRVVSVLTVLHQDWL NGKEYKCKVSNKGLPSSIEKTISKAKGQPREPQVYTLPP SQEEMTKNQVSLTCLVKGFYPSDIAVEWESNGQPENNY KTTPPVLDSDGSFFLYSRLTVDKSRWQEGNVFSCSVMH EALKFHYTQKSLSLSLGK | 47 |
| hyFc2 (GG)+YTE+KF | RNTGRGGEEKKGGKEKEEQEERETKPECPSHTQPLGVF LFPPKPKDTLYITREPEVTCVVVDVSQEDPEVQFNWYV DGVEVHNAKTKPREEQFNSTYRVVSVLTVLHQDWLNG KEYKCKVSNKGLPSSIEKTISKAKGQPREPQVYTLPPSQ EEMTKNQVSLTCLVKGFYPSDIAVEWESNGQPENNYKT TPPVLDSDGSFFLYSRLTVDKSRWQEGNVFSCSVMHEA LKFHYTQKSLSLSLGK | 48 |
| hyFc2 (GG)+LS | RNTGRGGEEKKGGKEKEEQEERETKTPECPSHTQPLG VFLFPPKPKDTLMISRTPEVTCVVVDVSQEDPEVQFNW YVDGVEVHNAKTKPREEQFNSTYRVVSVLTVLHQDWL NGKEYKCKVSNKGLPSSIEKTISKAKGQPREPQVYTLPP SQEEMTKNQVSLTCLVKGFYPSDIAVEWESNGQPENNY KTTPPVLDSDGSFFLYSRLTVDKSRWQEGNVFSCSVLH EALHSHYTQKSLSLSLGK | 49 |
| hyFc2 (GG)+YTE+LS | RNTGRGGEEKKGGKEKEEQEERETKTPECPSHTGPLG VFLFPPKPKDTLYITREPEVTCVVVDVSQEDPEVQFNW YVDGVEVHNAKTKPREEQFNSTYRVVSVLTVLHQDWL NGKEYKCKVSNKGLPSSIEKTISKAKGQPREPQVYTLPP SQEEMTKNQVSLTCLVKGFYPSDIAVEWESNGQPENNY KTTPPVLDSDGSFFLYSRLTVDKSRWQEGNVFSCSVLH EALHSHYTQKSLSLSLGK | 50 |
| hyFc2 (GS)+YTE | RNTGRGGEEKKGSKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 51 |
| hyFc2 (GS)+KF | RNTGRGGEKKGSKEKEEQEERETKTPECPSHTQPLGVF LFPPKPKDTLMISRTPEVTCVVVDVSQEDPEVQFNWYV DGVEVHNAKTKPREEQFNSTYRVVSVLTVLHQDWLNG KEYKCKVSNKGLPSSIEKTISKAKGQPREPQVYTLPPSQ EEMTKNQVSLTCLVKGFYPSDIAVEWESNGQPENNYKT TPPVLDSDGSFFLYSRLTVDKSRWQEGNVFSCSVMHEA LKFHYTQKSLSLSLGK | 52 |
| hyFc2 (GS)+YTE+KF | RNTGRGGEEKKGSKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 53 |
| hyFc2 (GS)+LS | RNTGRGGEEKKGSKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVLHE ALHSHYTQKSLSLSLGK | 54 |
| hyFc2 (GS)+YTE+LS | RNTGRGGEEKKGSKEKEEQEERETKTPECPSHTQPLGV FLFPPKPKDTLYITREPEVTCVVVDVSQEDPEVQFNWY VDGVEVHNAKTKPREEQFNSTYFVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVLHE ALHSHYTQKSLSLSLGK | 55 |
| hyFc2 (SG)+YTE | RNTGRGGEEKKSGKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 56 |
| hyFc2 (SG)+KF | RNTGRGGEEKKSGKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 57 |
| hyFc2 (SG)+YTE+KF | RNTGRGGEEKKSGKEKEEQEERETKTPECPSHTQPLGV FLFPPKPKDTLYITREPEVTCVVVDVSQEDPEVQFNWY VDGVEHVNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 58 |
| hyFc2 (SG)+LS | RNTGRGGEEKKSGKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNYFSCSVLHE ALHSHYTQKSLSLSLGK | 59 |
| hyFc2 (SG)+YTE+LS | RNTGRGGEEKKSGKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMITNQVSLTCLVKGFYPSDIAVEWESNGQPENNYK TTPPVLDSDGSFFLYSRLTVDKSRWQEGNVFSCSVLHE ALHSHYTQKSLSLSLGK | 60 |
| hyFc2 (SS)+YTE | RNTGRGGEEKKSSKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALHNHYTQKSLSLSLGK | 61 |
| hyFc2 (SS)+KF | RNTGRGGEEKKSSKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 62 |
| hyFc2 (SS)+YTE+KF | RNTGRGGEEKKSSKEKEEQEERETKTPECPSHTQPLGV FLFPPKPKDTLYITREPEVTCVVVDVSQEDPEVQFNWY VDGVEVHNAKTKPREEQFNSTYRVVSVLVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVMHE ALKFHYTQKSLSLSLGK | 63 |
| hyFc2 (SS)+LS | RNTGRGGEEKKSSKEKEEQEERETKTPECPSHTQPLGV FLFPPKPKDTLMISRTPEVTCVVVDVSQEDPEVQFNWY VDGVEVHNAKTKPREEQFNSTYRVVSVLTVLHQDWLG KEYKCKVSNKGLPSSIEKTISKAKGQPREPQVYTLPPSQ EEMTKNQVSLTCLVKGFYPSDIAVEWESNGQPENNYKT TPPVLDSDGSFFLYSRLTVDKSRWQEGNVFSCSVLHEA LHSHYTQKSLSLSLGK | 64 |
| hyFc2 (SS)+YTE+LS | RNTGRGGEEKKSSKEKEEQEERETKTPECPSHTQPLGV FLFPPKPKDTLYITREPEVTCVVVDVSQEDPEVQFNWY VDGVEVHNAKTKPREEQFNSTYRVVSVLTVLHQDWLN GKEYKCKVSNKGLPSSIEKTISKAKGQPREPQVYTLPPS QEEMTKNQVSLTCLVKGFYPSDIAVEWESNGQPENNYK TTPPVLDSDGSFFLYSRLTVDKSRWQEGNVFSCSVLHE ALHSHYTQKSLSLSLGK | 65 |

Various constructs were generated to prepare the fusion protein IL-17RA/RC-Fc-TNFR2 of the present invention. In these constructs, IL-17RA/RC variants and TNFR2 variants were linked to the Fc variants (listed in Table 5) in the order from the N-terminus to the C-terminus, using the linkers listed in Table 4. Mutations with high affinity for FcRn were introduced into the Fc domains in the IL-17RA/RC hybrid protein (see Table 6). The applicable nucleic acid was cloned into a pBispec vector. Table 6 provides details on the IL-17RA/RC sequences of the fusion protein IL-17RA/RC-Fc-TNFR2. The detailed sequences of the TNFR2 variants are summarized in Table 7.

**Table 6: Information on IL-17RA/RC sequences**

| **Name** | **IL-17RA/RC Sequence** | **SEQ ID NO** |
|---|---|---|
| T1Rf | MGAARSPPSAVPGPLLGLLLLLLGVLAPGGASRLLDHRA LVCSQPGLNCTVKNSTCLDDSWIHPRNLTPSSPKDLQIQL HFAHTQQGDLFPVAHIEWTLQTDASILYLEGAELSVLQL NTNERLCVRFEFLSKLRHHHRRWRFTFSHFVVDPDQEYE VTVHHLPKPIPDGDPNHQSKNFLVPDCEHARMKVTTPC MSSALPWLNVSADGDNVHLVLNVSEEQHFGLSLYWNQ VQGPPKPRWHKNLTGPQIITLNHTDLVPCLCIQVWPLEPD SVRTNICPFREDPRAHQNLWQAARLQLLTLQSWLLDAPC SLPAEAALCWRAPGGDPCQPLVPPLSWENVTVDKVLEFP LLKGHPNLCVQVNSSEKLQLQECLWADSLGPLKDDVLL LETRGPQDNRSLCALEPSGCTSLPSKASTRAARLGEYLL QDLQSGQCLQLWDDDLGALWACPMDKYIHK | 66 |
| T1a | MGAARSPPSAVPGPLLGLLLLLLGVLAPGGSLRLLDHRA LVCSQPGLNCTVKNSTCLDDSWIHPRNLTPSSPKDLQIQL HFAHTQQGDLFPVAHIEWTLQTDASILYLEGAELSVLQL NTNERLCVRFEFLSKLRHHHRRWRFTFSHFVVDPDQEYE VTVHHLPKPIPDGDPNHQSKNFLVPDCEHARMKVTTPC MSSALPWLNVSADGDNVHLVLNVSEEQHFGLSLYWNQ VQGPPKPRWHKNLTGPQIITLNHTDLVPCLCIQVWPLEPD SVRTNICPFREDPRAHQNLWQAARLRLLTLQSWLLDAPC SLPAEAALCWRAPGGDPCQPLVPPLSWENVTVDKVLEFP LLKGHPNLCVQVNSSEKLQLQECLWADSLGPLKDDVLL LETRGPQDNRSLCALEPSGCTSLPSKASTRAARLGEYLL QDLQSGQCLQLWDDDLGALWACPMDKYIHK Q267R | 67 |
| T1b | MGAARSPPSAVPGPLLGLLLLLLGVLAPGGASLRLLDHR APVCSQPGLNCTVKNSDCLDDSWIHPRNLTPSSPKDLQI QLHFAHTQQGDLVPVAHIEWTLQTDASILYLEGAELSVL QLDTNERLCVRFEFLSKLRHHHRRWRFTFSHFVVDPGQE YEVTVHHLPKPIPDGDPNHQSKNFLVPDCEHARMKVTTP CMSSALPWLNVSADGDNVHLVLNVSEEQHFGLSLYWN QVQGPPKPRWHKNLTGPQIITLNHTDLVPCLCIQVWPLEP DSVRTNICPFREDPRAHQNLWQAARLRLLTLQSWLLDAP CSLPAEAALCWRAPGGDPCQPLVPPLSWENVTVDKVLE FPLLKGHPNLCVQVNSSEKLQLQECLWADSLGPLKDDV LLLETRGPQDNRSLCALEPSGCTSLPSKASTRAARLGEYL LQDLQSGQCLQLWDDDLGALWACPMDKYIHK L9P/T24D/F59V/M88D/D122G/Q267R | 68 |
| T1c | MGAARSPPSAVPGPLLGLLLLLLGVLAPGGASLRLLDHR APVCSQPGLNCTVKNSDCLDDSWIHPRNLTPSSPKDLQI QLHFAHTQQGDLVPVAHIEWTLQTDASILYLEGAELSVL QLDTNERLCVRFEFLSKLRHHHRRWRFTFSHFVVDPGQE YEVTVHHLPKPIPDGDPNHQSKNFLVPDCEHPRMKVTTP CMSSALPWLNVSADGDNVHLVLNVSEEQHFGLSLYWN QVQGPPKPRWHKNLTGPQIITLNHTDLVPCLCIQVWPLEP DSVRTNICPFREDPRAHQNLWQAARLRLLTLQSWLLDAP CSLPAEAALCWRAPGGDPCQPLVPPLSWENVTVDKVLE FPLLKGHPNLCVQVNSSEKLQLQECLWADSLGPLKDDV LLLETRGPQDNRSLCALEPSGCTSLPSKASTRAARLGEYL LQDLQSGQCLQLWDDDLGALWACPMDKYIHK L9P/T24D/F59V/N88D/D122G/A156P/Q267R | 69 |

**Table 7: Information on TNFR2 sequences**

| **Name** | **TNFR2 Sequence** | **SEQ ID NO** |
|---|---|---|
| T4 wt (wt TNFR2) | LPAQVAFTPYAPEPGSTCRLREYYDQTAQMCCSKCSPGQ HAKVFCTKTSDTVCDSCEDSTYTQLWNWVPECLSCGSR CSSDQVETQACTREQNRICTCRPGWYCALSKQEGCRLC APLRKCRPGFGVARPGTETSDVVCKPCAPGTFSNTTSST DICRPHQICNVVAIPGNASMDAVCTSTSTRSMAPGAVHLP QPVSTRSQHTQPTPEPSTAPSTSFLLPMGPSPPAEGSTGD | 70 |
| T4a | LPAQVAFTPYAPEPGSTCRLREYYDQTAQMCCAKCSPGQ HAKVFCTKTSDTVCDSCEDSTYTQLWNWVPECLSCGSR CSSDQVETQACTREQNRICTCRPGWYCALSKQEGCRLC APLRKCRPGFGVARPGTETSDVVCKPCAPGTFSNTTSST DICRPHQICNVVAIPGNASMDAVCTSTSPTRSMAPGAVHL PQPVSTRSQHTQPTPEPSTAPSTSFLLPMGPSPPAEGSTGD S33A | 71 |
| T4b | LPAQVAFTPYAPEPGSTCRLREYYDQTAQMCCAKCPPGQ HAKVFCTKTSDTVCDSCEDSTYTQLWNWVPECLSCGSR CSSDQVETQACTREQNRICTCRPGWYCALSKQEGCRLC APLRKCRPGFGVARPGTETSDVVCKPCAPGTFSNTTSST DICRPHQICNVVAIPGNASMDAVCTSTSPTRSMAPGAVHL PQPVSTRSQHTQPTPEPSTAPTSTSFLLPMGPSPPAEGSTG D S33A/S36P | 72 |
| T4c | LPAQVAFTPYAPEPGSTCRLREYYDQTAQMCCAKCPPGQ HAKVFCTKTSDTVCDSCEDSTYTQLWNWVPECLSCGSR CSSDQVETQACTREQNRICTCRPGWYCALSKQEGCRLC APLSKCRPGFGVARPGTETSDVVCKPCAPGTFSNTTSSTD ICRPHQICNVVAIPGNASMDAVCTSTSPTRSMAPGVAHLP QPVSTRSQHTQPTPEPSTAPSTSFLLPMGPSPPAEGSTGD S33A/S369/R119S | 73 |
| T4d | LPAQVAFTPYAPEPGSTCRLREYYDQTAQMCCAKCPPGQ HAKVFCTKTSDTVCDSCEDSTYTQLWNWVPECLSCGSR CSSDQVETQACTREQNRICTCRPGWYCALSKQEGCRLC APLSKCRPGFGVARPGTETSDVVCKPCAPGTFSNTTSSTD ICRPHQICSVVAIPGNASMDAVCTSTSPTRSMAPGAVHLP QPVSTRSQHTQPTPEPSTAPSTSFLLPMGPSPPAEGSTGD S33A/S36P/R119S/N164S | 74 |
| T4e | LAPQVAFTPYAPEPGSTCRLREYYDQTAQMCCAKCPPGQ HAKVFCTKTSDTVCDSCEDSTYTQLWNWVPECLSCGSR CSSDQVETQACTREQNRICTCRPGWYCALSKQEGCRLC APLSKCRPGFGVARPGTETSDVVCKPCAPGTFSNTTSSTD ICRPHQICSVVAIPGNASMDAVCTSTSPTRSMAPGAVHLP QPVSTRSQHTQPTPGPSTAPSTSFLLPMGPSPPAEGSTGD S33A/S36P/R119S/N164S/E210G | 75 |
| T4f | LPAQVAFTPYAPEPGSTCRLREYYDQTAQMCCAKCPPGQ HAKVFCTKTSDTVCDSCEDSTYTQLWNWVPECLSCGSR CSSDQVETQACTREQNRICTCRPGWYCALSKQEGCRLC APLSKCRPGFGVARPGTETSDVVCKPCAPGTFSNTTSSTD ICRPHQICSVVAIPGNASMDAVCTSTSPTRSMPGAVHLPQ PVSTRSQHTQPTPGPSTAPSTSILLPMGPSPPAEGSTGD S33A/S36P/R119S/N164S/E210G/F219I | 76 |
| T4g | LPAQVAFTPYAPEPGSTCRLREYYDQTAQMCCAKCPPGQ HAKVFCTKTSDTVCDSCEDSTYTQLWNWVPECLSCSSR CSSDQVETQACTREQNRICTCRPGWYCALSKQEGCRLC APLSKCRPGFGVARPGTETSDVVCKPCAPGTFSNTTSSTD ICRPHQICSVVAIPGNASMDAVCTSTSPTRSMAPGAVHLP QPVSTRSQHTQPTPGPSTAPSTSILLPMGPSPPAEGSTGD S33A/S36P/G75S/R119S/N164S/E210G/F219I | 77 |
| T4h | LPAQVAFTPYAPEPGSTCRLREYYDQTAQMCCSKCSPGQ HAKVFCTKTSDTVCDSCEDSTYTQLWNWVPECLSCSSR CSSDQVETQACTREQNRICTCRPGWYCALSKQEGCRLC APLRKCRPGFGVARPGTETSDVVCKPCAPGTFSNTTSST DICRPHQICNVVAIPGNASMDAVCTSTSPTRSMAPGAVHL PQPVSTRSQHTQPTPEPSTAPSTSFLLPMGPSPPAEGSTGD G75S | 78 |
| T4i | LPAQVAFTPYAPEPGSTCRLREYYDQTAQMCCAKCPPGQ HAKVFCTKTSDTVCDSCEDSTYTQLWNWVPECLSCSSR CSSDQVETQACTREQNRICTCRPGWYCALSKQEGCRLC APLSKCRPGFGVARPGTETSDVVCKPCAPGTFSNTTSSTD ICRPHQICSVVAIPGNASMDAVCTSTSPTRSMAPGAVHLP QPVSTR S33A/S36P/G75S/R119S/N164S | 79 |
| T4TR | LPAQVAFTPYAPEPGSTCRLREYYDQTAQMCCAKCPPGQ HAKVFCTKTSDTVCDSCEDSTYTQLWNWVPECLSCSSR CSSDQVETQACTREQNRICTCRPGWYCALSKQEGCRLC APLSKCRPGFGVARPGTETSDVVCKPCAPGTFSNTTSSTD ICRPHQICSVVAIPGNASMDAVCTSTSPTRSMAPGAVHLP QPVSTRSQHTQPTPGPSTAPSTSILLPMGPSPPAEGSTGDG SGEGEGSEGSGLPAQVAFTPYAPEPGSTCRLREYYDQTA QMCCAKCPPGQHAKVFCTKTSDTVCDSCEDSTYTQLW NWVPECLSCSSRCSSDQVETQACTREQNRICTCRPGWYC ALSKQEGCRLCAPLSKCRPGFGVARPGTETSDVVCKPCA PGTFSNTTSTDICRPHQICSVVAIPGNASMDAVCTSTSPTR SMAPGAVHLPQPVSTR TNFR2-linker-TNFR2 S33A/S36P/G75S/R119S/N164S/E210G/F2191-LinkerS33A/S36P/G75S/R119S/N164S | 80 |
| T4m | VPAQVVLTPYKPEPGYECQISQEYYDRKAQMCCAKCPP GQYVKHFCNKTSDTVCADCEASMYTQVWNQFRTCLSC SSSCTTDQVEIRACTKQQNRVCACEAGRYCALKTHSGSC RQCMRLSKCGPGFGVASSRAPNGNVLCKACAPGTFSDT TSSTDVCRPHRICSILAIPGNASTDAVCAPESPTLSAIPRTL YVSQPEPTRSQPLDQEPGPSQTSILTSLGSTIIEQSTKGG | 81 |

The expression of IL-17RA/RC-Fc-TNFR2 fusion protein was performed using ExpiCHO^{™} (Gibco, Cat: A29127) cells in accordance with the protocol of the ExpiCHO^{™} expression system kit. The host CHO cells were cultured in ExpiCHO^{™} expression medium (Gibco, Cat: A29100-01) under conditions of 8% CO₂, 37°C, and 120 rpm for one day. On the day of DNA transfection, the cells were grown to a density of 0.6-1.0 × 10⁷ cells/ml with over 95% viability and then diluted to 1.0 × 10⁷ cells/ml using fresh medium. For transfection, an ExpiFectamine^{™} CHO transfection kit (Gibco, Cat: A29129) was used to prepare the ExpiFectamine^{™} CHO & plasmid DNA complex. Cold OptiPRO^{™} SFM^{®} (Gibco, Cat: 12309019) medium was dispensed separately, and DNA and ExpiFectamine^{™} CHO reagent were each added at the appropriate concentration. After mixing, the solution was incubated at room temperature for 5 minutes before being applied to the host cells for transfection, and the culture process was initiated. At 18 hours post-transfection, an enhancer and feed included in the ExpiFectamine^{™} CHO transfection kit were added to the transfected cells, and the culture was maintained under conditions of 8% CO₂, 37°C, and 120 rpm for 7-10 days to complete recombinant protein production. After cultivation, the culture supernatant was separated by centrifugation at 4°C and 4000 rpm for 60 minutes. The collected supernatant was then stored at -80°C.

### Example 3: Separation and Purification of IL-17RA/RC-Fc and IL-17RA/RC-Fc-TNFR2

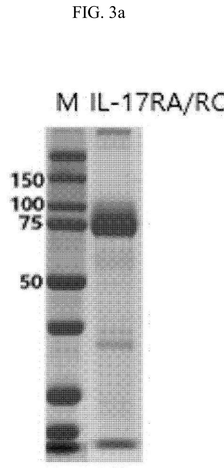

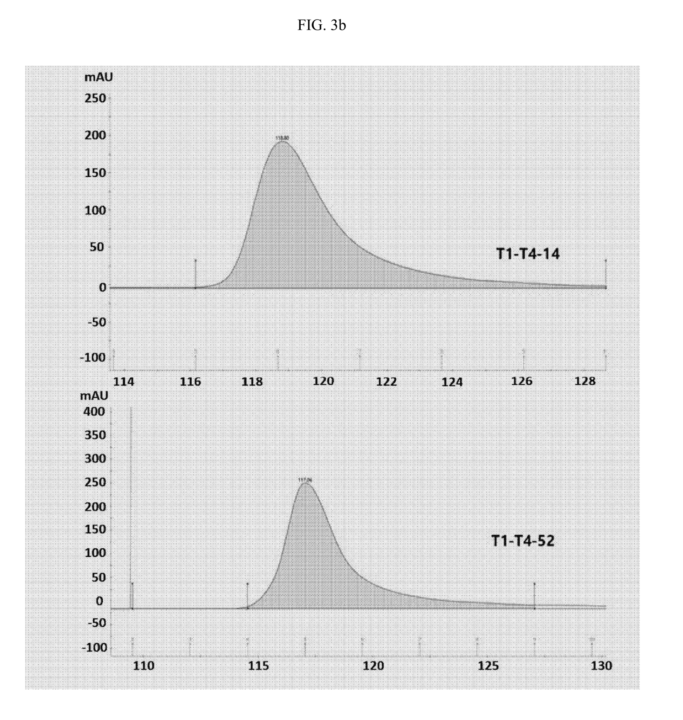

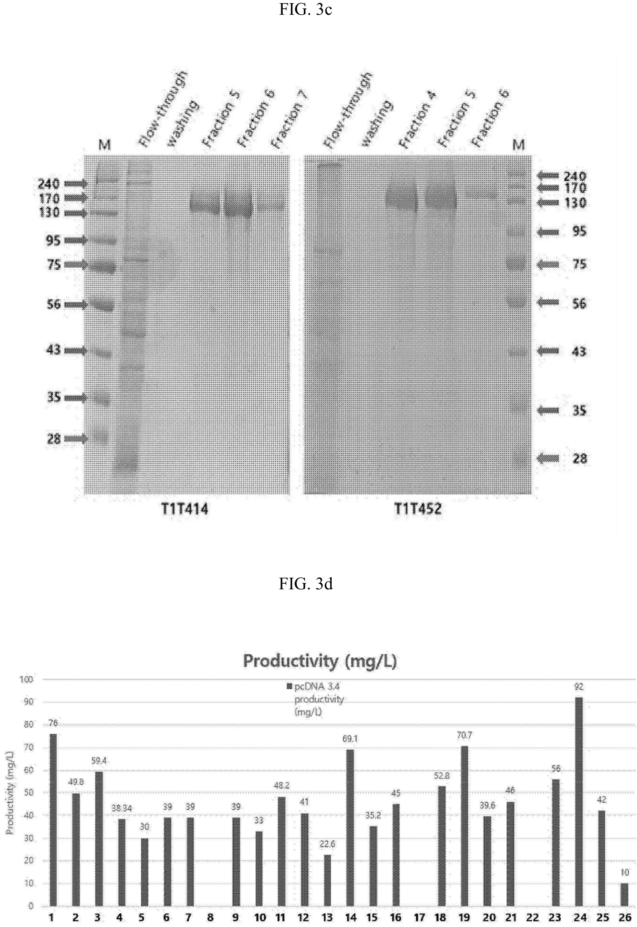

The productivity and stability of the IL-17RA/RC-Fc and IL-17RA/RC-Fc-TNFR2 fusion proteins were analyzed. Variants with enhanced productivity, stability, and neutralizing ability were obtained by introducing the combination of mutations selected in Examples 1 and 2 into the wtIL-17RA (UniProt: human IL-17RA: Q96F46), wtIL-17RC (UniProt: human IL-17RC: Q8NAC3), and wtTNFR2 proteins (UniProt: human TNFR2: Q9UIG9). The variants showed significantly improved productivity and stability compared to the wild-type fusion protein. The IL-17RA/RC-Fc and IL-17RA/RC-Fc-TNFR2 fusion proteins were prepared using CHO cells and purified using Protein A affinity chromatography. Subsequently, the supernatant containing the IL-17RA/RC hybrid fusion protein (IL-17RA/RC-Fc-TNFR2) was centrifuged at 4°C, 4000 rpm for 60 minutes, and the harvested cell culture fluid (HCCF) was filtered through a 0.45 µm filter. Next, a HiTrap MabSelect Sure column (Cytiva, 11-0034-95) was prepared. The column was equilibrated with 10 column volumes (CV) of binding buffer (BB) (20 mM sodium phosphate, 150 mM NaCl, pH 7.2) before loading the sample (HCCF). After equilibration with 6 column volumes (CV) of binding buffer (BB), the column was subsequently washed using the wash buffer (WB) (100 mM sodium citrate, 150 mM NaCl, pH 5.0). The bound protein was eluted using an elution buffer (EB) (1 M glycine, 150 mM NaCl, pH 3.0), and the eluted fractions were collected in tubes containing 1 M Tris buffer (pH 9.0) in 2.5 ml aliquots. After protein elution, purified proteins were confirmed by SDS-PAGE. The SDS-PAGE analysis of the IL-17RA/RC-Fc and IL-17RA/RC-Fc-TNFR2 (T1-T4-14) fusion proteins under reducing conditions at 10% revealed bands at approximately 77 kDa and 103 kDa, respectively (FIGs. 3a to 3c). The eluted proteins were further purified using a secondary purification process involving size exclusion chromatography (SEC) as follows: First, a purified sample containing the IL-17RA/RC hybrid fusion protein (IL-17RA/RC-Fc-TNFR2) was loaded onto the column at a flow rate of 1 ml/min using an AKTA^{™} pure 25 system (Cytiva, 29018224). Specifically, the overpressure was set to 0.5 MPa, and the flow rate was maintained at 1 ml/min according to the column specifications. The column used was Superdex 200 (Cytiva), and the elution buffer consisted of an LPS solution, CBP007B, diluted to 1/10, with the addition of 0.005% Tween-20 (Cytiva, BR100054). The separation was performed by monitoring the peaks corresponding to elution buffer volume (HiLoad 16/600 Superdex 200 pg, 1x 120 ml, Cytiva, 28989335). The final purification process was conducted in two stages, starting with Protein A chromatography, followed by size exclusion chromatography (SEC) for final purification. The yield of the chromatography process for the fusion proteins is summarized in Tables 8 and 9.

**Table 8: Yield of IL-17RA/RC-Fc purified using a Protein A column**

| | |
|---|---|
| **Concentration** | 1.251 mg/mL (1 mL) |
| **Yield rate** | 1.251 mg/50 mL |
| **Total volume** | 1.251 mg |

**Table 9: Yield of two types of IL-17RA/RC-Fc-TNFR2 variants**

| | **T1-T4-14** | **T1-T4-52** |
|---|---|---|
| **TF vol** | 50 mL | 50 mL |
| **Molecular Weight (MW) of targets** | Approximately 100 kDa | Approximately 100 kDa |
| **Time** | 7 days | 7 days |
| **Yield rate** | 24.7 mg/L | 38.4 mg/L |

The productivity of twenty-six IL-17RA/RC-Fc-TNFR2 variants, newly cloned from pBispec vectors into pcDNA3.4 vectors, as listed in Table 10, was analyzed. The results showed that productivity may increase simply by replacing the vectors, without introducing mutations to the constructs (see FIG. 3d). The yield rate was measured using proteins purified by the method described in Embodiment 3. Information on the IL-17RA/RC-Fc-TNFR2 variant constructs of the present invention is summarized in Table 10.

**Table 10: Information on IL-17RA/RC-Fc-TNFR2 variant constructs**

| **No.** | **Vector** | **IL-17RA/RC RA (1-199) RC (208-466)** | **Linker** | **Fc** | **Linker** | **TNFR2 (1-235)** |
|---|---|---|---|---|---|---|
| 1 | pcDNA3.4 | SEQ ID NO: 69 | L1 | SEQ ID NO: 33 | L2 | SEQ ID NO: 77 |
| 2 | pcDNA3.4 | SEQ ID NO: 69 | L1 | SEQ ID NO: 39 | L2 | SEQ ID NO: 77 |
| 3 | pcDNA3.4 | SEQ ID NO: 69 | L1 | SEQ ID NO: 34 | L2 | SEQ ID NO: 77 |
| 4 | pcDNA3.4 | SEQ ID NO: 69 | L1 | SEQ ID NO: 35 | L2 | SEQ ID NO: 77 |
| 5 | pcDNA3.4 | SEQ ID NO: 69 | L1 | SEQ ID NO: 37 | L2 | SEQ ID NO: 77 |
| 6 | pcDNA3.4 | SEQ ID NO: 69 | L1 | SEQ ID NO: 38 | L2 | SEQ ID NO: 77 |
| 7 | pcDNA3.4 | SEQ ID NO: 69 | L1 | SEQ ID NO: 46 | L2 | SEQ ID NO: 77 |
| 8 | pcDNA3.4 | SEQ ID NO: 69 | L1 | SEQ ID NO: 47 | L2 | SEQ ID NO: 77 |
| 9 | pcDNA3.4 | SEQ ID NO: 69 | L1 | SEQ ID NO: 49 | L2 | SEQ ID NO: 77 |
| 10 | pcDNA3.4 | SEQ ID NO: 69 | L1 | SEQ ID NO: 50 | L2 | SEQ ID NO: 77 |
| 11 | pcDNA3.4 | SEQ ID NO: 69 | L1 | SEQ ID NO: 41 | L2 | SEQ ID NO: 77 |
| 12 | pcDNA3.4 | SEQ ID NO: 69 | L1 | SEQ ID NO: 56 | L2 | SEQ ID NO: 77 |
| 13 | pcDNA3.4 | SEQ ID NO: 69 | L1 | SEQ ID NO: 57 | L2 | SEQ ID NO: 77 |
| 14 | pcDNA3.4 | SEQ ID NO: 69 | L1 | SEQ ID NO: 59 | L2 | SEQ ID NO: 77 |
| 15 | pcDNA3.4 | SEQ ID NO: 69 | L1 | SEQ ID NO: 60 | L2 | SEQ ID NO: 77 |
| 16 | pcDNA3.4 | SEQ ID NO: 69 | L1 | SEQ ID NO: 40 | L2 | SEQ ID NO: 77 |
| 17 | pcDNA3.4 | SEQ ID NO: 69 | L1 | SEQ ID NO: 51 | L2 | SEQ ID NO: 77 |
| 18 | pcDNA3.4 | SEQ ID NO: 69 | L1 | SEQ ID NO: 52 | L2 | SEQ ID NO: 77 |
| 19 | pcDNA3.4 | SEQ ID NO: 69 | L1 | SEQ ID NO: 54 | L2 | SEQ ID NO: 77 |
| 20 | pcDNA3.4 | SEQ ID NO: 69 | L1 | SEQ ID NO: 55 | L2 | SEQ ID NO: 77 |
| 21 | pcDNA3.4 | SEQ ID NO: 69 | L1 | SEQ ID NO: 42 | L2 | SEQ ID NO: 77 |
| 22 | pcDNA3.4 | SEQ ID NO: 69 | L1 | SEQ ID NO: 61 | L2 | SEQ ID NO: 77 |
| 23 | pcDNA3.4 | SEQ ID NO: 69 | L1 | SEQ ID NO: 62 | L2 | SEQ ID NO: 77 |
| 24 | pcDNA3.4 | SEQ ID NO: 69 | L1 | SEQ ID NO: 64 | L2 | SEQ ID NO: 77 |
| 25 | pcDNA3.4 | SEQ ID NO: 69 | L1 | SEQ ID NO: 65 | L2 | SEQ ID NO: 77 |
| 26 | pcDNA3.4 | SEQ ID NO: 69 | L1 | SEQ ID NO: 33 | L2 | SEQ ID NO: 77 |

### Example 4: Analysis of Thermal Stability of IL-17RA-Fc Protein

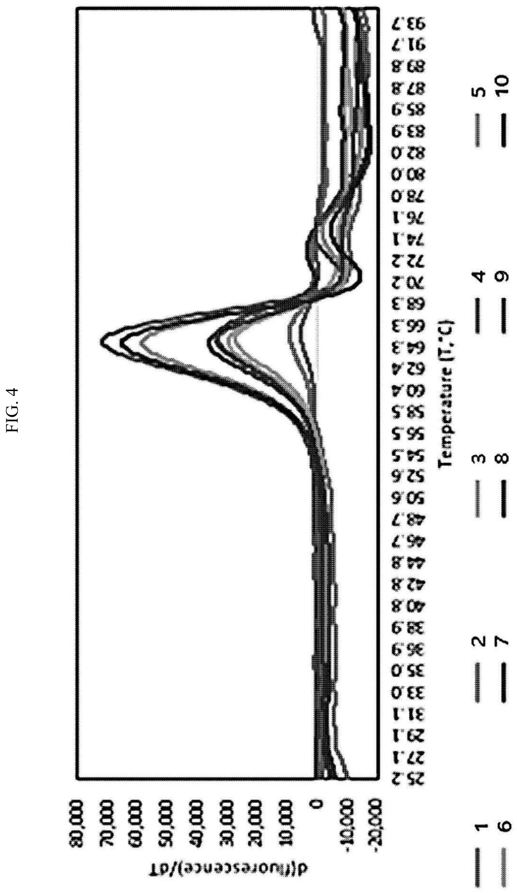

For thermal stability analysis, IL-17RA-Fc protein and wtIL-17RA-Fc control protein were prepared at a concentration of 0.5-1 mg/ml. A 1000x dye from the Thermal Shift Assay Dye Kit (Applied Biosystems, 4461146) was diluted to 8x, and the sample was further diluted with triple-distilled water. Subsequently, the reaction buffer of the kit, the protein, and the dye mixture were combined in the following ratio: reaction buffer (7.5 µl): protein (10 µl): 8X dye (2.5 µl). The melting curve was then measured using a PCR instrument (QuantStudio 6 Flex, Applied Biosystems, 4485689). The Tm and ΔTm values were determined using the Thermal Shift Assay Software (Applied Biosystems, 4466038). The Tm corresponds to the maximum value of the first derivative of the DSF melting curve obtained from the software. The thermal stability analysis of IL-17RA-Fc proteins with various mutations showed that stability was maintained up to 65°C. A comparison of IL-17RA-Fc proteins numbered 3, 6, and 7 in Table 11 indicated that the IL-17RA-Fc protein with both the T24D and N88D mutations exhibited increased thermal stability compared to IL-17RA-Fc proteins with only the T24D or N88D mutation. According to FIG. 4, lane numbers 2, 8, and 10, the addition of T24D to the combination of F59V, R108K, and D122G mutations in IL-17RA-Fc proteins did not enhance thermal stability. To determine combinations of Back-to-Consensus mutations that could simultaneously affect IL-17A affinity and stability, experiments were conducted to analyze both thermal stability and affinity. The results of the thermal stability analysis of the IL-17RA-Fc fusion proteins are summarized in Table 11 below.

**Table 11: Results of thermal stability analysis**

| N**o.** | **Construct** | **Tm (°C)** | **Std. dev** | **ΔTm (°C)** | **IL-17RA/RC mutations** |
|---|---|---|---|---|---|
| 1 | IL-17RA-Fc (wt) | 64.80 | 0.09 | - | WT |
| 2 | IL-17RA-Fc | 65.50 | 0.05 | 0.70 | F59V/R108K/D122G |
| 3 | IL-17RA-Fc | 64.99 | 0.05 | 0.19 | N88D |
| 4 | IL-17RA-Fc | 65.55 | 0.05 | 0.75 | L9P/T24D/R108K/D 122G/H155 D/G243 W/A267V |
| 5 | IL-17RA-Fc | 65.08 | 0.17 | 0.28 | L9P/T24D/F59V/R108K/D122G/ A156P |
| 6 | IL-17RA-Fc | 64.75 | 0 | - | T24D |
| 7 | IL-17RA-Fc | 64.57 | 0.05 | 0.23 | T24D/N88D |
| 8 | IL-17RA-Fc | 65.17 | 0 | 0.37 | T24D/F59V/R108K/D122G |
| 9 | IL-17RA-Fc | 64.85 | 0.05 | - | L9P/R108K/D122G/H155D/G243 W/A267V |
| 10 | IL-17RA-Fc | 64.71 | 0.09 | - | L9P/F59V/R108K/D 122G/A 156P |

### Example 5: Analysis of Binding Affinity of IL-17RA/RC-Fc-TNFR2 for the Ligands IL-17A, IL-17F, and TNFα

For affinity analysis, IL-17AA (Peprotech, 200-17), IL-17FF (Peprotech, 200-25), IL-17AF (R&D Systems, 5837-IL), and TNFα (Novus Biologicals, NBP2-35076) were prepared along with a microplate (96-well/pureGrade, Black/F bottom, 350 µl, BRAND. 781608) and AHC2 biosensors (Sartorius, 18-5019). Affinity measurements were conducted using the Octet system from Sartorius AG. First, the AHC2 biosensors were hydrated using a 2x kinetic buffer. The ligand concentration was prepared to allow IL-17RA/RC-Fc-TNFR2 to bind to the biosensor within a range of 0.8-1 nm. The ligand concentrations used in the experiment were set across 5 to 7 different levels, ensuring that the expected KD value fell within the middle range of the chosen concentrations. Using the hydrated biosensors, a baseline was established with a buffer solution. The biosensors were then transferred into a solution containing IL-17RA/RC-Fc-TNFR2, ensuring that IL-17RA/RC-Fc-TNFR2 binding reached 0.8-1 nm. The bound biosensors were then placed back into the buffer solution used for baseline setting to allow for equilibrium. Subsequently, the biosensors were transferred to solutions containing the ligands for the association phase, followed by a dissociation phase in the buffer solution used for baseline setting.

The binding affinity values of IL-17RA/RC-Fc-TNFR2 for each ligand (IL-17AA, IL-17AF, IL-17FF, and TNFα) were determined using the Octet system, as summarized in Table 12 below. The affinity of IL-17RA/RC-Fc-TNFR2 for IL-17AA was comparable to that of the positive control, Bimekizumab. The affinity for IL-17AF was either comparable to or lower than that of Bimekizumab, while the affinity for IL-17FF was measured as lower than that of Bimekizumab. The affinity of IL-17RA/RC-Fc-TNFR2 for TNFα was compared with that of the positive control, Etanercept, and was found to be equal to or greater than that of Etanercept. The binding affinity values of IL-17RA/RC-Fc-TNFR2 for each ligand (IL-17AA, IL-17AF, IL-17FF, and TNFα) were all higher than those of wild-type IL-17RA (wtIL-17RA), wild-type IL-17RC (wtIL-17RC), and wild-type TNFR2 (wtTNFR2). The affinity analysis results of IL-17RA/RC-Fc-TNFR2 for each ligand (IL-17A, IL-17F, and TNFα) are summarized in Table 12.

**Table 12: Results of binding affinity analysis**

| **Molecular Cytokine** | **K_{D} (M) of IL-17RA/RC-Fc-TNFR2** | **K_{D} (M) of wtIL-17RA** | **K_{D} (M) of wtIL-17RC** | **K_{D} (M) of positive control** |
|---|---|---|---|---|
| IL-17AA | <1.0E-12 | 7.0E-10 | 4.0E-10 | 3.2E-12 (Bimekizumab) |
| IL-17AF | 1.888E-10 | 1.75E-8 | 6.0E-10 | 2.6E-11 (Bimekizumab) |
| IL-17FF | 2.48E-10 | 1.36E-7 | 4.0E-9 | 2.3E-11 (Bimekizumab) |
| TNFα | <1.0E-12 | - | - | 1.8E-11 (Etanercept) |

| | | | | |
|---|---|---|---|---|
| IL-17AA: IL-17RA/RC-Fc-TNFR2 - Bimekizumab > IL-17RA wild type - IL-17RC wild type IL-17AF: Bimekizumab > IL-17RA/RC-Fc-TNFR2 - IL-17RC wild type > IL-17RA wild type IL-17FF: Bimekizumab > IL-17RA/RC-Fc-TNFR2 > IL-17RC wild type > IL-17RA wild type TNFα: IL-17RA/RC-Fc-TNFR2 > Etanercept | | | | |

### Example 6: Analysis of Binding Interactions Between IL-17RA/RC-Fc-TNFR2 and the Target Ligands IL-17A and TNFα

Recombinant multi-target fusion proteins of the present invention are proteins in which two or more protein domains are fused. In these fusion proteins, it is crucial to maintain the biological activity of each protein domain intact. With suitable linkers between domains and appropriate constructs, the activity of each protein domain can be maintained without steric hindrance in ligand-receptor interactions. Maintaining the activity of recombinant multi-target fusion proteins is expected to enhance stability, prolong plasma half-life, and improve *in vivo* efficacy.

The experiments were conducted to determine whether IL-17RA/RC-Fc-TNFR2 can simultaneously bind to the ligands IL-17AA, IL-17FF, IL-17AF, and TNFα, and whether its neutralizing ability against all ligands is not significantly different from that of the constituent IL-17RA/RC or TNFR2. IL-17RA/RC-Fc-TNFR2 and IL-17A proteins were produced by co-expression via co-transfection into ExpiCHO cells. Human TNFα (hTNFα) proteins were produced through overexpression in *E. coli* cells. The obtained IL-17RA/RC-Fc-TNFR2, hIL-17A, and hTNFα proteins were purified using His-tag affinity chromatography, anion exchange chromatography, and size-exclusion chromatography. The multi-target fusion protein IL-17RA/RC-Fc-TNFR2 and the target ligands IL-17A and TNFα, purified as described above, were incubated overnight at 4°C. The incubated proteins were then centrifuged at 13,000 rpm for 10 minutes to remove aggregates. The proteins without aggregates were separated into bound and unbound fractions using size-exclusion chromatography.

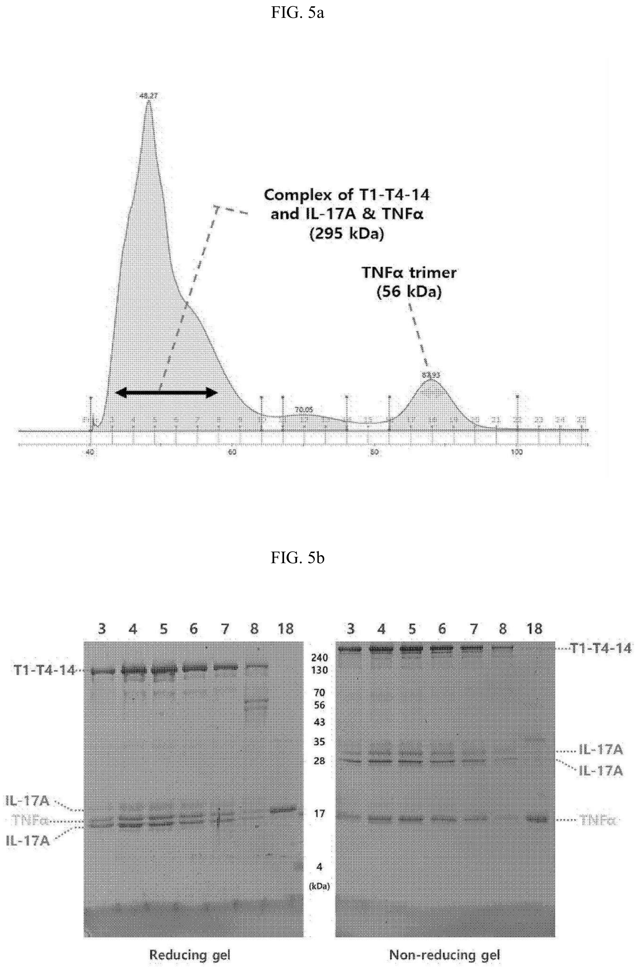

In FIG. 5a, the first peak on the left represents a form in which all three proteins are bound to each other, whereas the peak on the right represents a form where IL-17RA/RC-Fc-TNFR2 is unbound to TNFα. According to the results of SDS-PAGE gel analysis, TNFα forms trimers not through disulfide bonds but through hydrogen or ionic bonds, and it was identified as a monomer in both non-reducing and reducing gels. Since IL-17A forms dimers through disulfide bonds, it was identified as a monomer in the reducing gel and as a dimer in the non-reducing gel. Additionally, IL-17A incubated in the mammalian cell, ExpiCHO cells has a glycosylation site, so it was identified as multiple bands due to glycosylation (see FIG. 5b).

When IL-17RA/RC-Fc-TNFR2 and IL-17A proteins, produced by co-expression, were co-incubated at 4°C for 24 hours with TNFα, produced in *E. coli* cells, the tri-specific fusion protein IL-17RA/RC-Fc-TNFR2 was found to stably bind the first ligand, IL-17A, and the second ligand, TNFα, forming a complex. Since the multi-target fusion protein IL-17RA/RC-Fc-TNFR2 was found to simultaneously bind to each target, no difference in neutralizing ability is expected between its simultaneous and sequential binding to each ligand. The biolayer interferometry (BLI) measurement conducted in Embodiment 5 showed that the binding affinity for the ligand TNFα was <1.0E-12 for the fusion protein IL-17RA/RC-Fc-TNFR2 and 1.6E-11 for the IL-17RA/RC-Fc-TNFR2 and IL-17A complex. No significant difference in binding affinity was identified when IL-17RA/RC-Fc-TNFR2 bound to the first ligand followed by the second, compared to these two measured values."

**Example 7: Analysis of Neutralizing Ability of the Fusion Proteins IL-17RA/RC-Fc and IL-17RA/RC-Fc-TNFR2 Against the Target Ligands IL-17AA, IL-17AF, and IL-17FF**

The following materials were prepared to analyze neutralizing ability: HEK-Blue^{™} reporter cells (InvivoGen, hkb-IL-17 or HEK-Blue^{™} TNFα), Dulbecco's phosphate-buffered saline (DPBS; Welgene, LB 001-02), fetal bovine serum (FBS; Gibco, 16000044), Dulbecco's modified Eagle medium (DMEM; Welgene, LM 001-05), antibiotics (Welgene, LS 203-01), HEK-Blue^{™} selection (InvivoGen, hb-sel), Normocin^{™} (InvivoGen, ant-nr-1), QUANTI-Blue^{™} solution (InvivoGen, rep-qbs), Tween-20 (Junsei, 69295S1601), autoclaved distilled water (DW), IL-17A (PeproTech, Cat# 200-17), IL-17F (PeproTech, Cat# 200-25), and TNFα (PeproTech, Cat# 300-01A).

In the inflammatory environment of autoimmune diseases, the ligands IL-17AA, IL-17AF, and IL-17FF form a complex with IL-17RA/RC, thereby inducing the formation of the IL-17 signalosome. The IL-17 signalosome complex amplifies the production of inflammatory cytokines and chemokines through intracellular signaling (Arnaud Goepfert et al., Ann. Rheum. Dis. CellReports 41: 111489, 2022). The selective neutralization by the fusion protein IL-17RA/RC-Fc-TNFR2 against the ligands IL-17A, IL-17F, and IL-17A/F in the inflammatory environment is closely associated with the treatment of autoimmune diseases.

To assess the neutralizing ability of the fusion proteins IL-17RA/RC-Fc and IL-17RA/RC-Fc-TNFR2 against each ligand, HEK-Blue^{™} reporter cells (InvivoGen, hkb-IL-17 or HEK-Blue^{™} TNFα) were treated with hIL-17AA, hIL-17AF, or hTNFα at various concentrations. Consequently, the reporter protein, secreted embryonic alkaline phosphatase (SEAP), was secreted in a concentration-dependent manner. The SEAP levels in the cell culture fluids of cells treated with the fusion proteins IL-17RA/RC-Fc and IL-17RA/RC-Fc-TNFR2 were quantified to assess the inhibition of signaling pathways. To separate HEK-Blue^{™} reporter cells, the media was removed during incubation, followed by two washes with 1X PBS. The cells obtained from this process were transferred into a 15-mL conical tube and centrifuged at 1,200 rpm for 4 minutes. The separated cells were then resuspended in test media (DMEM, 10% FBS, 1% PS). After adding 10 µL each of trypan blue and resuspended cells to the media and mixing them, 10 µL of this mixture was loaded onto a hemocytometer, followed by counting the viable cells. The mixture was resuspended in test media to achieve a final concentration of 2.5 × 10⁵ cells/mL. Then, 100 µL of the resuspension was pipetted into a well of a 96-well plate and incubated overnight. The fusion protein IL-17RA/RC-Fc was diluted in 0.005% Tween-20/PBS to prepare concentrations ranging from 10 to 100 µg/mL. Next, three types of ligands (hIL-17AA, hIL-17AF, and hTNFα) were prepared at a concentration of 10 ng/mL, and 100 µL of each ligand was pipetted into a well of a 96-well plate. The cells were treated with IL-17RA/RC-Fc and each ligand, then incubated for 24 hours. To assess the neutralizing ability of the fusion protein IL-17RA/RC-Fc-TNFR2 against each ligand in HEK-Blue^{™} reporter cells, 20 µL of the incubated samples was mixed with 180 µL of QUANTI-Blue^{™} and incubated for 10 minutes. The incubated samples were placed into a Bio-Tek microplate reader to measure the optical density (OD) at a wavelength of 630 nm. The fusion protein IL-17RA/RC-Fc-TNFR2 was diluted in 0.005% Tween-20/PBS to prepare concentrations ranging from 10 to 100 µg/mL. The ligands hIL-17AA, hIL-17FF, and TNFα were prepared at concentrations of 10 ng/mL, 10 ng/mL, and 1 ng/mL, respectively. The samples, prepared using the same method as described above, were placed into a Bio-Tek microplate reader to measure the OD value at a wavelength of 630 nm. The positive controls used in this neutralizing ability analysis were Bimekizumab for neutralizing IL-17A and IL-17F, and Adalimumab for neutralizing TNFα.

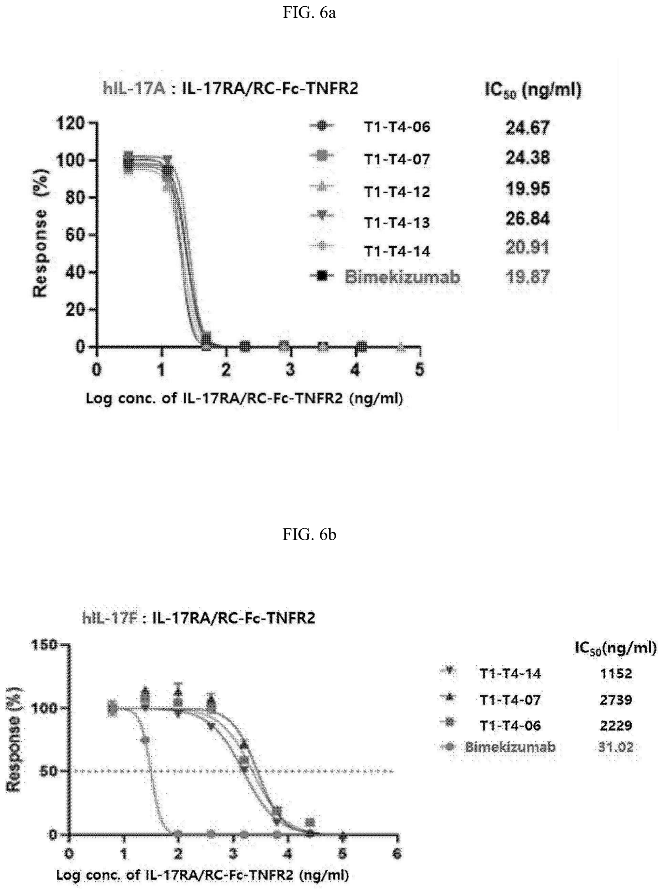

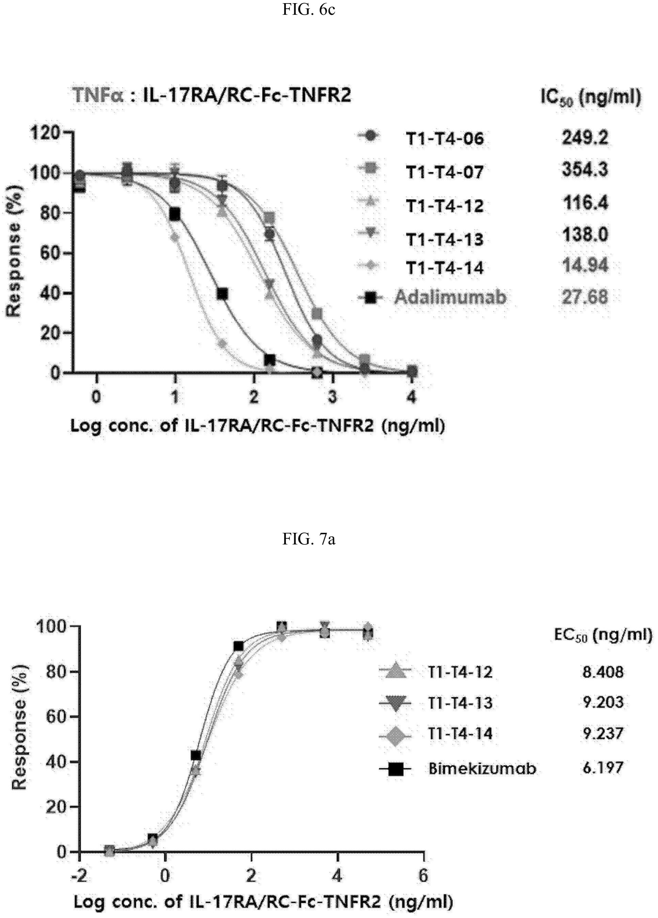

The half-maximal inhibitory concentration (IC₅₀), representing the neutralizing ability against IL-17AA, was 20.91 ng/mL for the fusion protein IL-17RA/RC-Fc-TNFR2 and 19.87 ng/mL for the positive control Bimekizumab, demonstrating comparable neutralizing abilities. The IC₅₀ value, representing the neutralizing ability against TNFα, was 14.94 ng/mL for the fusion protein IL-17RA/RC-Fc-TNFR2 and 27.68 ng/mL for the positive control Adalimumab. These results indicate that the fusion protein exhibits greater neutralizing ability than the positive control. The IC₅₀ value, representing the neutralizing ability against IL-17FF, was 1152 ng/mL for the fusion protein IL-17RA/RC-Fc-TNFR2 and 31.02 ng/mL for the positive control Bimekizumab. These results reveal that the fusion protein exhibits lower neutralizing ability than the positive control but demonstrates specificity for IL-17FF. Accordingly, the neutralizing abilities of the multi-target fusion protein, IL-17RA/RC-Fc-TNFR2, against the two ligands, IL-17AA and TNFα, were comparable to those of the positive controls, Bimekizumab and Adalimumab (see FIGs. 6a to 6c). Table 13 presents the results of the neutralizing ability analysis for IL-17RA/RC-Fc-TNFR2 against the ligands hIL-17AA, hIL-17FF, and hTNFα, as assessed by the SEAP reporter assay.

**Table 13: Results of analysis of neutralizing ability of IL-17RA/RC-Fc-TNFR2**

| **IL-17RA/RC-Fc-TNFR2** | **IC₅₀ (ng/mL) of hIL-17A** | **IL-17RA/RC-Fc-TNFR2** | **IC₅₀ (ng/mL) of hIL-17F** | **IL-17RA/RC-Fc-TNFR2** | **IC₅₀ (ng/mL) of hTNFα** |
|---|---|---|---|---|---|
| T1-T4-06 | 24.67 | T1-T4-06 | 2229 | T 1-T4-06 | 249.2 |
| T1-T4-07 | 24.38 | T1-T4-07 | 2739 | T1-T4-07 | 354.3 |
| T1-T4-12 | 19.95 | T1-T4-14 | 1152 | T1-T4-12 | 116.4 |
| T1-T4-13 | 26.86 | T1-T4-28 | 6798 | T1-T4-13 | 138.0 |
| T1-T4-14 | 20.91 | T1-T4-29 | 7746 | T1-T4-14 | 14.94 |
| T1-T4-28 | 35.57 | T1-T4-30 | 1030 | Adalimumab | 27.68 |
| T1-T4-29 | 28.87 | T1-T4-31 | 1238 | | |
| T1-T4-30 | 23.67 | T1-T4-32 | 1262 | | |
| T1-T4-31 | 15.33 | Bimekizumab | 31.02 | | |
| T1-T4-32 | 20.47 | | | | |
| Bimekizumab | 19.87 | | | | |

### Example 8: Analysis of Binding Affinity of IL-17RA/RC-Fc-TNFR2 for IL-17A, IL-17F, and TNFα using ELISA

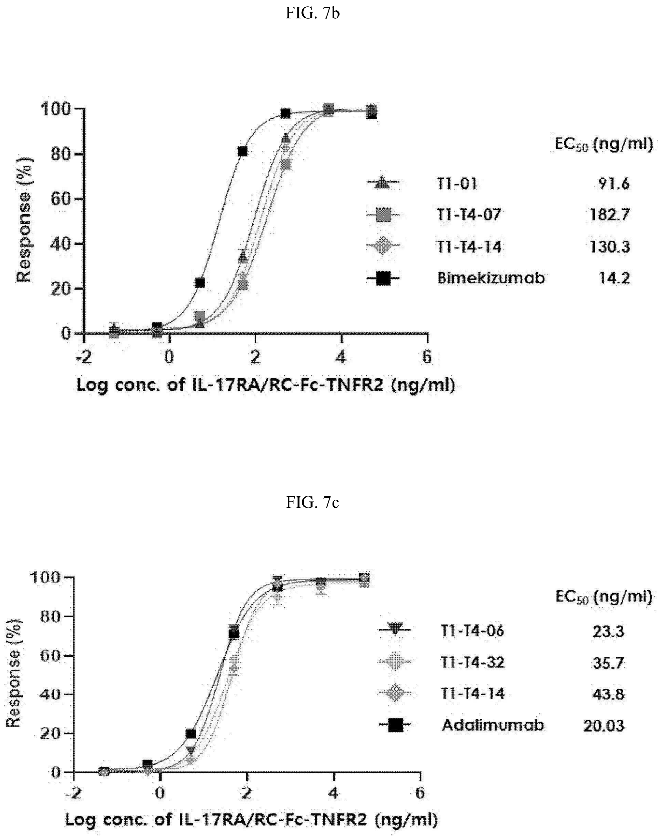

For affinity analysis, a coating buffer of 50 ml carbonate buffer (pH 9.6, NaTNFR2 against the ligands hNaHCO₃aHCOffinity analysis, a coating buffer of 50 ml carbonate buffer (pH 9.6, NaTNFR2 against the ligands hIL-PeproTech; 200-17, Human IL-17F - PeproTech; 200-25, Human TNFα - PeproTech; 300-01A, Murine IL-17A - PeproTech; 210-17, Murine IL-17F - PeproTech; 210-17F, Murine TNFα - PeproTech; 315-01A) was added to the coating buffer and coated onto a 96-well plate at 40 µl/well overnight at 4°C. On the second day, the plate was washed three times with ELISA TBS buffer (1X TBS and 0.1% Tween 20) at 200 µl/well. The wells were then blocked with ELISA TBS buffer containing 5% BSA at 100 µl/well and incubated at room temperature for 1 hour, followed by three washes with ELISA TBS buffer (200 µl/well). Subsequently, IL-17RA/RC-Fc-TNFR2 protein was added at 100 µl/well in blocking buffer and incubated at room temperature for 2 hours. The IL-17RA/RC-Fc-TNFR2 protein was treated at concentrations of 0, 0.05, 0.5, 5, 50, 500, 5000, and 50000 ng/ml. After three washes with ELISA TBS buffer (200 µl/well), HRP-conjugated secondary antibody (Abcam, Cat. Ab98624) was applied and incubated at room temperature for 1 hour. The plate was then washed five times with ELISA TBS buffer, followed by the addition of 100 µl of TMB solution (Seracare KPL, Cat# 5120-0080) for reaction at room temperature. The reaction was stopped by adding 100 µl of stop solution (Cat# 5150-0024), and absorbance was measured at 650 nm using an ELISA reader. The EC₅₀ value of the fusion protein IL-17RA/RC-Fc-TNFR2 for hIL-17A was 9.237 ng/ml, which was comparable to the positive control, Bimekizumab (EC₅₀ = 6.197 ng/ml). The EC₅₀ value for hIL-17F was 130.3 ng/ml, which was nearly equivalent to the positive control, Bimekizumab (EC₅₀ = 14.2 ng/ml). Additionally, the EC₅₀ value for hTNFα was 43.08 ng/ml, which was similar to the positive control, Adalimumab (EC₅₀ = 20.03 ng/ml). Notably, the affinity of the multi-target fusion protein IL-17RA/RC-Fc-TNFR2 for the two ligands IL-17A and TNFα was measured to be almost equivalent to the positive controls, Bimekizumab and Adalimumab (see FIGs. 7a to 7c). The affinity measurement results of the fusion protein IL-17RA/RC-Fc-TNFR2 for IL-17A, IL-17F, and TNFα are summarized in Table 14.

**Table 14: Results of analysis of binding affinity for IL-17A, IL-17F, and TNFα**

| **IL-17RA/RC-Fc-TNFR2** | **EC₅₀ (ng/mL) of hIL-17A** | **IL-17RA/RC-Fc-TNFR2** | **EC₅₀ (ng/mL) of hIL-17F** | **IL-17RA/RC-Fc-TNFR2** | **EC₅₀ (ng/mL) of hTNFα** |
|---|---|---|---|---|---|
| T1-T4-06 | 10.25 | T1-T4-07 | 182.7 | T1-T4-06 | 23.3 |
| T1-T4-07 | 7.064 | T1-T4-14 | 130.3 | T 1-T4-07 | 2044 |
| T1-T4-8 | 12.57 | T1-T4-28 | 147.4 | T1-T4-14 | 43.8 |
| T1-T4-9 | 8.697 | T1-T4-29 | 111.5 | T1-T4-19 | 769.3 |
| T1-T4-10 | 10.55 | T1-T4-31 | 85.32 | T1-T4-27 | 90.05 |
| T1-T4-11 | 10.55 | Bimekizumab | 46.74 | T1-T4-28 | 44.73 |
| T1-T4-12 | 8.408 | | 14.2 | T1-T4-29 | 47.93 |
| T1-T4-13 | 9.203 | | | T1-T4-31 | 62.71 |
| T1-T4-14 | 9.237 | | | T1-T4-32 | 40.64 |
| Bimekizumab | 6.197 | | | Adalimumab | 20.03 |

### Example 9: Evaluation of the Anti-inflammatory Effects of the Multi-target Fusion Protein IL-17RA/RC-Fc-TNFR2 in a Psoriasis-like Environment

Inflammatory cytokines and chemokines are expressed through the synergistic stimulation of TNFα and IL-17 in human keratinocyte (HaCaT) cell lines. In other words, multiple inflammatory cytokines and chemokines, such as IL-6, IL-17C, IL-8, CXCL8, CCL20, BD2, LNC2, and S100A7, which are present in the skin lesions of human psoriasis, along with the amplified production of antimicrobial peptide genes, were similarly identified in an *ex vivo* experiment (Andrea Chiricozzi et al., J. Invest. Dermatol. 131(3): 677-687, 2011).

IL-17RA/RC-Fc and TNFR2-Fc described in one embodiment of the present invention were compared with the positive controls, Bimekizumab and Adalimumab, to evaluate their therapeutic effectiveness in the skin lesions of human psoriasis. In addition, the efficacy of IL-17RA/RC-Fc, TNFR2-Fc, and IL-17RA/RC-Fc-TNFR2, as described in the present invention, was evaluated by comparing the anti-inflammatory effects of the combination of IL-17RA/RC-Fc and TNFR2-Fc fusion proteins or IL-17RA/RC-Fc-TNFR2 alone with those of the combination of the positive controls Bimekizumab and Adalimumab. To prepare for the experiment, HaCaT (human keratinocyte) cells were maintained in 2% FBS, low-calcium DMEM (Gibco, 21068028) for more than two weeks. Then, the cells were treated with 0.53 mM EDTA (10 min, 37°C), followed by Trypsin-EDTA treatment (2 min, 37°C). Afterward, 2% FBS low-calcium DMEM was added to create a single-cell suspension, which was then centrifuged (1200 rpm, 5 min, 4°C). The number of cells suspended in 2% FBS low-calcium DMEM was counted, and cells were seeded into a 12-well plate (1.0 × 10⁵ cells/1 mL). After overnight incubation, stabilized cells were washed with PBS and subjected to starvation by adding 0.9 mL of 0.5% FBS low-calcium DMEM (24 h, 37°C).

To induce a psoriasis model, the cytokines IL-17AA, AF, FF, and TNFα were treated in 0.1 mL of 0.5% FBS low-calcium DMEM (24 h, 37°C). Subsequently, the fusion proteins IL-17RA/RC-Fc-TNFR2, IL-17RA/RC-Fc, and TNFR2-Fc, as well as the positive control antibodies bimekizumab (Antibody System, DHH28803) and adalimumab (Selleckchem, A2010), were treated in a concentration-dependent manner (15 h, 37°C). The effects of the cytokines and inhibitors (fusion proteins IL-17RA/RC-Fc-TNFR2, IL-17RA/RC-Fc, TNFR2-Fc, and the positive control antibodies bimekizumab and adalimumab) on the psoriasis-like HaCaT cell model were compared under the treatment conditions listed in Table 15.

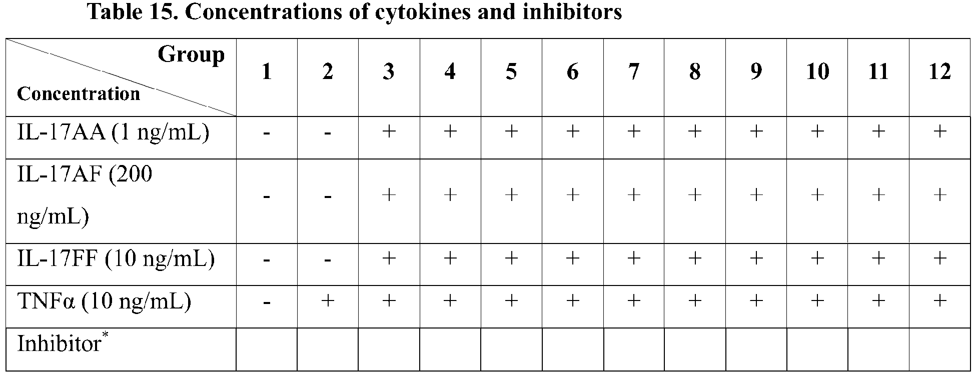

Inhibitor-treated Groups (Groups 4 to 12)
Group 4: IL-17RA/RC-Fc 20 ng/mL
Group 5: TNFR2-Fc 50 ng/mL
Group 6: IL-17RA/RC-Fc 20 ng/mL + TNFR2-Fc 50 ng/mL
Group 7 - 9: IL-17RA/RC-Fc-TNFR2 5 ng/mL, 20 ng/ml and 80 ng/ml, respectively
Group 10: Bimekizumab 20 ng/mL
Group 11: Adalimumab 50 ng/mL
Group 12: Bimekizumab 20 ng/mL + Adalimumab 50 ng/mL

To perform qRT-PCR analysis for each treatment group, cells were lysed using Qiazol lysis reagent (Qiagen, 79306). Chloroform was then added, followed by centrifugation at 13,200 rpm at 4°C for 15 minutes to separate the aqueous phase. The separated organic phase was mixed with isopropanol to precipitate RNA, followed by centrifugation at 13,200 rpm at 4°C for 10 minutes. The resulting pellet was washed with 75% DEPC-EtOH and centrifuged at 13,200 rpm for 5 minutes. After centrifugation, the ethanol was evaporated from the pellet. The pellet was then dissolved in 30-50 µl of DEPC-water. The concentration of the solubilized RNA was measured to be 200-250 ng/µl. cDNA was synthesized using a cDNA synthesis kit (abm, G236), and the obtained cDNA (1 µl) was mixed with target gene primers (Table 16) and 10 µl of SYBR Green. RT-qPCR was performed using the QuantStudio^{™} 5 Real-Time PCR system.

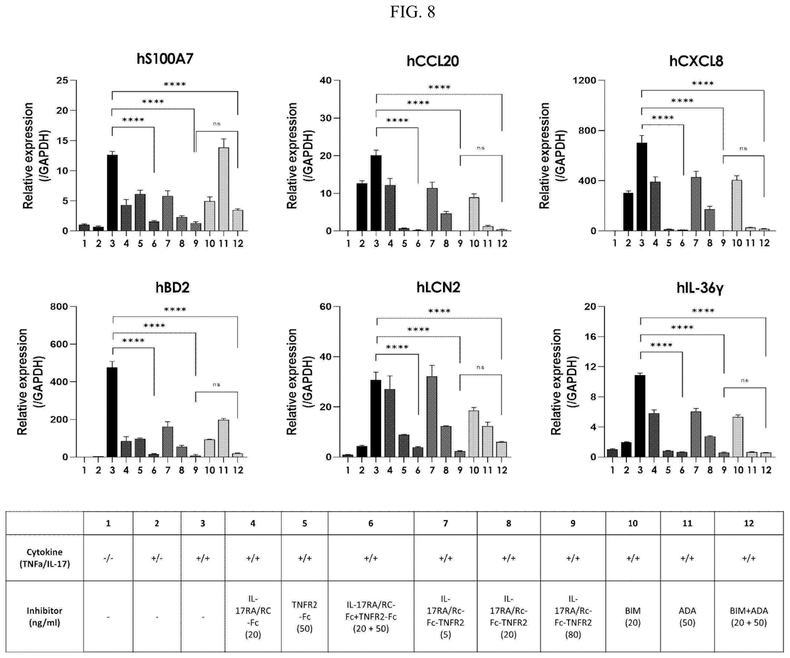

IL-8 (CXCL8) is a chemokine that recruits neutrophils and macrophages, while S100A7 (Psoriasin) is a protein overexpressed in psoriatic skin. Additionally, CCL20 is an inflammatory cytokine induced by LPS and IFNy, which also functions as a chemokine recruiting neutrophils. BD2 is a skin antimicrobial peptide produced by keratinocytes in psoriasis patients. LCN2 (Lipocalin-2) is expressed in neutrophils and serves as a marker of skin damage. IL-36γ (IL1F9) is a cytokine belonging to the IL-1 family, activated by NFκB, and associated with psoriatic lesions, making it a useful biomarker for psoriasis. When comparing HaCaT cells treated with IL-17 alone or TNF-α alone (lanes 1 and 2, respectively) to those treated with both IL-17 and TNF-α simultaneously (lane 3), a significant increase in the expression of S100A7, CCL20, BD2, LCN2, CXCL8, and IL-36γ was observed (FIG. 8). This suggests that the synergistic effect of IL-17 and TNF-α plays a major role in the hyperproliferation and pro-inflammatory responses characteristic of psoriasis, more so than either cytokine alone.

In the group treated with both IL-17 and TNF-α, the addition of adalimumab led to an unexpected increase in the expression of S100A7 and BD2. Furthermore, CXCL8, IL-36γ, and CCL20 were not significantly inhibited by bimekizumab alone. However, when adalimumab and bimekizumab were administered together, a marked suppression of expression was observed. The primer sequences used for RT-qPCR are summarized in Table 16.

**Table 16: Information of primer sequences**

| **Gene** | **Sequence (5'→3')** | **SEQ ID NO** |
|---|---|---|
| hGAPDH F | TCA CCA GGG CTG CTT TTA AC | 100 |
| hGAPDH R | GAC AAG CTT CCC GTT CTC AG | 101 |
| hCXCL8 F | AGC TCT GTG TGA AGG TGC AG | 102 |
| hCXCL8 R | TCT CAG CCC TCT TCA AAA ACT TC | 103 |
| hS100A7 F | CTT CCC CAA CTT CCT TAG TG | 104 |
| hS100A7 R | GTA GTC TGT GGC TAT GTC TC | 105 |
| hCCL20 F | GCAAGC AAC TTT GAC TGC TG | 106 |
| hCCL20 R | CAA GTC CAG TGA GGC ACA AA | 107 |
| hBD2 F | ATC AGC CAT GAG GGT CTT G | 108 |
| hBD2 R | GCA GCA TTT TGT TCC AGG | 109 |
| hLCN2 F | TGA GTG CAC AGG TGC CG | 200 |
| hLCN2 R | TTT AGC AGA CAA GGT GGG GC | 201 |
| hIL-36γF | CAG CCC ACA TTG CAG CTA AA | 202 |
| hIL-36γR | AGG AGG CAA TGA ACC AGT CC | 203 |

IL-17RA/RC-Fc prepared in one embodiment of the present invention is a fusion protein that targets IL-17AA, IL-17AF, and IL-17FF. TNFR2-Fc is a fusion protein that targets TNFα. The inhibitory effects of IL-17RA/RC-Fc and TNFR2-Fc on their respective target ligands was compared to that of the positive controls Bimekizumab and Adalimumab. The inhibitory effects of IL-17RA/RC-Fc-TNFR2 on cytokines and chemokines induced by treatment with both IL-17 and TNFα were compared to those of IL-17RA/RC-Fc and TNFR2-Fc, used either alone or in combination, as well as to the positive controls Bimekizumab and Adalimumab, used either alone or in combination. IL-17RA/RC-Fc and Bimekizumab exhibited equivalent anti-inflammatory effects (see Lane Nos. 4 and 10 in FIG. 8). The group treated with the combination of IL-17RA/RC-Fc and TNFR2-Fc exhibited greater inhibitory effects on cytokines and chemokines compared to the groups treated with either IL-17RA/RC-Fc or TNFR2-Fc alone. The anti-inflammatory effects of TNFR2-Fc were equivalent to or greater than those of Adalimumab (see Lane Nos. 5 and 11 in FIG. 8). Notably, the inhibitory effects on S100A7, BD2, and LCN2 were greater with TNFR2-Fc than with Adalimumab.
The group treated with the combination of IL-17RA/RC-Fc and TNFR2-Fc showed greater inhibitory effects on cytokines and chemokines compared to the group treated with either IL-17RA/RC-Fc or TNFR2-Fc alone. The group treated with the combination of IL-17RA/RC-Fc and TNFR2-Fc exhibited greater inhibitory effects on cytokines and chemokines compared to the groups treated with either IL-17RA/RC-Fc or TNFR2-Fc alone. The multi-target fusion protein IL-17RA/RC-Fc-TNFR2, capable of simultaneously targeting IL-17AA, IL-17AF, and IL-17FF, demonstrated dose-dependent anti-inflammatory effects on the psoriatic factors S100A7, CCL20, BD2, LCN2, CXCL8, and IL-36γ, compared to inhibitors used alone or in combination (see Lane Nos. 7 to 9 in FIG. 8). The groups treated with IL-17RA/RC-Fc plus TNFR2-Fc (Lane No. 6), Bimekizumab plus Adalimumab (Lane No. 12), and IL-17RA/RC-Fc-TNFR2 at a concentration of 20 ng/mL demonstrated sufficient inhibitory effects on cytokines and chemokines. IL-17RA/RC-Fc-TNFR2 at a concentration of 80 ng/mL demonstrated superior inhibitory effects on cytokines and chemokines compared to all combination treatment groups. When the concentration of inhibitors with equivalent inhibitory effects is converted into molarity, the fusion protein IL-17RA/RC-Fc-TNFR2 at 80 ng/mL corresponds to 392 pM, while the combination of Bimekizumab at 20 ng/mL and Adalimumab at 50 ng/mL corresponds to 473 pM. IL-17RA/RC-Fc-TNFR2 from the present invention demonstrated efficacy at a lower dose compared to other inhibitors.

In a psoriasis-like model induced by the synergistic stimulation of both IL-17A and TNFα, single-target inhibitors for either IL-17A or TNFα demonstrated therapeutic limitations. The expression of S100A7 and BD2 was increased by Adalimumab, while the inhibitory effects of Bimekimab on CXCL8, IL-36γ, and CCL20 were not significant. However, the combination of Adalimumab and Bimekizumab demonstrated superior inhibitory effects on all cytokines compared to either Adalimumab or Bimekizumab alone. Accordingly, the simultaneous inhibition of both IL-17A and TNFα may be necessary to effectively control inflammatory responses.

The IL-17RA/RC-Fc-TNFR2 fusion protein described in the present invention demonstrated dose-dependent inhibitory effects on S100A7, CCL20, BD2, LCN2, CXCL8, and IL-36γ. Sufficient inhibitory effects on cytokines and chemokines were observed in the group treated with IL-17RA/RC-Fc-TNFR2 at 20 ng/mL. IL-17RA/RC-Fc-TNFR2 at 80 ng/mL exhibited superior inhibitory effects on cytokines and chemokines compared to the combination of Adalimumab and Bimekizumab. Psoriatic lesions are chronic inflammatory skin diseases mediated by immune cells and are characterized by the infiltration of inflammatory monocytes, macrophages, eosinophils, and inflammatory Th17 and Th1 cells, as well as keratinocyte hyperplasia and abnormal differentiation. To effectively manage these lesions, it is important to simultaneously reduce the expression of inflammatory cytokines, chemotactic chemokines, and antimicrobial peptides. The multi-target fusion protein IL-17RA/RC-Fc-TNFR2, which simultaneously controls both IL-17 and TNFα, can serve as an excellent treatment for psoriasis by effectively regulating various cytokines and chemokines involved in hyperplasia and pro-inflammatory responses through the synergistic action of IL-17 and TNFα.

### Example 10: Evaluation of the Anti-inflammatory Effects of IL-17RA/RC-Fc-TNFR2 in a Rheumatic Inflammatory Disease-like Environment

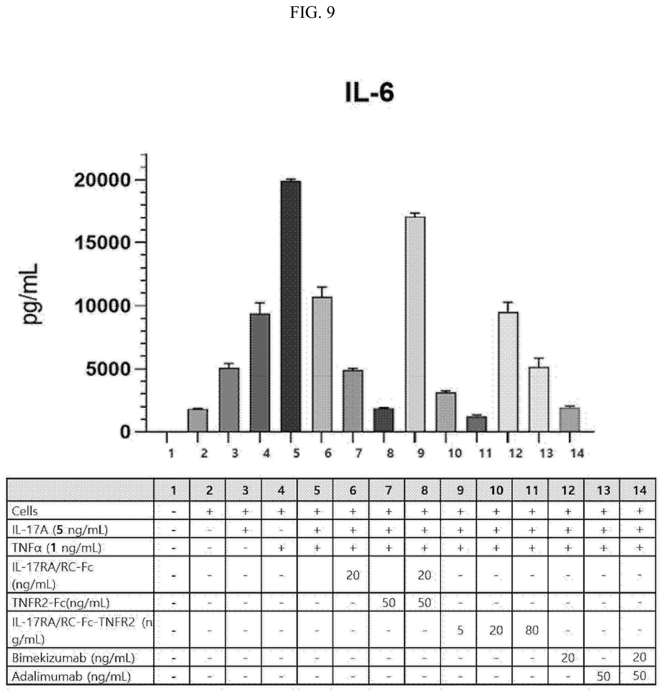

Rheumatoid arthritis is a synovial inflammatory disease accompanied by systemic symptoms. The inflammation originates in the synoviocytes and progresses to the destruction of cartilage and soft tissues. Inflammatory responses occurring around synovial fibroblasts, leukocytes near the inflammation site, and stromal cells are amplified by the synergistic stimulation of IL-17 and TNFα. Notably, IL-17 alone does not exhibit a significant pro-inflammatory effect; however, the combined action of IL-17A and IL-17F leads to synergistic increases in TNFα, IL-6, IL-8, CXCL1, CCL20, CXCL5, IL-23, E-selectin, and EGR-1 via MAPK family pathways. The addition of TNFα stabilizes mRNA, leading to the induction of chronic inflammatory responses (Amaud Hot and Pierre Miossec., Ann. Rheum. Dis. 70(5): 727-32, 2011). To determine whether the multi-target fusion protein IL-17RA/RC-Fc-TNFR2 from the present invention improves disease outcomes in a rheumatoid arthritis (RA)-like cell model, hFLS-RA cell lines (Cell Applications Inc.) were treated with both TNFα and IL-17, and IL-6 expression levels were subsequently assessed (Bilal Osta *et al., Front. Immunol.* 6: 151, 2015). Specifically, rheumatoid hFLS were cultured in each well of a 96-well plate with 2×10^4 cells in DMEM (Welgene, LM001-005) supplemented with 10% FBS (Gibco, 16000044) (24 hours, 37°C). On day 2, to induce the RA model, the cells were treated with cytokines IL-17A and TNFα, single-target fusion proteins IL-17RA/RC-Fc and TNFR2-Fc, and the multi-target fusion protein IL-17RA/RC-Fc-TNFR2. As control treatments, bimekizumab (Antibody system, DHH28803) and adalimumab (Selleckchem, A2010) were administered at the indicated concentrations (FIG. 9) (24 hours, 37°C). On the following day, the culture supernatant was collected, and an ELISA was performed to assess IL-6 expression using the human IL-6 DuoSet ELISA (R&D systems, DY206-05) and the DuoSet ELISA Ancillary Reagent Kit 2 (R&D systems, DY008B). The coating buffer was prepared using 50 mL of carbonate buffer (pH 9.6) made by mixing Na2CO3 (Sigma, S7795, 0.1 M) and NaHCO3 (Sigma, S8875, 0.1 M) at a 1:1 ratio. A 96-well plate was coated with 40 µL per well of 1 µg/mL IL-6 recombinant protein in the coating buffer overnight at 4°C. On day 2, the plate was washed three times with 200 µL per well of ELISA TBS buffer (1× TBS with 0.1% Tween20). Then, 100 µL per well of ELISA TBS buffer containing 5% BSA was added for dilution, and the plate was incubated at room temperature for 1 hour. After washing three times with 200 µL per well of ELISA TBS buffer, 100 µL per well of blocking buffer was added and incubated for 2 hours. The IL-6 standard curve concentrations were set at 9.38, 18.8, 37.5, 75, 150, 300, and 600 pg/mL according to the instructions. Following three washes with 200 µL per well of ELISA TBS buffer, the HRP-conjugated secondary antibody was applied and allowed to react at room temperature for 1 hour. Then, after five washes with ELISA TBS buffer, 100 µL of TMB solution was added at room temperature for the reaction, followed by the addition of 100 µL of stop solution. The ELISA was measured using a BioTek Synergy HTX microplate reader at 540 nm and 450 nm absorbance. Evaluation of the anti-inflammatory efficacy of the IL-17RA/RC-Fc-TNFR2 protein, based on IL-6 expression levels, showed a concentration-dependent decrease (FIG. 9, lanes 9, 10, 11). In particular, the IL-17RA/RC-Fc-TNFR2 treatment group (FIG. 9, lane 11) exhibited a reduction in IL-6 levels comparable to the control group treated with the combination of bimekizumab and adalimumab, thereby confirming its anti-inflammatory efficacy (FIG. 9, lane 14).

### Example 11: Binding Affinity of the Fusion Protein IL-17RA/RC-Fc-TNFR2 for tmTNFα

The monoclonal antibodies Adalimumab and Infliximab bind to tmTNFα, regulating inflammatory responses through the MAP/ERK signaling pathways. Additionally, these antibodies promote the cross-linking of tmTNFα with TNFR2, resulting in the proliferation of Treg cells and the induction of immune tolerance. TNFα inhibitors can enhance therapeutic effectiveness by neutralizing sTNFα, promoting reverse signaling through binding to tmTNFα, and activating Treg cells. The efficacy of the TNFR fusion protein Etanercept in binding to tmTNFα is lower than that of therapeutic antibodies, which may result in reduced therapeutic effectiveness in certain diseases.

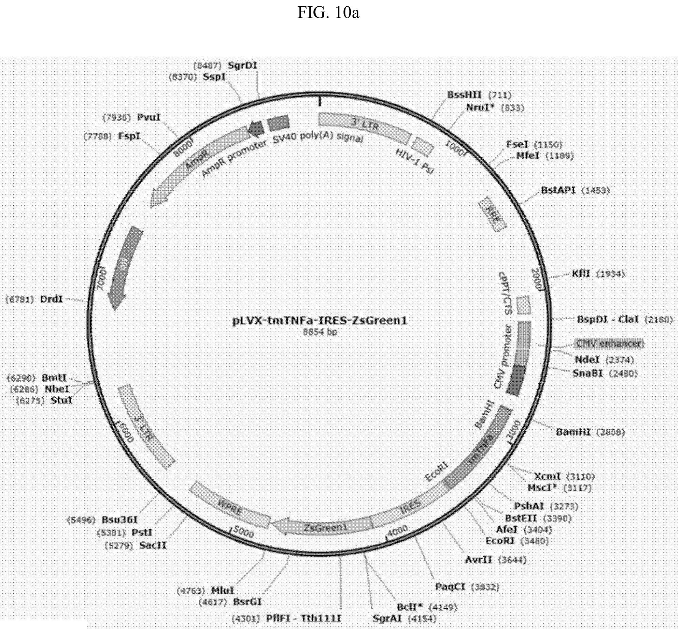

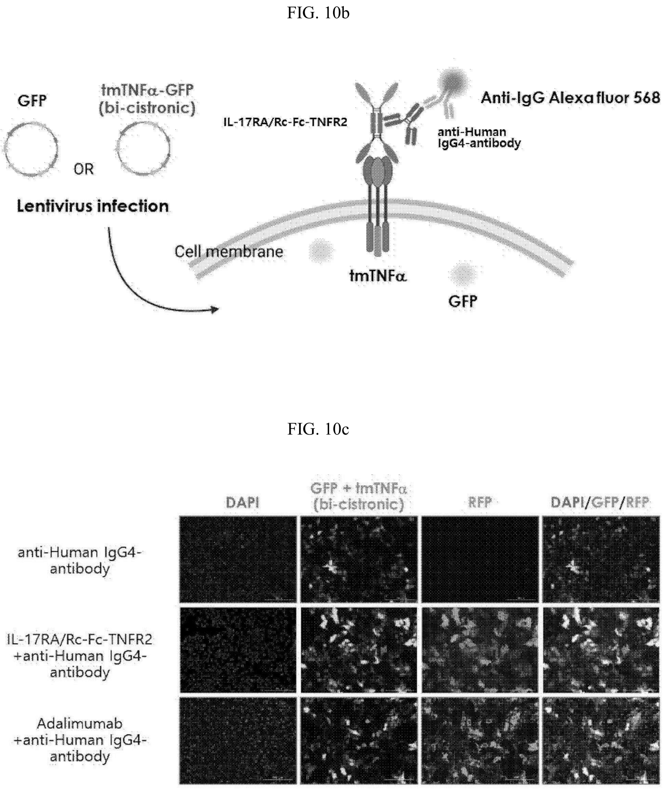

In the fusion protein IL-17RA/RC-Fc-TNFR2 of the present invention, TNFR2 is a TNFR2 variant in which a combination of various mutations was introduced into wtTNFR2. Cells expressing tmTNFα were prepared to determine the presence or absence of binding affinity for tmTNFα. To produce the tmTNFα-IRES-puro lentivirus, HEK-293T cells (1.2 x 10⁷ cells) were seeded in a 90 mm culture dish and incubated for 24 hours at 37°C. The following day, after replacing the medium with fresh 37°C medium, the cells were transfected with DNA vectors (pLVX tmTNFα-IRES-puro, pCMV VSVG, and pCMV delta8.2 in a 2:1:2 ratio, mixed with 15 µg PEI; source: Korea Atomic Energy Medical Center) for 2-4 hours at 37°C. Thereafter, the medium was replaced with 13 mL of fresh medium, and at 72 hours the culture supernatant was collected into a 50 mL conical tube. The virus was then harvested by centrifugation (500g for 5 minutes), impurities were removed using a 0.45 µm filter, and the virus was aliquoted in 1 mL portions into EP tubes and stored at -80°C. Subsequently, to verify the binding ability of the test drug IL-17RA/RC-Fc-TNFR2 with tmTNFα, HeLa cells (human cervical cancer cell line) were cultured in DMEM (Welgene, LM001-05) supplemented with 10% FBS (Gibco, 16000044) for 24-48 hours at 37°C. To subculture these cells into a 12-well plate, the FBS was removed by washing with 10 mL of 1x DPBS, followed by treatment with 0.53 mM EDTA (10 minutes at 37°C) and then Trypsin-EDTA (2 minutes at 37°C). After adding 2% FBS low-calcium DMEM, a single-cell suspension was obtained by centrifugation (1200 rpm for 5 minutes at 4°C). Meanwhile, cover glasses appropriate for each well of the 12-well plate were thoroughly flame-sterilized using an alcohol lamp and placed into the wells. The collected cells were then resuspended in fresh DMEM and seeded into each well at a density of 1 x 10^5 cells/mL, and allowed to stabilize for 24 hours at 37°C. Next, to transduce the cells with the pre-prepared tmTNFα-IRES-puro lentivirus, 500 µL of the lentiviral mixture was combined with polybrene transduction reagent (8 µg/mL; Merck, TR-1003-G) and mixed with 500 µL of culture medium (1:1 ratio, total 1 mL). This mixture was added to each well and cultured for 24 hours at 37°C. The following day, the viral mixture was removed, and the cells were washed three times with 1x DPBS, after which 500 µL of fresh culture medium was added, and the cells were maintained for another 24 hours at 37°C. For the treatment, both the control drug (the TNFα antibody therapy adalimumab; Selleckchem, A2010) and the test drug IL-17RA/RC-Fc-TNFR2 (T1-T4-14) were applied at 1 µg/mL and the cells were cultured for 6 hours at 37°C. Subsequently, after stabilizing the cells in the culture medium for 18 hours, the medium was removed for fluorescence staining. The cells were washed three times with 1x DPBS, then fixed with 4% paraformaldehyde (PFA) for 30 minutes at room temperature. After removal of the PFA solution and three additional washes with 1x DPBS, the cells were incubated with a blocking buffer (1% BSA and 0.1% NaN3 in 1x DPBS) at 4°C for 1 hour. For fluorescence staining, the primary antibody (Adalimumab; IgG1, T1-T4-14; Fc antibody) was diluted 500-fold in culture medium and added to the corresponding wells, followed by incubation for 24 hours at 4°C. After removing the medium and washing three times with 1x DPBS, the secondary antibody (Donkey anti-mouse IgG Alexa Fluor 568) was diluted 500-fold in culture medium and added to the wells. Subsequently, after three washes with 1 mL of 1x DPBS, the cover glasses with the cells on the well plate were placed onto slide glasses, and the cells were treated with DAPI solution along with an antifade mounting media. Thereafter, nail polish was used to secure the cover glasses onto the slide glasses by coating around the edges. After sufficient drying time, the samples were observed using a Lionheart FX Automated Microscope (BioTek). The results showed that when both tmTNFα (GFP) and the antibody detecting IL-17RA/RC-Fc-TNFR2 (Alexa Fluor 568; RFP) were co-expressed using the bicistronic vector shown in FIG. 10a, a yellow signal was observed. In the fourth column of FIG. 10c, the yellow fluorescence was noted to be stronger than that observed with the control drug adalimumab. These findings confirmed that IL-17RA/RC-Fc-TNFR2 binds to tmTNFα at levels comparable to the antibody therapy, thereby promoting tmTNFα reverse signal transduction and inducing the activation and proliferation of Treg cells, which may enable additional anti-inflammatory responses. The **tmTNFα** sequence information (sequence number 82) inserted into the pLVX tmTNFα-IRES-ZsGreenl vector is summarized in Table 17.

**Table 17: Genetic sequence of tmTNFα inserted into the pLVX tmTNFα-IRES-ZsGreen1 vector (SEQ ID NO: 82)**

| |
|---|
| ATCAGCACTGAAAGCATGATCCGGGACGTGGAGCTGGCCGAGGAGGCGCTCCCCAAG AAGACAGGGGGGCCCCAGGGCTCCAGGCGGTGCTTGTTCCTCAGCCTCTTCTCCTTC CTGATCGTGGCAGGCGCCACCACGCTCTTCTGCCTGCTGCACTTTGGAGTGATCGGCC CCCAGAGGGAAGAGTTCCCCAGGGACCTCTCTCTAATCAGCCCTCTGGCCCAGGCAG TAGCCCATGTTGTAGCAAACCCTCAAGCTGAGGGGCAGCTCCAGTGGCTGAACCGCC GGGCCAATGCCCTCCTGGCCAATGGCGTGGAGCTGAGAGATAACCAGCTGGTGGTGC CATCAGAGGGCCTGTACCTCATCTACTCCCAGGTCCTCTTCAAGGGCCAAGGCTGCCC CTCCACCCATGTGCTCCTCACCCACACCATCAGCCGCATCGCCGTCTCCTACCAGACC AAGGTCAACCTCCTCTCTGCCATCAAGAGCCCCTGCCAGAGGGAGACCCCAGAGGGG GCTGAGGCCAAGCCCTGGTATGAGCCCATCTATCTGGGAGGGGTCTTCCAGCTGGAG AAGGGTGACCGACTCAGCGCTGAGATCAATCGGCCCGACTATCTCGACTTTGCCGAGT CTGGGCAGGTCTACTTTGGGATCATTGCCCTGTGA |

### Example 12: Evaluation of the Immunogenicity of the Fusion Protein IL-17RA/RC-Fc-TNFR2 Using Human Peripheral Blood Mononuclear Cells (PBMCs)

Immunogenicity refers to the ability of macromolecular protein drugs to induce an immune response in the body. For example, an antibody acting as an immunogen in the human body can trigger either cell-mediated or humoral immunity. Anti-drug antibodies (ADAs) related to cell-mediated immunity may affect the efficacy, safety, pharmacokinetics, and pharmacodynamics of medicinal products. For this reason, the analysis of anti-drug antibodies (ADAs) is essential during the development of macromolecular protein drugs. In particular, drugs for the treatment of autoimmune diseases are medications intended for chronic administration, where the production and persistence of ADAs, as well as their impact on clinical outcomes, must be carefully considered. *In silico* and *in vitro* assessments were conducted to evaluate immunogenicity potential of the fusion protein IL-17RA/RC-Fc-TNFR2 described in the present invention. The results of these studies demonstrated that IL-17RA/RC-Fc-TNFR2 exhibited low immunogenicity.

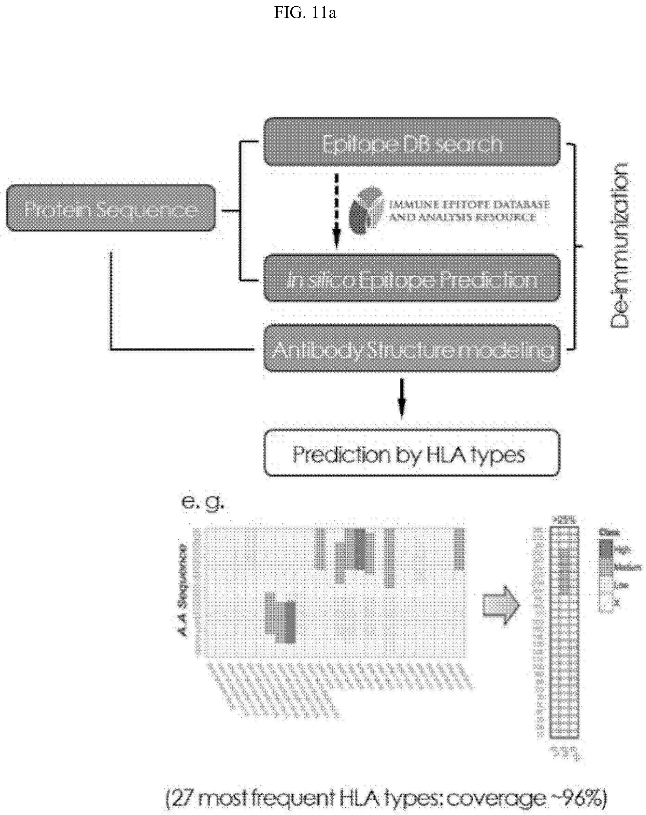

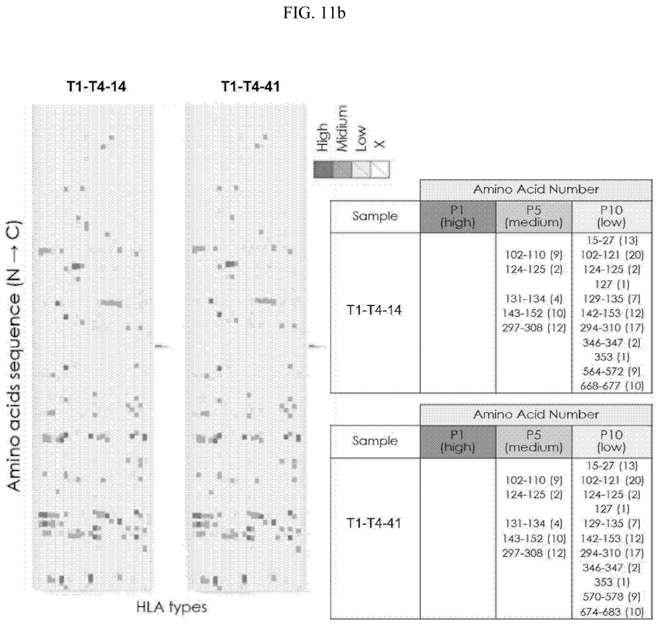

The fusion protein IL-17RA/RC-Fc-TNFR2, described in the present invention, utilizes hIL-17RA, hIL-17RC, and hTNFR2 as binding moieties to neutralize all three target ligands, including IL-17A, IL-17F, and TNFα. This protein was developed as a tri-specific fusion protein, incorporating a combination of mutations to enhance its stability and productivity while maintaining affinity for a ligand at each binding moiety. *In silico* immunogenicity prediction, as well as *in vitro* analysis using five types of PBMCs, was performed in two steps to evaluate the immunogenicity of the fusion protein IL-17RA/RC-Fc-TNFR2. The *in silico* immunogenicity analysis utilized the Immune Epitope Database (IEDB). The IEDB is supported by the National Institute of Allergy and Infectious Diseases (NIAID) under the National Institutes of Health (NIH). This database incorporates the latest information by tabulating and continuously updating experimental data on antibodies and T cell epitopes studied in humans, primates, and other animal species, in the context of infectious and allergic diseases, autoimmune responses, and organ transplantation. The IEDB server, which employs analytic methods with improved accuracy in predicting T cell epitopes, is the most widely utilized tool in *in silico* immunogenicity analysis. T cell major histocompatibility complex (MHC) Class II epitopes were analyzed using the consensus method in the IEDB (http://www.iedb.org/), which represents the mean of result values from the recommended methods, including NN-align, SMM-align, and CombLib/Sturniolo (see FIG. 11a). After dividing the input amino acid sequences into all possible peptides of 15 a.a. in length (9 binding sequences and 6 side sequences), the predicted values for the 15-amino-acid peptides, obtained from each analytic method, were compared with the random score distribution of a large set of random peptides and converted into statistical percentile ranks. The median percentile rank of the values obtained from the analytic methods is used as the final prediction value. A low percentile rank indicates a high prediction value, signifying strong affinity for MHC Class II receptors. The prediction system for MHC Class II epitopes shows the results after simplifying and visualizing the values, which are provided in an Excel format by the IEDB. The X-axis represents 27 HLA types, while the Y-axis represents the input amino acid sequence. The final prediction value is represented using different colors based on the degree of immunogenicity (Red: High, Orange: Medium, Yellow: Low, White: None) (see FIG. 11b).

According to the results of *in silico* immunogenicity analysis, the two fusion proteins, T1-T4-14 and T1-T4-41, contained in IL-17RA/RC-Fc-TNFR2, are predicted to exhibit a medium degree of immunogenicity in five sites each (see FIG. 11b). The *in silico* analysis method for immunogenicity predicts the likelihood of a specific peptide binding to MHC Class II molecules; however, it tends to over-predict when compared to actual results. Therefore, an *in vitro* immunogenicity assessment investigating T cell activation was conducted to determine whether the sites predicted to have high immunogenicity in the *in silico* analysis cause high immunogenicity in the body. In the *in vitro* method, likelihood of causing immunogenicity in clinical settings is predicted by analyzing cytokines, such as IFNγ and IL-2, through the proliferation or activation of T cells using human PBMCs, PBMC-derived dendritic cells (DCs), and T cells. In order to isolate cells from PBMC, after thawing the PBMC in an Automated Cell Thawing System (BioCision, 13-900-314), the cells were spun down in a centrifuge at 300 g for 5 minutes (Peripheral Blood Mononuclear Cells (PBMC), source: Catholic University Hematopoietic Stem Cell Bank). After removing the supernatant, the cell pellet was resuspended in AIM-V medium (GIBCO, 12055-083) and seeded in a 100 mm cell dish. Then, after treating with DNase, the cells were cultured in a 37°C, 5% CO₂ incubator, and the following day, all cells were harvested by centrifugation (300 g, 5 minutes) and resuspended in Running Buffer. Following this, FcR Blocking Reagent (Miltenyi Biotec, 130-059-901) was added and maintained at 4°C for 5 minutes, then CD14 MicroBeads (Miltenyi Biotec, 130-050-20) were added and kept at 4°C for 15 minutes. Next, Running Buffer (Miltenyi Biotec, 130-091-221) was added and the cells were centrifuged at 300 g at room temperature for 10 minutes to obtain the cells. After resuspending in culture medium, CD14⁻ and CD14⁺ cells were collected using an LS Column. The collected CD14⁻cells were then treated with CD4 MicroBeads (Miltenyi Biotec, 130-045-101) and CD8 MicroBeads (Miltenyi Biotec, 130-045-201) and maintained at 4°C for 15 minutes. After adding Running Buffer, the cells were gently spun down at 300 g at room temperature for 10 minutes, the supernatant was removed, and the cells were resuspended in Running Buffer. Then, the cells were loaded onto an LS Column (Miltenyi Biotec) to collect the flow-through (CD4⁻CD8⁻ cells), and the LS Column was washed three times with Running Buffer. Using a plunger, the cells (CD4⁺CD8⁺ cells) retained in the LS Column were collected. The collected CD14⁻CD4⁻CD8⁻ cells were then loaded onto an LS Column to collect the flow-through (CD4⁺CD8⁺ cells). The obtained CD4⁺/CD8⁺ cells were stored in liquid nitrogen for subsequent CD4⁺/CD8⁺ T cell activation experiments. CD14⁺ cells were cultured in AIM-V medium (containing GM-CSF (JW Creagen, HGM-100) and IL-4 (JW Creagen, HI4-100)) for 72 hours at 37°C. The AIM-V medium containing GM-CSF and IL-4 was then transferred to a culture dish for an additional 72 hours of culture (72 hours, 37°C).

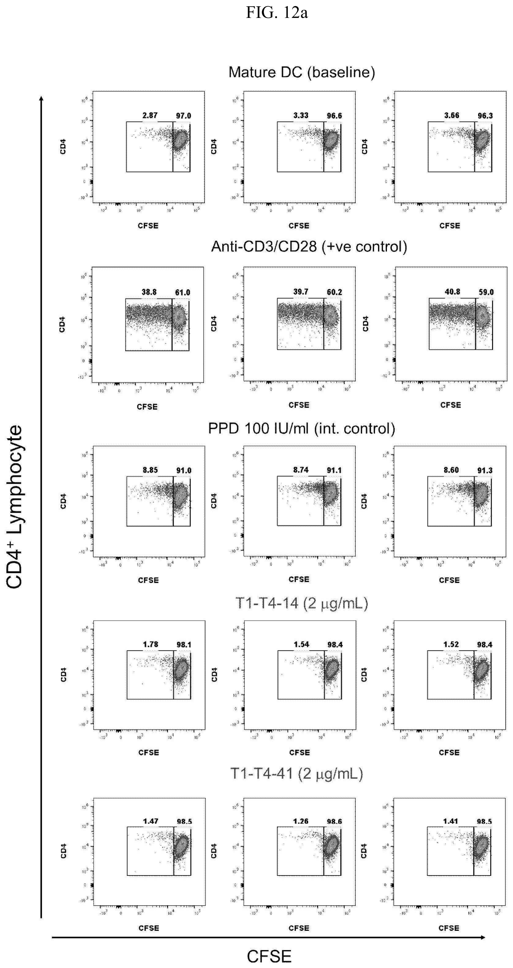

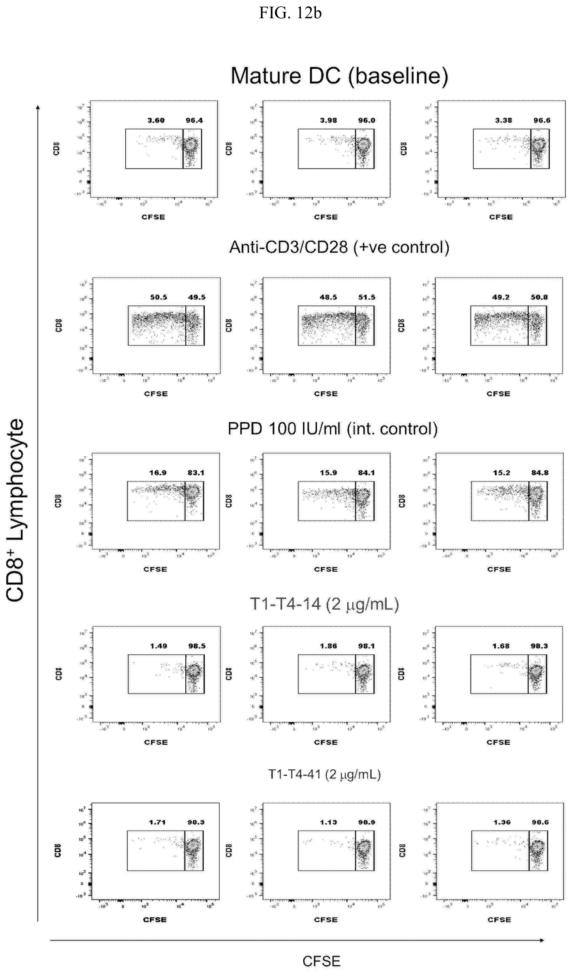

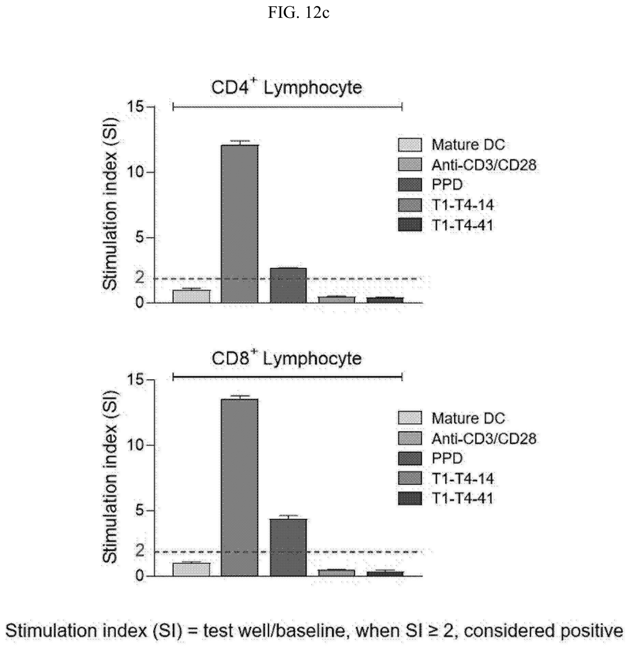

After a total of 6 days of dendritic cell (DC) differentiation, AIM-V medium containing GM-CSF and IL-4 was added to the harvested cells. The immature DCs were then treated with the test substances T1-T4-14 and T1-T4-41 as well as the positive control, PPD (Thermo Fisher, 7600060), for sensitization (24 hours, 37°C). The antigen-sensitized DCs were further cultured in maturation medium (IL-6; JW Creagen, HIL6-100, IL-1β; JW Creagen, HIL-1B-100, TNFα; JW Creagen, HTNA-100, PGE₂; Sigma, P0409, AIM-V medium) for 24 hours at 37°C. During the DC culture, the CD4⁺/CD8⁺ cells stored in liquid nitrogen were thawed in an Automated Cell Thawing System, and then the cells were harvested by centrifugation at 300 g for 5 minutes. After removing the supernatant, the cells were resuspended in AIM-V medium and plated in a 60 mm culture dish (24 hours, 37°C). The following day, to confirm the proliferation of CD4⁺/CD8⁺ T cells, the cells were treated with 5 µM CFSE (Invitrogen, C34554) and incubated at 37°C for 10 minutes. The CFSE-stained CD4⁺/CD8⁺ T cells were washed three times at room temperature by centrifugation at 300 g for 5 minutes in AIM-V medium (24 hours, 37°C). After harvesting the antigen-sensitized DCs, they were washed three times with AIM-V medium by centrifugation at 300 g at room temperature for 5 minutes. Then, 2 x 10⁵ CD4⁺/CD8⁺ T cells and 1 x 10⁴ matured DCs were plated in a 96-well plate. The CFSE-stained CD4⁺/CD8⁺ T cells were treated with anti-CD3 antibody (Biolegend, 300438) and anti-CD28 antibody (Biolegend, 302934). After co-culturing for 7 days, the cells were harvested, resuspended in antibody solution (CD3 and CD4 or CD8 antibodies), and reacted in the dark at 4°C for 30 minutes. After washing with Running Buffer, the cells were collected by centrifugation at 300 g for 5 minutes. The supernatant was removed, and the cells were resuspended in Running Buffer for analysis of CD4⁺/CD8⁺ T cell proliferation using a FACS Canto-II instrument (BD, 338962) (FIGs. 12a, 12b, and 12c).

In the *in vitro* immunogenicity evaluation, anti-CD3/anti-CD28 was used as a positive control and PPD was used as the internal control. The test substances IL-17-RA/RC-Fc-TNFR2 (T1-T4-14, T1-T4-41) were compared by treating in triplicate under a single concentration condition (2 µg/mL). The immunogenicity of the test substances IL-17-RA/RC-Fc-TNFR2 (T1-T4-14, T1-T4-41) was generally determined by the stimulation index (SI), which is used to assess the effect of drugs on immune cell activation. The SI value was obtained by dividing the T cell proliferation rate under each condition by the proliferation rate of the control. An SI value of 2 or greater is generally considered indicative of immunogenicity induced by lymphocyte proliferation according to known standards (Stimulation Index (SI) = test well/control baseline; SI ≥ 2 is considered ADA positive). In the case of the ADA positive control, PPD, immunogenicity was confirmed with SI values of 2 or greater for both MHC Class I and MHC Class II in all 5 types of PBMC. Based on the average SI values obtained from three replicates of the test substances, it was finally confirmed that IL-17-RA/RC-Fc-TNFR2 (T1-T4-14, T1-T4-41) did not induce immunogenicity, with SI values of 2 or less for both MHC Class I and MHC Class II in the 5 types of PBMC.

### Example 13: Efficacy Evaluation of IL-17RA/RC Hybrid Fusion Protein (IL-17RA/RC-Fc) in PBMCs

Patients with autoimmune diseases, as chronic conditions, differ from healthy individuals in the population of immune cells within PBMCs, the characteristics of each immune cell, and the properties of PBMCs and tissues at the lesion site. In psoriasis or psoriatic arthritis, IL-17A⁺CD8⁺ T cells are associated with disease severity. In addition, the proportion of IL-17A⁺CD8⁺ T cells was locally elevated in the tissue or damaged joint tissue (Xiaofei Xu et al., Arthritis Rheumatol. 72(8): 1303-1313, 2020). Both IL-17A and IL-17F are expressed in the lesions of psoriasis or psoriatic arthritis, with IL-17F expression being approximately 32-fold higher than that of IL-17A in the lesions of psoriasis (Helena Iznardo et al., Ther. Adv. Chronic. Dis. 12: 1-16, 2021). IL-17F amplifies pro-inflammatory responses, and its co-expression with IL-17A or both IL-17A and TNFα further enhances the production of inflammatory cytokines. In a clinical study evaluating the efficacy of Bimekizumab in the joints and skin of patients with psoriatic arthritis (Study No. NCT01087788), dual neutralization of IL-17A and IL-17F more effectively inhibited inflammatory responses compared to single neutralization of IL-17A. At 8 weeks, the ACR20 response rate was 80% for Bimekizumab and 17% for placebo. Compared to therapeutic antibodies that neutralize IL-17A only, the ACR20 response rates were 54% for Bimekizumab versus 15% for Secukinumab, and 62% for Bimekizumab versus 30% for Ixekizumab. These results demonstrated that Bimekizumab has superior efficacy compared to placebo, Secukinumab, and Ixekizumab (Glatt S. et al., Rheum. Dis. 77(4): 523-532, 2017). The effectiveness of regulating cytokine production was evaluated by treating PBMCs from patients with psoriasis using the following test materials: IL-17RA/RC-Fc (inhibiting IL-17A/F), TNFR2-Fc (inhibiting TNFα), a combination of IL-17RA/RC-Fc and TNFR2-Fc, IL-17RA/RC-Fc-TNFR2 (inhibiting both IL-17A/F and TNFα), or a positive control (Bimekizumab and Adalimumab, each at 0.5 µg/mL)

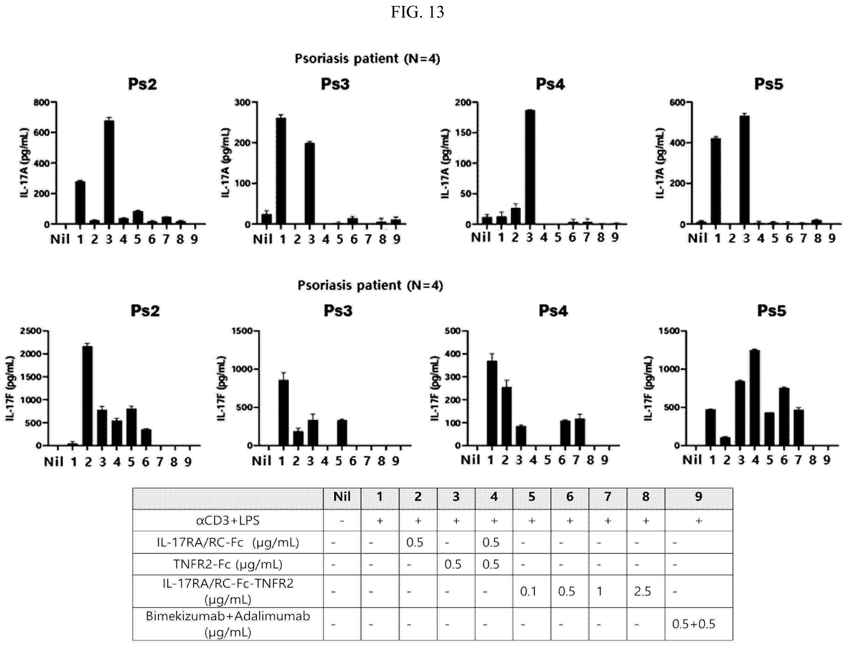

Four psoriasis patients (n=4) were recruited, and blood was collected to obtain peripheral blood mononuclear cells (PBMCs) (Catholic University of Korea, Seoul St. Mary's Hospital, Approval No. KC21TNSI0375). The isolated PBMCs were dispensed at 5×10⁵ cells/well in 500 µL into 48-well plates pre-coated with 2 µg/mL anti-CD3. Additionally, the cells were stimulated with 100 ng/mL LPS and treated with the test compounds under each condition, then cultured for 72 hours at 37°C. Subsequently, the culture supernatant was collected and used to perform ELISAs for IL-17A (Human IL-17A Duoset ELISA, R&D System DY317) and IL-17F (Human IL-17F Duoset, R&D System DY1335B) (absorbance measured using Molecular Devices). As a result, the production of IL-17A was reduced in all treatment groups, both in the test compound and control drug groups, based on the average analysis of psoriasis patient PBMC Ps2-5 results and the percentage change in production. Specifically, IL-17RA/RC-Fc (0.5 µg/mL) combined with TNFR2-Fc (0.5 µg/mL) showed a reduction of 96.3%, while IL-17RA/RC-Fc-TNFR2 at 0.1, 0.5, 1.0, and 2.5 µg/mL exhibited reductions of 91.9%, 89.85%, 88.3%, and 96.5%, respectively; the control drug group (combination treatment of Bimekizumab 0.5 µg/mL and Adalimumab 0.5 µg/mL) showed a 97.4% reduction (FIG. 13). Similarly, the production of IL-17F was decreased in all treatment groups based on the same analyses, with IL-17RA/RC-Fc-TNFR2 at 1.0 and 2.5 µg/mL showing reductions of 67.3% and 100%, respectively, and the control drug group confirming a 100% reduction (FIG. 13). Furthermore, treatment with IL-17RA/RC-Fc-TNFR2 (which simultaneously inhibits IL-17A/F and TNFα) resulted in a reduction in the production of IL-17A, IL-17F, IL-21, IL-22, TNFα, and IFN-γ. In particular, regarding IL-17F inhibition, the efficacy of IL-17RA/RC-Fc-TNFR2 (inhibiting IL-17A/F and TNFα simultaneously) was confirmed when compared to the single inhibition with IL-17RA/RC-Fc, and a similar result was observed in TNFα inhibition. In Example 7 (SEAP analysis) of the present invention, although the neutralizing activity of IL-17RA/RC-Fc-TNFR2 against IL-17F was slightly lower than that of Bimekizumab, its inhibition of IL-17F in psoriasis patient PBMCs was equivalent to the 100% reduction observed in the positive control group (combination treatment of Bimekizumab 0.5 µg/mL and Adalimumab 0.5 µg/mL). Compared to the combination treatment of Bimekizumab and Adalimumab which possess high affinities for the elevated IL-17A, IL-17F, or TNFα in psoriasis patient PBMCs, the IL-17RA/RC-Fc-TNFR2 that simultaneously inhibits IL-17A, IL-17F, and TNFα demonstrated an equivalent level of inhibitory efficacy. This indicates that selectively inhibiting pathogenic cytokines in psoriasis and autoimmune disease environments can effectively improve inflammatory diseases with minimal side effects.

### Example 14: Neutralizing Ability Analysis of IL-17RA/RC-Fc-mTNFR2 Against IL-17A and TNFα in a Psoriasis-Inducing Animal Model

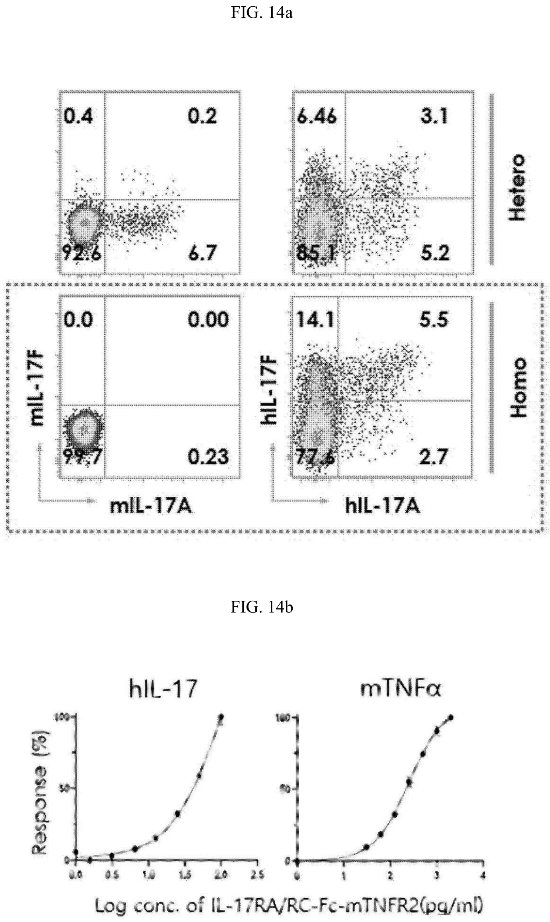

To evaluate the neutralizing ability in a psoriasis-inducing animal model, murine IL-17A and IL-17F were removed using the CRISPR/Cas 9 technique, followed by the creation of humanized double knock-in mice in which human IL-17A and IL-17F are expressed (see FIG. 14a). The IL-17RA/RC-Fc-TNFR2 described in the present invention is a multi-target fusion protein that targets hIL-17A, hIL-17F, and hTNFα. C57BL/6 hIL-17A/17F humanized double knock-in mice express hIL-17A and hIL-17F, while TNFα corresponds to murine TNFα. For this reason, it is difficult to evaluate the inhibitory effects on all three ligands (hIL-17A, hIL-17F, and hTNFα) and the therapeutic effectiveness of therapies targeting them in a psoriatic disease model using humanized double knock-in mice. Accordingly, to evaluate the efficacy of IL-17RA/RC-Fc-TNFR2 in double knock-in mice, IL-17RA/RC-Fc-mTNFR2 was created by substituting the hTNFR2 ECD, which is the hTNFα-binding moiety of IL-17RA/RC-Fc-TNFR2, with the mTNFR2 ECD. This modification enables targeting of hIL-17A, hIL-17F, and murine TNFα (mTNFα) expressed in humanized double knock-in mice. The experiment was conducted as described in Embodiment 8 to evaluate the interaction of IL-17RA/RC-Fc-mTNFR2 (T1-mT4-35, SEQ ID NO: 32) with hIL-17A, hIL-17F, and mTNFα. The EC₅₀ value of IL-17RA/RC-Fc-mTNFR2 for each cytokine was 80.31 ng/mL for hIL-17A, 90.74 ng/mL for hIL-17F, and 71.01 ng/mL for mTNFα, suggesting a similar affinity for each cytokine (see FIG. 14b).

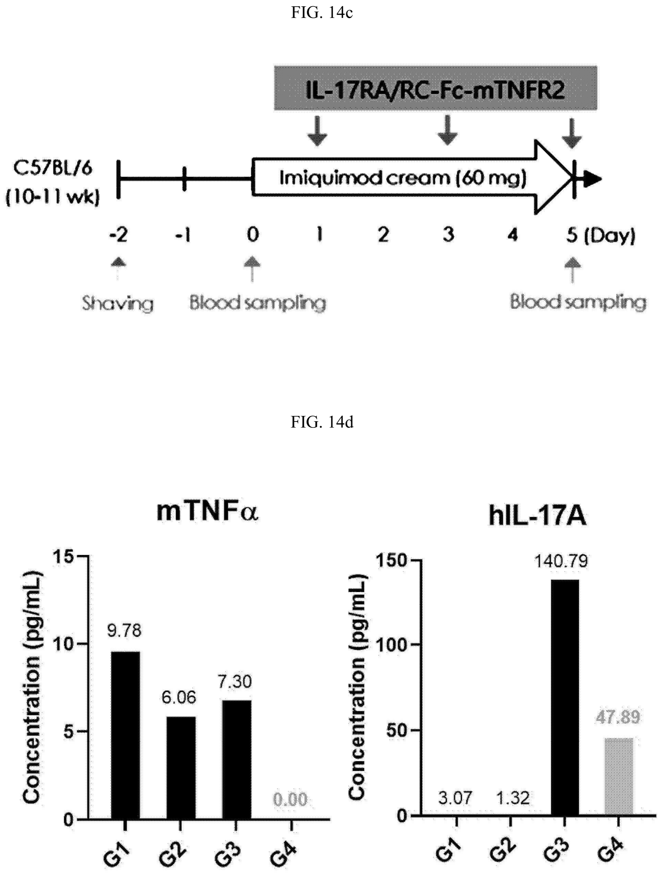

To induce psoriasis-like disease, hair removal was performed (using clippers to widely shave from the neck to the buttocks, then applying depilatory cream and wiping off with a dry tissue after 40 seconds), and 60 mg of Imiquimod (IMQ; Aldera Cream, FIG. 14c) was applied to the skin of the depilated area (the back) in the entire experimental groups (G1 ~ G4) (once daily for a total of 5 treatments). As an additional disease model test substance, 200 µl of LPS (lipopolysaccharide from Escherichia coli, O111:B4) was administered once intraperitoneally on the final day of the experiment (day 5). Subsequently, the test substance of the present invention, IL-17RA/RC-Fc-mTNFR2, was administered at 1 mg/kg (once every 2 days, starting the day after IMQ application). Blood was collected from each individual, allowed to clot at room temperature for 10 to 20 minutes, and then centrifuged (2,500 RPM, 15 minutes) to separate plasma from the supernatant, which was then stored frozen. Blood collection was performed twice in total: once before IMQ application and once on the final day (day 5). Using ELISA (Duoset ELISA, R&D system), standard curves for IL-17A and TNFα were generated, and the neutralizing activity of hIL-17RA/RC-Fc-mTNFR2 against the cytokines (hIL-17A and mTNFα) was confirmed. In the *in vivo* neutralization study in mice, the ELISA results from mouse blood samples on the final day (day 5) confirmed that the neutralizing activity against mouse TNFα was most significantly inhibited in G4 (C57BL/6 hIL-17A/17F double KI mice + hT1-mT4-35) (G3; 7.30 pg/mL, G4; 0.0 pg/mL), and that the neutralizing activity against human IL-17A was markedly inhibited in G4 (C57BL/6 hIL-17A/17F double KI mice + hT1-mT4-35) compared to G3 (C57BL/6 hIL-17A/17F double KI mice) (G3; 140.79 pg/mL, G4; 47.89 pg/mL) (Fig. 14d). The affinity analysis results of IL-17RA/RC-Fc-mTNFR2 (hT1-mT4-35) for IL-17A and mTNFα are presented in Table 18, and the psoriasis analysis data for G1 to G4 in C57BL/6 hIL-17A/17F double Knock-In mice are shown in Table 19.

**Table 18: Affinity analysis in a psoriasis mouse model**

| **Cytokine** | **IL-17RA/RC-Fc-TNFα (hTl-mT4-35, SEQ ID NO: 32)** | **EC₅₀ (ng/mL)** |
|---|---|---|
| Human IL-17A | hT1-mT4-35 | 80.31 |
| Human IL-17F | hT1-mT4-35 | 90.74 |
| Murine TNFα | hT1-mT4-35 | 71.01 |

**Table 19: Experimental conditions (G1 to G4)**

| **Group** | **Experimental Conditions** |
|---|---|
| G1 | C57BL/6 wild-type mice |
| G2 | C57BL/6 wild-type mice + "hIL-17RA/RC-Fc-mTNFR2" |
| G3 | C57BL/6 hIL-17A/17F double KI mice |
| G4 | C57BL/6 hIL-17A/17F double KI mice + "hIL-17RA/RC-Fc-mTNFR2" |

### Example 15: Cryogenic Electron Microscopy (cryo-EM) Analysis of the IL-17RA/RC-Fc-TNFR2 Hybrid Fusion Protein

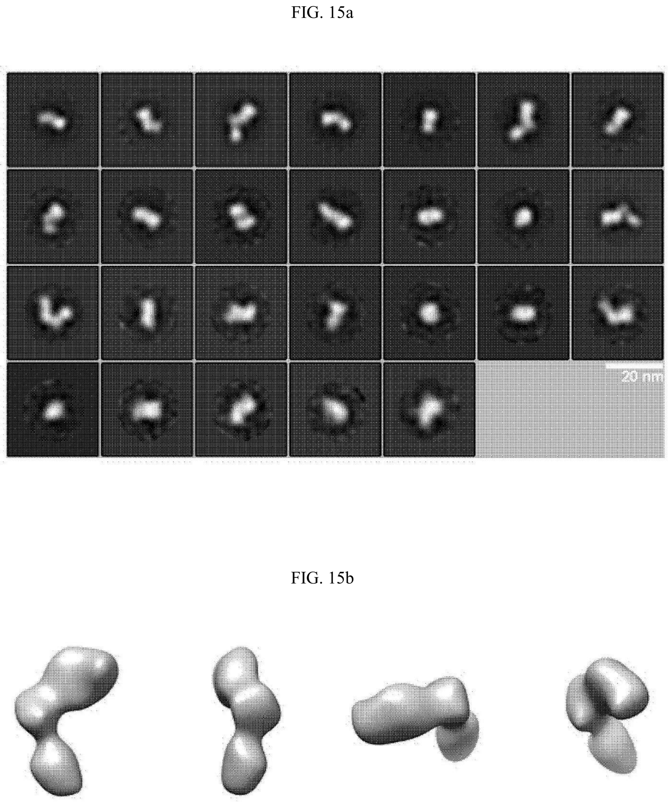

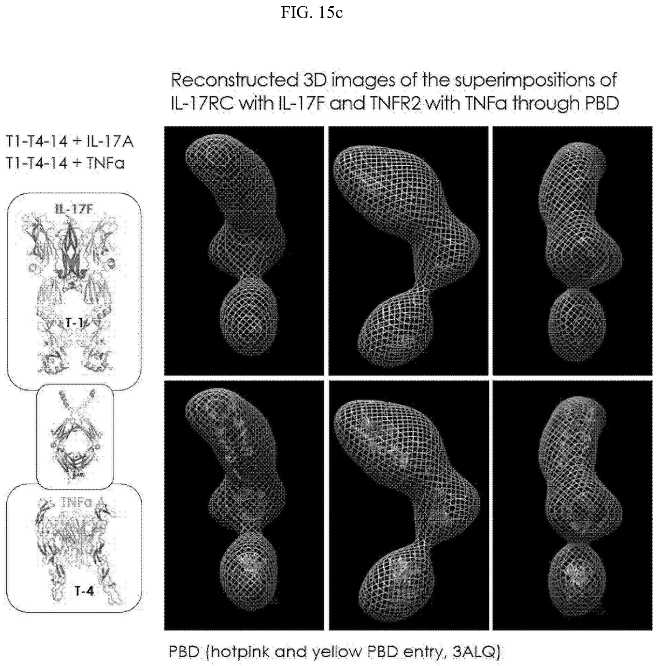

A three-dimensional tumor microenvironment (3D TME) is a technique used to study the structure of protein complexes by analyzing biological imaging data. The structure of each domain of IL-17RA/RC-hybrid fusion protein, as well as the interactions between its binding domains and target proteins, can be analyzed using high-resolution three-dimensional structures. *De novo* structure analysis was performed to determine the interactions between the IL-17RA/RC-Fc-TNFR2 fusion protein and its target proteins. Negative staining was performed with 1% uranyl acetate after adding 5 µL of IL-17RA/RC-hybrid fusion protein samples onto a carbon-coated grid prepared using glow-discharge (Harrick Plasma, USA), held in place with a self-closing tweezer (Dumont, Switzerland). Excess staining solution was removed by placing a filter paper (Whatman, UK) into contact with the edge of the grid. After drying the grid for 10 seconds, the experiment was conducted using a Tecnai 10 TEM (FEI, USA) operated at 100 kV. Images were captured at a resolution of 0.32 nm/pixel using an UltraScan 1000 CCD camera (Gatan, USA). Image analysis was performed using automated data from a total of 7,084 IL-17RA/RC-Fe-TMFR2 particles, followed by image alignment, reconstruction, 3D volume rendering, and visualization. The mean value of the class was calculated using the RELION 3.1 program. Excluding images that were not compact in the initial classification process or were poorly stained, folded images with clearly observable right and left sides were selected for the analysis of IL-17RA/RC-Fe-TMFR2 hybrid fusion protein particles. As shown in FIG. 15a, images obtained through 2D classification of particles from the raw data revealed that the IL-17RA/RC-Fc-TNFR2 protein particles were scanned at a variety of angles. Based on this result, 3D reconstruction was performed (see FIG. 15b). In an electron density map, the binding models of IL-17RC-IL-17F and TNFR2-TNFα, currently deposited in the protein data bank (PDB), along with Fc were fitted using the superimposition method (see FIG.15c). For the binding models of IL-17RC-IL-17F and TNFR2-TNFα, the structures from PDB IDs 6HG9 and 3ALQ were utilized. As the binding sites of TNFR2 and TNFα contain longer and more flexible linkers, a sparse electron density map and an even sparser 3D reconstruction are observed. In addition, since TNFR2 ECD, where the substrate is unbound, contains a flexible linker, it is difficult to identify the electron density map. However, as displayed in FIG. 15c, the model was well fitted to the electron density map due to the binding of TNFR2 and TNF. Accordingly, it was demonstrated that IL-17RA/RC-hybrid and TNFR2 ECD of the IL-17RA/RC-Fc-TNFR2 hybrid protein bind to each substrate in a 1:1 ratio, and that IL-17RC, which is relatively farther from the Fc in the middle, forms the upper part.

### Embodiment 16: Attachment of N-Glycans to the IL-17RA/RC-Fc-TNFR2 Hybrid Fusion Protein

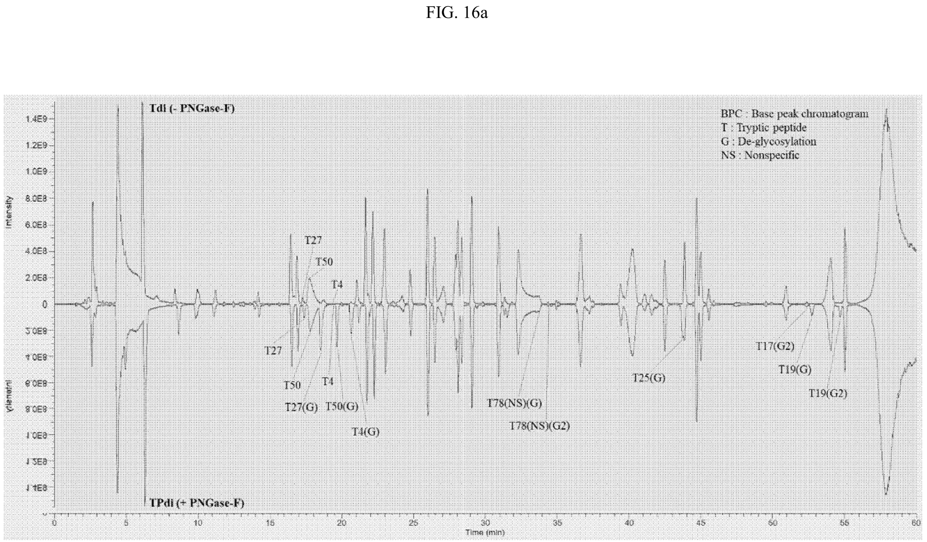

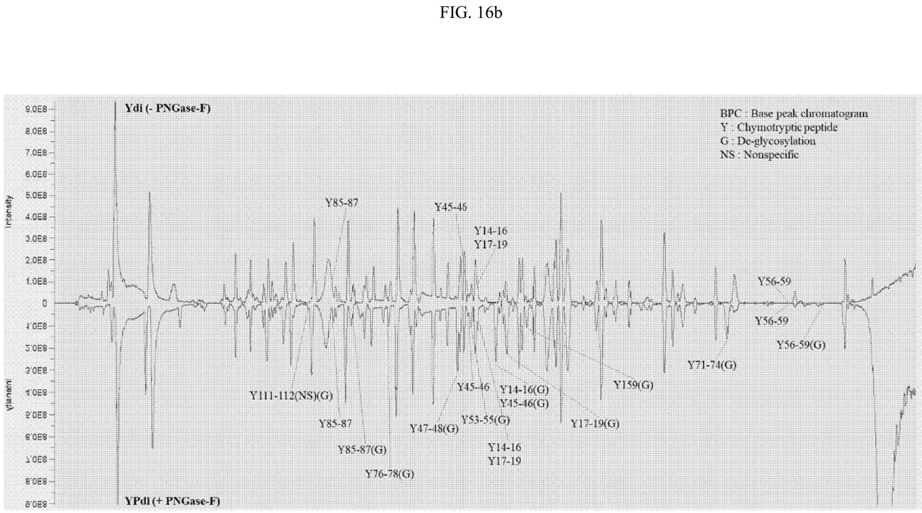

The composition and structure of Fc fusion glycans may affect changes in the structural domains. Changes in the specific glycosylation of a therapeutic Fc fusion protein may also impact the pharmacokinetics and pharmacodynamics of the molecule. Therefore, the present inventors investigated the possibility of establishing an expression system needed to improve the productivity by determining whether N-glycans were attached to the IL-17RA/RC-Fc-TNFR2 hybrid fusion protein (T1-T4-14, SEQ ID NO: 1). To determine whether N-glycans are theoretically attached to the sequences of the Fc fusion protein prepared in this embodiment, the N-glycan attachment sites were identified using peptide mapping. The produced Fc-fusion protein was reacted at -20°C for 30 minutes after adding a 50% TCA (trichloroacetic acid) solution to achieve a final concentration of 10%. Thereafter, desalting was performed by centrifuging at 13,000 rpm for 10 minutes and discarding the supernatant. The desalted Fc-fusion protein was then analyzed using UPLC-ESI-MS equipment-which connects ultra-performance liquid chromatography with a mass spectrometer-to confirm the attachment status by the percentage of N-glycan site occupancy (FIGs. 16a and 16b). The cleaved peptides were separated by C18 reverse-phase gradient chromatography and analyzed for 70 minutes using mobile phase A (purified water containing 0.1% formic acid) and mobile phase B (acetonitrile containing 0.1% formic acid). The gradient conditions for mobile phase B were set as follows: 2% from 0 to 5 minutes, 30% from 5 to 50 minutes, 95% from 51 to 61 minutes, and 2% from 62 to 70 minutes. The separated peptides were ionized via the mass spectrometer and analyzed by detecting ionized molecular weights in the range of 200 m/z to 2000 m/z. As a result of the analysis of N-glycosylation sites and occupancy (%) using the UPLC-ESI-MS system, a total of 12 N-glycosylation sites were identified when the results from the two proteolytic enzymes were combined, with only the N22 position of the IL-17RA/RC-Fc-TNFR2 hybrid fusion protein not being confirmed. The determination of the N-glycan sites using different proteolytic enzymes is summarized in Table 20.

**Table 20. Selection of N-glycan sites**

| **Site** | | **Trypsin** | **Chymotrypsin** |
|---|---|---|---|
| | | **N-glycosylation occupancy rate (%)** | |
| hIL-17RA/RC | N17 | 98.6 | 97.8 |
| | N22 | N.D. | N.D. |
| | N35 | N.D. | 97.3 |
| | N173 | 100.0 | 78.5 |
| | N186 | 100.0 | 100.0 |
| | N213 | 52.7 | 100.0 |
| | N223 | 52.7 | 98.5 |
| | N309 | 100.0 | 100.0 |
| | N332 | 99.4 | 100.0 |
| | N366 | N.D. | 98.3 |
| Fc | N95 | 99.9 | 100.0 |
| hTNFR2 | N149 | 51.7 | 100.0 |
| | N171 | 51.7 | N.D. |

### Example 17: Glycan Sialylation of the IL-17RA/RC-Fc-TNFR2 Hybrid Fusion Protein

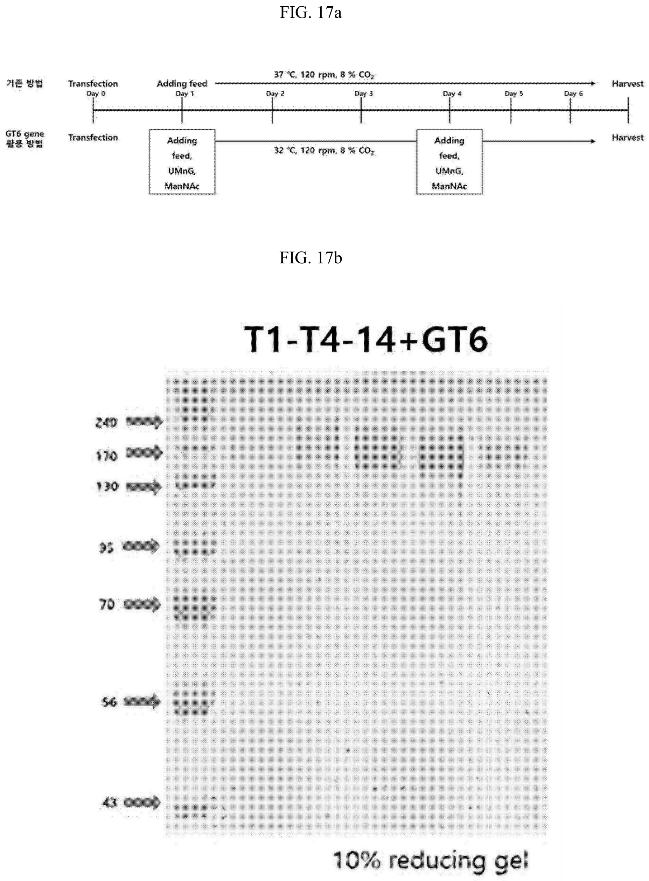

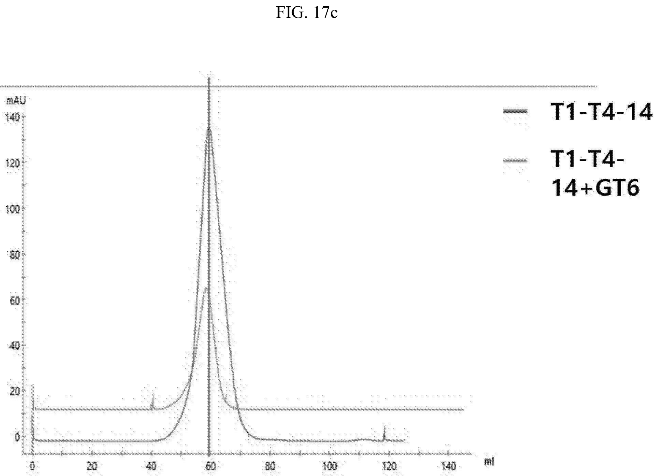

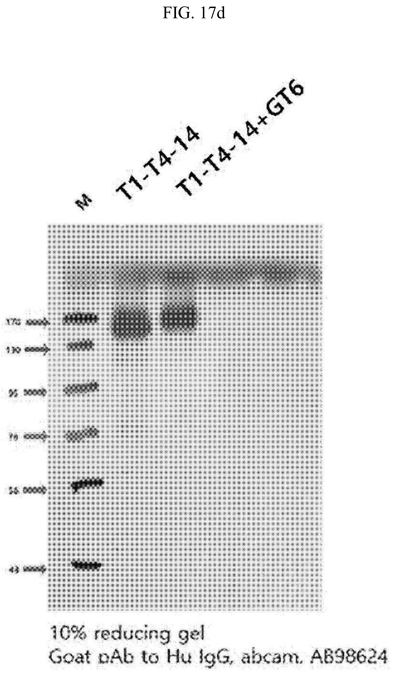

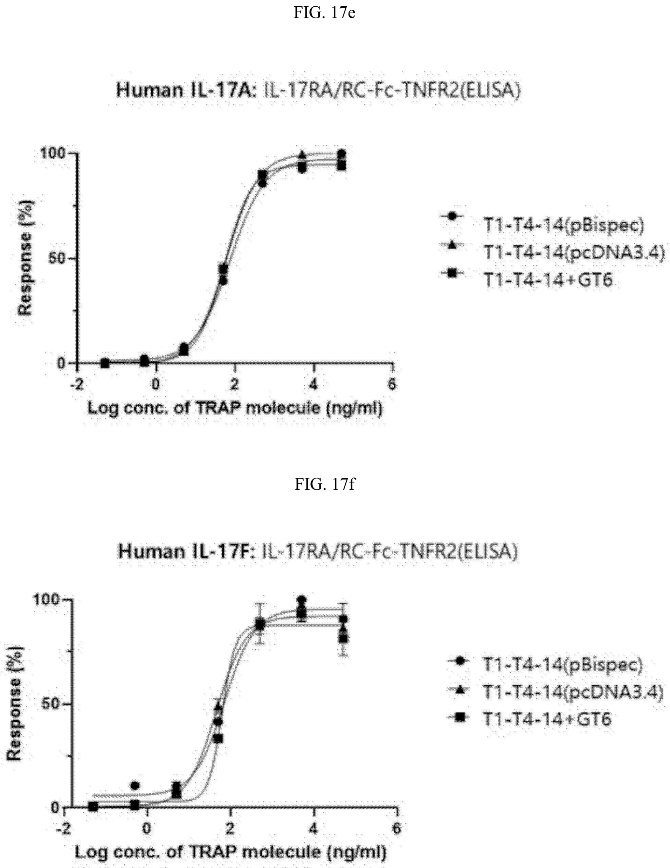

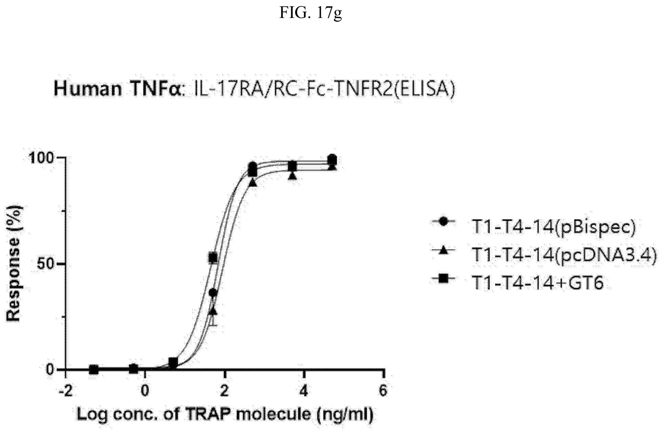

A significant proportion of biologics are recombinant proteins, with at least 70% of these classified as glycoproteins. Human-like glycosylation is linked to therapeutic effectiveness and plays a crucial role in reducing immunogenicity while increasing serum half-life. The degree of sialylation of the glycosylated proteins (glycoproteins) may be a crucial factor affecting their plasma half-life or safety. Recombinant expression of sialylated proteins for therapeutic purposes can be challenging when organisms lack the enzymes required for sialylation or when proteins with non-human sialylation patterns are produced (Xiaotian Zhong et al., Methods. Mol. Biol. 12(3): 53, 2022). The introduction of galactosyltransferase-6 (GT6) into the ExpiCHO-S^{™} system can promote the synthesis of sialylated glycans. The IL-17RA/RC-Fc-TNFR2 fusion protein was expressed in ExpiCHO-S cells according to the protocol of the ExpiCHO^{™} Expression System Kit, as described in Example 1. For the above experiment, the DNA of the IL-17RA/RC-Fc-TNFR2 fusion protein and the GT6 DNA were added at a ratio of 5:4. The GT6 DNA utilized was from Beta-1,4-galactosyltransferase 6 (B4GALT6) using the Lenti-ORF clone of B4GALT6 (mGFP-tagged)-Human UDP-Gal (Origene CAT#: RC210213L4). T1-T4-14 (50 µC) and GT6 (40 µ ) were transfected with the IL-17RA/RC-Fc-TNFR2 fusion protein DNA and GT6 DNA, respectively. After 18 hours, the enhancer and feed included in the ExpiFecta^{™} CHO Transfection Kit were added, along with UMnG (200 mM Uridine (GLENTHAM, Cat: GP7250-5G), 0.4 mM MnCl₂ (Sigma, Cat: 244589-50G), 1 M Galactose (GLENTHAM, Cat: GC7360-500G)) at 2 ml each, and ManNAc (65 mM N-acetylmannosamine, Glentham, Cat: GK3319) at 1.55 ml. Subsequently, the culture was maintained at 8% CO2, 32°C, and 120 rpm. On day 4, along with the enhancer and feed, UMnG and ManNAc were again added at 2 ml and 1.55 ml, respectively, and the culture was continued under the same conditions (8% CO2, 32°C, and 120 rpm). On day 7, the protein was harvested to complete production. FIG. 17a is a schematic diagram showing the production schedule for the glycan sialylation of the IL-17RA/RC-Fc-TNFR2 hybrid fusion protein. As in Example 3, the IL-17RA/RC-Fc-TNFR2 fusion protein that underwent glycan sialylation was purified via Protein A affinity chromatography. The glycan sialylated IL-17RA/RC-Fc-TNFR2 fusion protein and the unsialylated IL-17RA/RC-Fc-TNFR2 fusion protein were then loaded onto a Superdex 200 (Cytiva) column for separation and purification (HiLoad 16/600 Superdex 200 pg 1x120ml (Cytiva, 28989335), Buffer: 1x PBS, 0.005% Tween 20). The SEC results showed that the peak shifted forward due to the change in protein size (FIGs. 17b and 17c), and Western blot analysis confirmed that the glycan sialylated protein appeared larger (FIG. 17d). The effect of glycan sialylation on the affinity of the IL-17RA/RC-Fc-TNFR2 fusion protein for its ligands IL-17A, IL-17F, and TNFα was also analyzed. As a control, the affinity for each ligand was analyzed by ELISA for T1-T4-14 produced in Bispec (SEQ ID NO: 1), T1-T4-14 produced in pcDNA3.4, and glycan sialylated T1-T4-14. The affinities for IL-17A, IL-17F, and TNFα were measured separately for T1-T4-14 produced in pBispec, in pcDNA3.4, or in pcDNA3.4+GT6 (FIGs. 17e, 17f, and 17g). For IL-17A, the EC50 values of T1-T4-14 were determined to be 75.18 ng/ml, 60.65 ng/ml, and 54.32 ng/ml, respectively; for IL-17F, the EC₅₀ values were 70.20 ng/ml, 45.48 ng/ml, and 62.06 ng/ml, respectively; and for TNFα, the EC₅₀ values were 68.00 ng/ml, 87.26 ng/ml, and 44.34 ng/ml, respectively.

To assess the *in vivo* stability of the IL-17RA/RC-Fc-TNFR2 fusion protein, the remaining protein amount in the serum of mice (C57BL/6) was measured after 4 hours of treatment. The results showed that T1-T4-14 with glycan sialylation introduced via GT6 exhibited more than a two-fold increase in stability (14.99 µg/ml vs. 28.94 µg/ml).

### Example 18: Analysis Affinity of the IL-17RA/RC-Fc-TNFR2 Hybrid Fusion Protein for TNFα

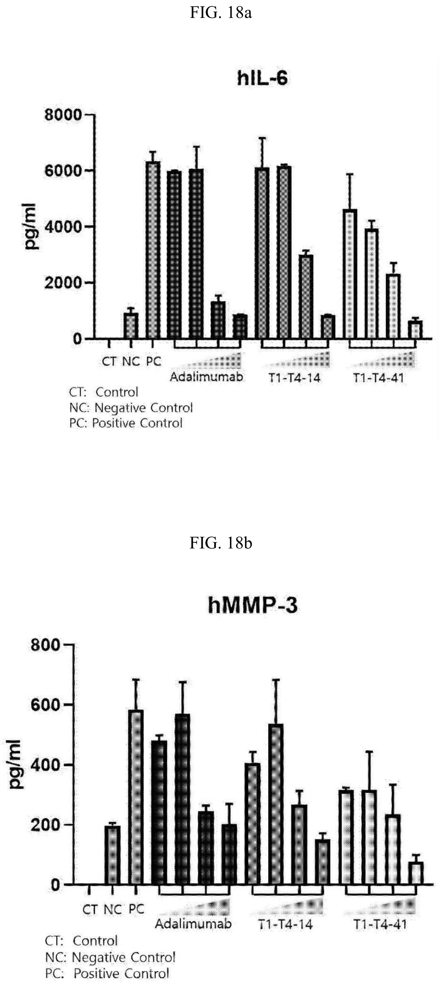

The expression levels of IL-6 and MMP3 were evaluated after treating SW982 cells with hTNFα to investigate disease improvement based on the neutralizing ability of TNFR2 from the multi-target fusion protein, IL-17RA/RC-Fc-TNFR2, in a rheumatoid arthritis-like cell model using SW982 cells. Rheumatoid arthritis is caused by the recruitment of inflammatory cells, such as neutrophils and macrophages, into the joints, leading to the overexpression of inflammatory cytokines (e.g., IL-Iβ, IL-6, and CXCL8) and the destruction of joint structures. SW982 cells were cultured overnight at 37°C in each well of a 96-well plate, seeding 1×10⁴ cells per well in DMEM (Welgene, LM001-005) supplemented with 10% FBS (Gibco, 16000044). The following day, the cells were treated with TNFα cytokine (10 ng/mL) to induce an RA model and simultaneously treated with IL-17RA/RC-Fc-TNFR2 (T1-T4-14) protein and the control drug adalimumab (Selleckchem, A2010) at concentrations of 2.5, 10, 40, and 160 ng/mL along with TNFα (48 hours, 37°C). After 48 hours of treatment, the culture media were collected and an ELISA was performed to determine IL-6 and MMP3 levels. The ELISA kits used were as follows, and all reagents were prepared according to the manufacturers' instructions: Human IL-6 DuoSet ELISA (R&D systems, DY206-05), Human Total MMP3 DuoSet ELISA (R&D systems, DY513-05), and DuoSet ELISA Ancillary Reagent Kit 2 (R&D systems, DY008B). The capture antibodies were diluted in 1X Reagent Diluent (1% PBS in PBS, pH 7.2-7.4, 0.2 µm filtered, R&D systems, Catalog #DY995) and added at 100 µL per well to the ELISA plates, then incubated at room temperature overnight for coating. The plates were washed three times with 300 µL per well of 1x ELISA wash buffer (0.05% Tween 20 in PBS, pH 7.2-7.4, R&D systems, Catalog #WA126). Next, 100 µL per well of reagent diluent was added, and the plates were blocked at room temperature for at least 1 hour. After washing with 300 µL per well of ELISA wash buffer, 100 µL per well of IL-6 and MMP3 standard proteins and culture media samples were added, and the reaction was allowed to proceed at room temperature for 2 hours (IL-6 samples were diluted 20-fold, whereas MMP3 was used undiluted). The standard curves were prepared according to the instructions: for IL-6, concentrations of 9.38, 18.8, 37.5, 75, 150, 300, and 600 pg/mL; for MMP3, concentrations of 31.3, 62.5, 125, 250, 500, 1000, and 2000 pg/mL. The plates were washed three times with 300 µL per well of ELISA wash buffer. Then, 100 µL per well of detection antibody was added and incubated for 2 hours. After three washes with 300 µL per well of ELISA wash buffer, an HRP-conjugated secondary antibody was applied and incubated at room temperature for 20 minutes. The plates were then washed five times with ELISA wash buffer. Finally, 100 µL of TMB solution was added at room temperature, and after the reaction, 50 µL of stop solution was added. The ELISA readings were measured using a BioTek Synergy HTX Microplate Reader at 540 nm and 450 nm. The results confirmed that the TNFR2 neutralizing activity of the IL-17RA/RC-Fc-TNFR2 of the present invention was capable of inhibiting IL-6 and MMP3 at a level equivalent to that of adalimumab (FIGs. 18a and 18b).

The present invention has been described with reference to the above examples, but these are merely exemplary. It will be understood by those skilled in the art that various modifications and equivalent alternative embodiments are possible. Accordingly, the true technical scope of the present invention should be determined by the technical spirit of the appended claims.

## Claims

1. An IL-17RA/RC variant hybrid protein having a sequence homology of at least 90% to the amino acid sequence of SEQ ID NO: 69 and comprising amino acid substitutions selected from the group consisting of:
i) Q267R,
ii) T24D, N88D, D122G, and Q267R,
iii) T24D, F59V, N88D, D122G, and Q267R,
iv) L9P, T24D, F59V, N88D, D122G, and Q267R, and
v) L9P, T24D, F59V, N88D, D122G, A156P, and Q267R.

2. The IL-17RA/RC variant hybrid protein of claim 1, wherein a glycan attached to the IL-17RA/RC variant hybrid protein.

3. The IL-17RA/RC variant hybrid protein of claim 2, wherein the glycan is at least one selected from the group consisting of N-glycan, O-glycan, and sialylated glycan.

4. The IL-17RA/RC variant hybrid protein of claim 1, wherein IL-17RA/RC variant hybrid protein has the amino acid sequence selected from the group consisting of SEQ ID NOs: 66 to 69.

5. A polynucleotide encoding the IL-17RA/RC variant hybrid protein of claim 1.

6. An expression vector comprising the polynucleotide of claim 5.

7. A transformed cell transduced with the expression vector described of claim 6.

8. A homodimer or heterodimer comprising the IL-17RA/RC variant hybrid protein of claim 1.

9. An IL-17RA/RC-Fc variant hybrid protein in which an antibody Fc domain is linked to either the N-terminus or the C-terminus of the IL-17RA/RC variant hybrid protein of claim 1.

10. The IL-17RA/RC-Fc variant hybrid protein of claim 9, wherein the antibody Fc domain is a hybrid Fc domain with two or more homozygous Fc domains mixed.

11. The IL-17RA/RC-Fc variant hybrid protein of claim 10, wherein the hybrid Fc domain comprises an amino acid sequenceis selected from the group consisting of the amino acid sequences of SEQ ID NOs: 33 to 65.

12. A polynucleotide encoding the IL-17RA/RC-Fc variant hybrid protein of claim 9.

13. An expression vector that includes the polynucleotide of claim 12.

14. A transformed cell transduced with the expression vector of claim 13.

15. A homodimer or heterodimer comprising the IL-17RA/RC-Fc variant hybrid protein of claim 9.

16. A TNFR2 ECD variant protein having a sequence homology of at least 90% to the amino acid sequence of SEQ ID NO: 77 and comprising amino acid substitutions selected from the group consisting of:
i) S33A,
ii) S33A and S36P,
iii) S33A, S36P, and R119S,
iv) S33A, S36P, R119S, and N164S,
v) S33A, S36P, R119S, N164S, and E210G,
vi) S33A, S36P, R119S, N164S, E210G, and F219I,
vii) S33A, S36P, G75S, R119S, N164S, E210G, F219I,
viii) G75S, and
ix) S33A, S36P, G75S, R119S, and N164S.

17. The TNFR2 ECD variant protein of claim 16, wherein at least one among i) to ix) form a complex linked to another by a linker peptide.

18. The TNFR2 ECD variant protein of claim 16, wherein a glycan attached to the TNFR2 ECD variant protein.

19. The TNFR2 ECD variant protein of claim 18, wherein the glycan is at least one selected from the group consisting of N-glycan, O-glycan, and sialylated glycan.

20. The TNFR2 ECD variant protein of claim 16, wherein TNFR2 ECD variant protein has an amino acid sequence selected from the group consisting of the amino acid sequences of SEQ ID NOs: 83 to 94.

21. A polynucleotide encoding the TNFR2 ECD variant protein of claim 16.

22. An expression vector comprising the polynucleotide of claim 21.

23. A transformed cell transduced with the expression vector of claim 22.

24. A homodimer or heterodimer comprising the TNFR2 ECD variant protein of claim 16.

25. A TNFR2 ECD-Fc variant protein in which an antibody Fc domain is linked to either the N-terminus or the C-terminus of the TNFR2 ECD variant protein of claim 16.

26. The TNFR2 ECD-Fc variant protein of claim 25, wherein the antibody Fc domain is a hybrid Fc domain with two or more homozygous Fc domains mixed.

27. The TNFR2 ECD-Fc variant protein of claim 26,wherein the hybrid Fc domain comprises an amino acid sequence selected from the group consisting of the amino acid sequences of SEQ ID NOs: 33 to 65.

28. A polynucleotide that encodes the TNFR2 ECD-Fc variant protein of claim 25.

29. An expression vector comprisingdes the polynucleotide of claim 28.

30. A transformed cell transduced with the expression vector of claim 29.

31. A homodimer or heterodimer comprising the TNFR2 ECD-Fc variant protein of claim 25.

32. A triple fusion protein comprising:
an IL-17RA/RC variant hybrid protein linked to the N-terminus of an antibody Fc domain, and an extra cellular domain (ECD) of tumor necrosis factor 2 (TNFR2) linked to the C-terminus the antibody Fc domain; or
the ECD of TRFR2 is linked to the N-terminus of the antibody Fc domain and the IL-17RA/RC variant hybrid protein is linked to the C-terminus of the antibody Fc domain.

33. The triple fusion protein of claim 32, wherein the IL-17RA/RC variant hybrid protein has a sequence homology of at least 90% to the amino acid sequence of SEQ ID NO: 69 and by comprises amino acid substitutions selected from the group consisting of:
i) Q267R,
ii) T24D, N88D, D122G, and Q267R,
iii) T24D, F59V, N88D, D122G, and Q267R,
iv) L9P, T24D, F59V, N88D, D122G, and Q267R, and
v) L9P, T24D, F59V, N88D, D122G, A156P, and Q267R.

34. The triple fusion protein of claim 32, wherein the TNFR2 ECD has a sequence homology of at least 90% to the amino acid sequence of SEQ ID NO: 77 and comprises amino acid substitutions selected from the group consisting of:
i) S33A,
ii) S33A and S36P,
iii) S33A, S36P, and R119S,
iv) S33A, S36P, R119S, and N164S,
v) S33A, S36P, R119S, N164S, and E210G,
vi) S33A, S36P, R119S, N164S, E210G, and F219I,
vii) S33A, S36P, G75S, R119S, N164S, E210G, F219I,
viii) G75S, and
ix) S33A, S36P, G75S, R119S, and N164S.

35. The triple fusion protein of claim 34, wherein at least one among i) to ix) form a complex linked to another by a linker peptide.

36. The triple fusion protein of claim 32, wherein a glycan attached to the triple fusion protein.

37. The triple fusion protein of claim 36, wherein the glycan is at least one selected from the group consisting of N-glycan, O-glycan, and sialylated glycan.

38. The triple fusion protein of claim 32, wherein the TNFR2 comprises an amino acid sequence selected from the group consisting of the amino acid sequences of SEQ ID NOs: 83 to 94.

39. The triple fusion protein of claim 32, wherein the triple fusion protein simultaneously targets IL-17A, IL-17F, and TNFα.

40. The triple fusion protein of claim 32, wherein the triple fusion protein comprises an amino acid sequence selected from the group consisting of the amino acid sequences of SEQ ID NOs of 1, 2, 4, 6, 8, 10, 12, 14, 16, 18, 20, 22, 24, 26, 28, 30, and 32.

41. A polynucleotide encoding the triple fusion protein of claim 32.

42. An expression vector comprising the polynucleotide of claim 41.

43. A transformed cell transduced with the expression vector of claim 42.

44. A homodimer or heterodimer comprising the triple fusion protein of claim 32.

45. A pharmaceutical composition for the treatment of autoimmune diseases comprising the IL-17RA/RC variant hybrid protein of claim 1 and the TNFR2 ECD variant protein of claim 16 as active ingredients.

46. A pharmaceutical composition for the treatment of autoimmune diseases comprising the IL-17RA/RC-Fc variant hybrid protein of claim 9 and the TNFR2 ECD-Fc variant protein of claim 25 as active ingredients.

47. A pharmaceutical composition for the treatment of autoimmune diseases comprising the triple fusion protein of any one claim among claims 32 to 40 or the homodimer or heterodimer of claim 44 as active ingredients.

48. The pharmaceutical composition of any one claim among claims 45 to 47, wherein the autoimmune disease is selected from the group consisting of rheumatoid arthritis, psoriasis, psoriatic arthritis, inflammatory bowel disease, and ankylosing spondylitis.

49. The pharmaceutical composition of claim 48, wherein the inflammatory bowel disease is selected from the group consisting of ulcerative colitis, Crohn's disease, and Behçet's disease.
